(19) Europäisches Patentamt
European Patent Office
Office européen des brevets

(11) Veröffentlichungsnummer: **0 114 045**
**B1**

(12) # EUROPÄISCHE PATENTSCHRIFT

(45) Veröffentlichungstag der Patentschrift: **03.09.86**

(51) Int. Cl.⁴: **C 07 F 9/65,** A 01 N 57/16

(21) Anmeldenummer: **84100062.3**

(22) Anmeldetag: **04.01.84**

(54) **Pyrimidinderivate und deren Verwendung als Schädlingsbekämpfungsmittel.**

(30) Priorität: **07.01.83 CH 89/83**
**18.11.83 CH 6214/83**

(43) Veröffentlichungstag der Anmeldung: **25.07.84 Patentblatt 84/30**

(45) Bekanntmachung des Hinweises auf die Patenterteilung: **03.09.86 Patentblatt 86/36**

(84) Benannte Vertragsstaaten: **AT BE CH DE FR GB IT LI LU NL SE**

(56) Entgegenhaltungen:
**EP-A-0 000 945**
**DE-A-2 804 889**

(73) Patentinhaber: **F. HOFFMANN- LA ROCHE & CO. Aktiengesellschaft, CH- 4002 Basel (CH)**

(72) Erfinder: **Lüthy, Christoph, Dr., Gfennstrasse 43, CH- 8603 Schwerzenbach (CH)**

(74) Vertreter: **Lederer, Franz, Dr., Patentanwälte Dr. Lederer Franz Meyer- Roxlau Reiner F. Lucile-Grahn- Strasse 22, D-8000 München 80 (DE)**

EP 0 114 045 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft heterocyclische Verbindungen, insbesondere Pyrimidinderivate der allgemeinen Formel

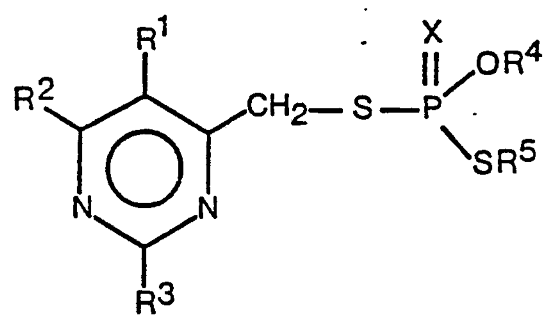

I

worin $R^1$ Wasserstoff, Fluor oder Chlor,

$R^2$ Wasserstoff, Fluor, Chlor, $C_{1-4}$-Alkyl, $C_{1-6}$-Alkoxy, 2-Propenyloxy, 2-Propinyloxy, 2-($C_{1-4}$-Alkoxy)-äthoxy, unsubstituiertes oder mit Halogen mono- oder disubstituiertes Phenoxy, $C_{1-6}$-Alkylthio, $C_{1-4}$-Alkylamino, Di($C_{1-4}$-alkyl)amino oder eine Gruppe (a) oder (b)

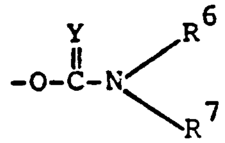

(a)

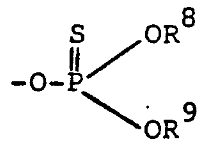

(b)

$R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkyl-thio, $C_{1-4}$-Alkylamino, Di($C_{1-4}$-alkyl)amino oder unsubstituiertes, monosubstituiertes oder disubstituiertes Phenyl, wobei die Substituenten Fluor, Chlor, Trifluormethyl und/oder Methoxy sind,

$R^4$ $C_{1-3}$-Alkyl,

$R^5$ $C_{1-6}$-Alkyl,

$R^6$ und $R^7$ bzw. $R^8$ und $R^9$ unabhängig voneinander $C_{1-3}$-Alkyl

und X und Y unabhängig voneinander Sauerstoff oder Schwefel bedeuten.

Die Verbindungen der Formel I sind Schädlingsbekämpfungsmittel und eignen sich insbesondere zur Bekämpfung von Insekten, Milben und Nematoden. Somit umfasst die Erfindung auch Schädlingsbekämpfungsmittel, welche Verbindungen der Formel I als Wirkstoffe enthalten, ein Verfahren zur Herstellung dieser Verbindungen sowie die Verwendung dieser Verbindungen bzw. Mittel zur Bekämpfung von Schädlingen.

Die in obiger Definition der Verbindungen der Formel I erwähnten $C_{1-3}$-, $C_{1-4}$- bzw. $C_{1-6}$-Alkylreste können sowohl geradkettig als auch verzweigt sein. Dies gilt auch für die $C_{1-4}$- bzw. $C_{1-6}$-Alkylreste der 2-($C_{1-4}$-Alkoxy)-äthoxy-, $C_{1-4}$-Alkylamino-, Di($C_{1-4}$-alkyl)amino-, ($C_{1-4}$-Alkoxy)-methyl-, ($C_{1-4}$-Alkylthio)methyl-, $C_{1-6}$-Alkoxy- und $C_{1-6}$-Alkylthiogruppen. Unter Alkyl sind je nach Anzahl der Kohlenstoffatome z.B. folgende Reste zu verstehen: Methyl, Aethyl, n-Propyl, Isopropyl, n-Butyl, Isobutyl, tert. Butyl, Isoamyl und n-Hexyl

Der Ausdruck "Halogen" umfasst Fluor, Chlor, Brom und-Jod, wobei Fluor und Chlor bevorzugt sind. In der mit Halogen mono- oder disubstituierten Phenoxygruppe ist vorzugsweise die p- und/oder die m-Stellung besetzt.

Durch das Vorliegen eines oder mehrerer asymmetrischer Phosphoratome und/oder eines oder mehrerer asymmetrischer Kohlenstoffatome in den Verbindungen der Formel I treten die Verbindungen in optisch

aktiver Form auf. Die Formel soll demnach die Racemate sowie die aufgetrennten optisch aktiven Formen umfassen.

Eine besondere Untergruppe von Verbindungen der Formel I besteht aus denjenigen Verbindungen der Formel I, in der $R^2$ Wasserstoff, Fluor, Chlor, $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, Di($C_{1-4}$-alkyl)-amino oder unsubstituiertes, monosubstituiertes oder disubstituiertes Phenyl bedeuten, wobei die Substituenten Fluor, Chlor, Trifluormethyl und/oder Methoxy sind, und $R^1$, $R^4$, $R^5$ und X die oben angegebenen Bedeutungen besitzen.

Unabhängig voneinander bedeuten $R^1$ vorzugsweise Wasserstoff oder Chlor, insbesondere Wasserstoff, $R^2$ vorzugsweise $C_{1-3}$-Alkoxy, 2-Propinyloxy, Phenoxy, $C_{1-3}$-Alkylthio oder eine Gruppe (a), insbesondere Methoxy, Aethoxy, Isopropoxy oder 2-Propinyloxy, $R^3$ vorzugsweise Wasserstoff; $C_{1-6}$-Alkyl, insbesondere Methyl, Isopropyl oder tert. Butyl; $C_{3-6}$-Cycloalkyl, insbesondere Cyclopropyl; $C_{1-6}$-Alkoxy, insbesondere Methoxy oder Aethoxy; $C_{1-6}$-Alkylthio, insbesondere Methyl- oder Aethylthio; $C_{1-4}$-Alkylamino, insbesondere Isopropylamino; oder Di($C_{1-4}$-alkyl)amino, insbesondere Dimethyl- oder Diäthylamino, $R^4$ vorzugsweise Aethyl, $R^5$ vorzugsweise n-Propyl oder sek. Butyl, insbesondere die erstere Gruppe, $R^6$ und $R^7$ vorzugsweise je Methyl, $R^8$ und $R^9$ vorzugsweise Methyl oder Aethyl, und X und Y unabhängig voneinander vorzugsweise Sauerstoff.

Bevorzugte Verbindungen der Formel I sind:
Dithiophosphorsäure-[O-äthyl-S-(2,6-diäthoxy-4-pyri-midinylmethyl)-S-(n-propyl)]ester und
Dithiophosphorsäure-[O-äthyl-S-[2-isopropyl-6-(2-propinyloxy)-4-pyrimidinylmethyl]-S-(n-propyl)] ester.

Weitere Vertreter von Verbindungen der Formel I sind:
Dithiophosphorsäure-[O-äthyl-S-(2-cyclopropyl-6-methylthio-4-pyrimidinylmethyl)-S-(n-propyl)]ester,
Dithiophosphorsäure-[O-äthyl-S-(6-äthylthio-2-methyl-4-pyrimidinylmethyl)-S-(n-propyl)]ester,
Dithiophosphorsäure-[O-äthyl-S-(6-chlor-2-cyclo-propyl-4-pyrimidinylmethyl)-S-(n-propyl)]ester,
Dithiophosphorsäure-[O-äthyl-S-(2-cyclopropyl-6-isopropoxy-4-pyrimidinylmethyl)-S-(n-propyl)]ester,
Dithiophosphorsäure-[O-äthyl-S-[2-cyclopropyl-6-(2-propenyloxy)-4-pyrimidinylmethyl] -S-(n-propyl)]ester,
Dithiophosphorsäure-[O-äthyl-S-[2-cyclopropyl-6-(2-propinyloxy)-4-pyrimidinylmethyl]-S-(n-propyl)]ester,
Dithio-phosphorsäure-[S-(2-äthoxy-6-methyl-4-pyrimi-dinylmethyl)-O-äthyl-S-(n-propyl)]ester,
Dithiophosphorsäure-[S-(6-äthoxy-2-methyl-4-pyrimi-dinylmethyl)-O-methyl-S-(n-propyl)]ester,
Dithiophosphorsäure-[O-äthyl-S-(2,6-diäthoxy-4-pyrimidinylmethyl)-S-isopropyl]ester,
Dithiophosphorsäure-[O-äthyl-S-(n-butyl)-S-(2,6-diäthoxy-4-pyrimidinylmethyl]ester,
Dithiophosphorsäure-[S-(6-äthoxy-5-chlor-2-methyl-4-pyrimidinylmethyl)-O-äthyl-S-(n-propyl)]ester,
Dithiophosphorsäure-[O-äthyl-S-(5-chlor-2-isopropyl-6-äthoxy-4-pyrimidinylmethyl)-S-(n-propyl)]ester,
Dithiophosphorsäure-[O-äthyl-S-(6-äthylthio-2-iso-propyl-4-pyrimidinylmethyl)-S-(n-propyl)]ester,
Dithiophosphorsäure-[O-äthyl-S-(2,6-dimethyl-4-pyri-midinylmethyl)-S-(n-propyl)]ester,
Dithiophosphorsäure-[S-(2-äthoxy-6-methoxy-4-pyrimi-dinylmethyl)-O-äthyl-S-(n-propyl)]ester,
Dithiophosphorsäure-[O-äthyl-S-(6-dimethylcarbamoyloxy-2-isopropyl-4-pyrimidinylmethyl)-S-(n-propyl)]ester,
Dithiophosphorsäure-[O-äthyl-S-(2-cyclopropyl-6-dimethylcarbamoyloxy-4-pyrimidinylmethyl)-S-(n-propyl)]-ester,
Dithiophosphorsäure-[O-äthyl-S-(6-dimethylcarbamoyloxy-2-methoxy-4-pyrimidinylmethyl)-S-(n-propyl)]ester,
Dithiophosphorsäure-[O-äthyl-S-[6-(O,O-diäthyl-thio-phosphoro)-2-isopropyl-4-pyrimidinylmethyl]-S-(n-propyl)] ester,
Dithiophosphorsäure-[O-äthyl-S-[6-(O,O-dimethyl-thio-phosphoro)-2-diäthylamino-4-pyrimidinylmethyl]-S-(n-propyl)]ester,
Dithiophosphorsäure-[O-äthyl-S-(2-cyclopropyl-6-fluor-4-pyrimidinylmethyl)-S-(n-propyl)]ester,
Dithiophosphorsäure-[S-(2-äthoxy-6-fluor-4-pyrimi-dinylmethyl)-O-äthyl-S-(n-propyl)]ester,
Dithiophosphorsäure-[O-äthyl-S-(6-methoxy-2-methoxy-methyl-4-pyrimidinylmethyl)-S-(n-propyl)]ester,
Dithiophosphorsäure-[S-(6-äthoxy-2-phenyl-4-pyrimi-dinylmethyl)-O-äthyl-S-(n-propyl)]ester,
Dithiophosphorsäure-[O-äthyl-S-[6-(O,O-dimethyl-thio-phosphoro)-2-äthyl-4-pyrimidinylmethyl]-S-(n-propyl)]ester,
Dithiophosphorsäure-[S-(2-äthoxy-6-isopropoxy-4-pyrimidinylmethyl)-O-äthyl-S-(n-propyl)]ester,
Dithiophosphorsäure-[S-(2-äthoxy-4-pyrimidinylmethyl)-O-äthyl-S-(n-propyl)]ester,
Dithiophosphorsäure-[O-äthyl-S-(2-diäthylamino-6-methyl-4-pyrimidinylmethyl)-S-(n-propyl)]ester,
Dithiophosphorsäure-[S-(2-äthoxy-6-methylthio-4-pyrimidinylmethyl)-O-äthyl-S-(n-propyl)]ester,
Dithiophosphorsäure-[S-(2-äthoxy-6-isopropylthio-4-pyrimidinylmethyl)-O-äthyl-S-(n-propyl)]ester,
Dithiophosphorsäure-[S-(2,6-diäthoxy-4-pyrimidinyl-methyl)-O,S-di(n-propyl)]ester,
Dithiophosphorsäure-[O-äthyl-S-(6-fluor-4-pyrimidinyl-methyl)-S-(n-propyl)]ester,
Dithiophosphorsäure-[O-äthyl-S-(6-methyl-2-methoxy-4-pyrimidinylmethyl)-S-(n-propyl)]ester,
Dithiophosphorsäure-[S-(6-äthoxy-2-isopropoxy-4-pyrimidinylmethyl)-O-äthyl-S-(n-propyl)]ester,
Dithiophosphorsäure-[S-(6-äthoxy-2-äthylthio-4-pyri-midinylmethyl)-O-äthyl-S-(n-propyl)]ester,
Dithiophosphorsäure-[S-(6-äthoxy-2-dimethylamino-4-pyrimidinylmethyl)-O-äthyl-S-(n-propyl)]ester,
Dithiophosphorsäure-[S-(6-äthoxy-5-chlor-2-methylthio-4-pyrimidinylmethyl)-O-äthyl-S-(n-propyl)]ester,
Dithiophosphorsäure-[O-äthyl-S-(5-chlor-6-isopropoxy-2-methoxy-4-pyrimidinylmethyl)-S-(n-propyl)]ester,

Dithiophosphorsäure-[O-äthyl-S-(n-propyl)-S-(4-pyrimidinylmethyl)]ester,
Dithiophosphorsäure-[S-(2-äthoxy-6-chlor-4-pyrimi-dinylmethyl)-O-äthyl-S-(n-propyl)]ester,
Dithiophosphorsäure-[O-äthyl-S-(6-chlor-2-methoxy-4-pyrimidinyl)-S-(n-propyl)]ester,
Dithiophosphorsäure-[O-äthyl-S-[2-chlor-6-isopropoxy-4-pyrimidinylmethyl)-S-(n-propyl)]ester und
Dithiophos-phorsäure-[S-[2-äthoxy-6-(2-methoxyäthoxy)-4-pyrimidinylmethyl]-O-äthyl-S-(n-propyl)]ester.

Das erfindungsgemässe Verfahren zur Herstellung der Verbindungen der Formel I ist dadurch gekennzeichnet, dass

a) ein Pyrimidinderivat der allgemeinen Formel

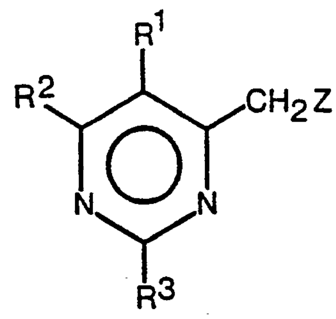

II

worin $R^1$, $R^2$ und $R^3$ die oben angegebenen Bedeutungen besitzen
und Z eine Abgangsgruppe, wie Chlor, Brom, Jod, Mesyloxy oder Tosyloxy, bedeutet,
mit einem Dithio- bzw. Trithiophosphat der allgemeinen Formel

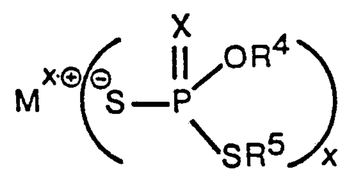

III

worin $R^4$, $R^5$ und X die oben angegebenen Bedeutungen besitzen,
$M^{x\oplus}$ für ein Alkalimetall-, Erdalkalimetalloder gegebenenfalls substituiertes Ammoniumion steht
und x die Wertigkeit des Kations $M^{x\oplus}$ bedeutet, umsetzt oder

b) ein Pyrimidinderivat der allgemeinen Formel

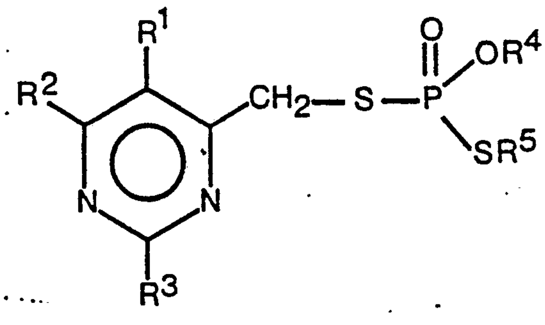

I'

worin $R^1$, $R^2$, $R^3$, $R^4$ und $R^5$ die oben angegebenen Bedeutungen besitzen,
mit Ausnahme der Derivate I' worin $R^2$

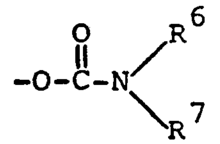 ist,

ist mit einem Schwefelungsmittel behandelt.

Beispiele von in der Verfahrensvariante a) verwendbaren Alkalimetall- bzw. Erdalkalimetallsalzen der Formel III sind das Natrium- und Kaliumsalz bzw. Calciumsalz. Das substituierte Ammoniumion kann beispielsweise Mono-, Dioder Tri(nieder alkyl)ammonium, wie tert. Butylammonium, Dimethylammonium oder Dimethyl-äthylammonium, sein.

Die Umsetzung gemäss Verfahrensvariante a) wird zweckmässigerweise in Gegenwart eines inerten Verdünnungsmittels, insbesondere eines inerten organischen Lösungsmittels, wie eines niederen aliphatischen Alkohols, z.B. Methanol oder Aethanol; eines niederen aliphatischen Ketons, z.B. Aceton oder Methyläthylketon; eines aliphatischen oder cyclischen Aethers, z.B. Diäthyläther, Glykolmethyläther oder -dimethyläther, Tetrahydrofuran oder Dioxan; eines halogenierten Kohlenwasserstoffes, z.B. Methylenchlorid, Chloroform oder 1,1,1-Trichloräthan; eines niederen aliphatischen Esters, z.B. Aethylacetat; eines niederen aliphatischen Nitrils, z.B. Acetonitril; eines Formamids, z.B. Dimethylformamid; oder eines aromatischen Kohlenwasserstoffes, z.B. Benzol oder Toluol; oder von Wasser, durchgeführt.

Die Reaktionstemperaturen können in einem grossen Bereich variieren, im allgemeinen wird jedoch zwischen 0° C und der Rückflusstemperatur des Reaktionsgemisches, vorzugsweise zwischen Raumtemperatur und 70° C, besonders bevorzugt zwischen 35° C und 50° C, gearbeitet.

Wird ein Pyrimidinderivat der allgemeinen Formel in der Z Chlor bedeutet, als Ausgangsmaterial verwendet, so kann die Umsetzung durch Zugabe einer katalytischen Menge eines Alkalimetalljodids, z.B. Natrium- oder Kaliumjodid (Finkelstein-Reaktion) oder eines Kupfer- oder Silbersalzes, z.B. Kupfer(I)-chlorid, Kupfer(II)-chlorid oder Silberacetat, beschleunigt werden.

Die Verfahrensvariante b) führt zu Verbindungen der Formel I, worin X Schwefel bedeutet. Zur Schwefelung der Derivate I' können Schwefelungsmittel wie Phosphorpentasulfid, allein oder in Gegenwart von Pyridin, der Phosphorpentasulfid-Pyridin (1:2)-Komplex und das Dimere von p-Methoxyphenylthiophosphinsulfid (siehe z.B. S.-0. Lawesson et al., Bull. Soc.Chim. Belg. 87, 229-238 (1978)) verwendet werden. Vorteilhaft wird in einem inerten organischen Verdünnungsmittel, wie einem Aromaten, z.B. Toluol, einem Xylol oder Pyridin, oder Hexamethylphosphorsäuretriamid, gearbeitet. Die Reaktionstemperaturen liegen zweckmässigerweise zwischen Raumtemperatur und dem Siedepunkt des Reaktionsgemisches, vorzugsweise zwischen Raumtemperatur und 80° C, besonders bevorzugt zwischen 45° C und 60° C.

Die Isolierung und die Reinigung der so erhaltenen Verbindungen der Formel I können in an sich bekannter Weise erfolgen.

Sofern keine gezielte Synthese zur Isolierung reiner optischer Isomerer durchgeführt wird, fällt normalerweise ein Produkt als Gemisch zweier oder mehrerer Isomerer an. Die Isomeren können nach an sich bekannten Methoden aufgetrennt werden. Gewünschtenfalls können allerdings die optischen Isomeren durch Einsatz entsprechender optisch aktiver Ausgangsmaterialien hergestellt werden.

Die als Ausgangsmaterialien verwendeten Pyrimidinderivate der Formel II sind entweder bekannt oder können nach an sich bekannten Methoden, z.B. gemäss T. Sakamoto et al., Chem. Pharm. Sull. 28, 3362-3368 (1980), DOS 2.703.310 und DOS 3.003.337, hergestellt werden. Bekannt sind insbesondere 4-Chlormethyl-2-isopropyl-6-methyl-pyrimidin (Chem. Pharm. Bull. 28, 3363), Thiophosphorsäure-[O-(6-chlormethyl-2-methoxymethyl-4-pyrimidinyl)-O,O-dimethyl]ester und Thiophosphorsäure-[O-(6-chlormethyl-2-methoxymethyl-4-pyrimidinyl)-O,O-diäthyl]ester [DOS 3.003.337, S. 11, Verbindungen (17) und (18)], deren Herstellung in diesen Literaturstellen beschrieben ist. Die 4-Halogenmethyl-2,6-dialkyl-pyrimidine und Thiophosphorsäure-[O-(2-alkoxymethyl oder -alkylthiomethyl-6-halogenmethyl-4-pyrimidinyl)-0,O-dialkyl]ester können generell gemäss den dort beschriebenen Verfahren hergestellt werden. Des weiteren kann man diejenigen Ausgangsmaterialien der Formel II, in der $R^1$ Wasserstoff, $R^2$ eine Gruppe (b), $R^3$ Wasserstoff, $C_{1-6}$-Alkyl, $C_{1-6}$-Alkoxy, $C_{1-6}$-Alkylthio oder $C_{1-4}$-Alkylamino und Z Chlor, Brom oder Jod bedeuten, nach dem in der DOS 2.703.310 beschriebenen Verfahren herstellen.

Aus DOS 3.118.700 ist ein Verfahren zur Herstellung derjenigen Ausgangsmaterialien der Formel II, in der $R^1$ Wasserstoff oder Chlor, eines der Symbole $R^2$ und $R^3$ Fluor und das andere Chlor, und Z Chlor bedeuten, bekannt geworden.

Diejenigen Ausgangsmaterialien der Formel II, in der $R^2$ und/oder $R^3$ $C_{1-6}$-Alkoxy, 2-Propenyloxy oder 2-Propinyloxy bedeutet, können beispielsweise durch direkte Alkyl-, Alkenyl- bzw. Alkinylierung des entsprechenden 2-Hydroxy-, 6-Hydroxy- oder 2,6-Dihydroxy-4-methyl-pyrimi-dinderivats der allgemeinen Formel

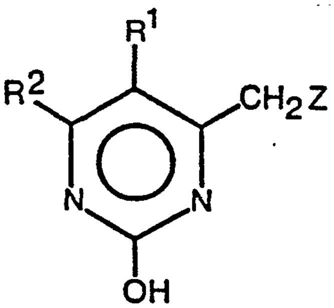 ; 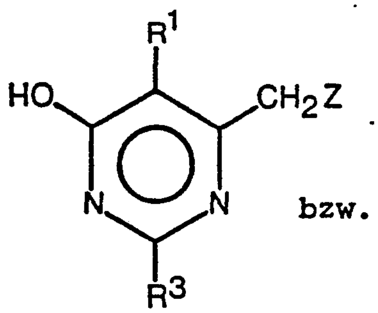 bzw. 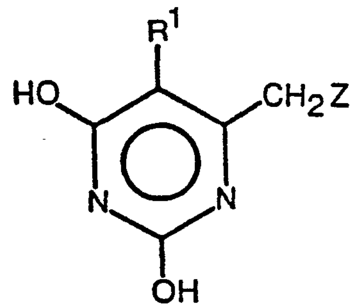

worin $R^1$, $R^2$, $R^3$ und Z die oben angegebenen Bedeutungen besitzen, hergestellt werden. Als Alkyl-, Alkenyl- und Alkinylierungsmittel eignen sich die entsprechenden aliphatischen Sulfate, wie Dialkylsulfate, z.B. Dimethylsulfat und Diäthylsulfat, aliphatischen Halogenide, z.B. Aethyljodid, Allylbromid und Propargylbromid, und Meerwein-Salze, z.B. Triäthyloxoniumtetraflorborat und Trimethyloxoniumtetrafluorborat. Zweckmässigerweise werden zudem ein Verdünnungsmittel, insbesondere ein organisches Lösungsmittel, wie ein Keton, z.B. Aceton, ein Nitril, z.B. Acetonitril, ein aliphatischer oder cyclischer Aether, z.B. Diäthyläther, Tetrahydrofuran oder Dioxan, ein Formamid, z.B. Dimethylformamid, oder N-Methylpyrrolidon, und ein säurebindendes Mittel, insbesondere eine Base, z.B. Natriumhydrid, Natriumhydroxid, Natriumcarbonat, Kaliumcarbonat, Kaliumtertiärbutylat oder Triäthylamin, verwendet. Gegebenenfalls kann die Umsetzung auch in einem Zwei-Phasen-System, wie Natriumhydroxidlösung/Chloroform oder Kaliumhydroxidlösung/Toluol, durchgeführt werden. Die Alkylierung, Alkenylierung bzw. Alkinylierung erfolgt im allgemeinen zwischen 0°C und der Rückflusstemperatur des Reaktionsgemisches. Wird die Alkylierung mit einem Meerwein-Salz durchgeführt, so arbeitet man vorzugsweise in der Schmelze oder bei der Rückflusstemperatur des entsprechenden Reaktionsgemisches, wobei das Lösungsmittel vorzugsweise ein halogenierter Kohlenwasserstoff, insbesondere Methylenchlorid, 1,1,2-Trichloräthan oder Chlorbenzol, ist.

Anstelle der oben erwähnten direkten Alkyl-, Alkenyl bzw. Alkinylierung des 2-Hydroxy-, 6-Hydroxy- oder 2,6-Dihydroxy-4-methyl-pyrimidinderivats kann dieses zunächst auch in das entsprechende 2-Chlor-, 6-Chlor- oder 2,6-Dichlor-4-methyl-pyrimidinderivat übergeführt werden und das chlorierte Produkt hierauf mit einem $C_{1-6}$-Alkanol, 2-Pro-penol oder 2-Propinol, zweckmässigerweise in Gegenwart von Natrium oder in Form des entsprechenden Natriumalkoxids, -alkenoxids bzw. -alkinoxids, zum erwünschten Pyrimidinderivat der Formel II, in der $R^2$ und/oder $R^3$ $C_{1-6}$-Alkoxy 2-Propenyloxy bzw. 2-Propinyloxy bedeutet, umgesetzt werden. Auf analoge Weise kann das 6-Chlor-4-methyl-pyrimidinderivat mit einem 2-($C_{1-4}$-Alkoxy)-äthanol oder einem gegebenenfalls halogenierten Phenol umgesetzt werden, um das erwünschte Pyrimidinderivat der Formel II, in der $R^2$ 2-($C_{1-4}$-Alkoxy)-äthoxy bzw. ggf. mit Halogen substituiertes Phenoxy bedeutet, herzustellen. Die Chlorierung und die Verätherung können unter dem Fachmann geläufigen Reaktionsbedingungen durchgeführt werden (siehe z.B. die europäische Patentpublikation Nr. 15.078).

Diejenigen Ausgangsmaterialien der Formel II, in der $R^2$ und/oder $R^3$ $C_{1-6}$-Alkylthio bedeutet, können ebenfalls aus den entsprechenden 2-Chlor-, 6-Chlor- bzw. 2,6-Dichlor-4-methyl-pyrimidinderivaten hergestellt werden, und zwar durch Umsetzung der letzteren mit einem $C_{1-6}$-Alkylmercap-tan. Diese Umsetzung kann unter dem Fachmann geläufigen Reaktionsbedingungen durchgeführt werden, insbesondere unter Verwendung eines Verdünnungsmittels und einer Base, wie oben bei der Alkylierung von 2-Hydroxy-pyrimidinderi-vaten erwähnt, und bei Temperaturen zwischen 0°C und der Rückflusstemperatur des jeweiligen Reaktionsgemisches. Steht Z in der Formel II für Chlor, Brom oder Jod, so ist die Gruppe $-CH_2Z$ vorerst zu schützen, vorzugsweise durch Austausch des Restes Z gegen eine Acyloxygruppe, insbesondere eine Acetyloxygruppe. Nach der Behandlung mit dem $C_{1-6}$-Alkylmercaptan wird die Schutzgruppe entfernt und die Gruppe $-CH_2Z$ nach an sich bekannten Methoden regeneriert. Beispielsweise wird das gegebenenfalls 2($R^3$)- und/oder 5($R^1$)-substituierte 4-Chlormethyl-6-hydroxy-pyrimidin mit Natriumacetat und einer katalytischen Menge Natriumjodid in Eisessig zum entsprechenden 4-Acetyloxymethyl-pyrimidin-derivat umgesetzt und dies anschliessend mittels Phosphoroxychlorid chloriert. Das resultierende geschützte 6-Chlor-pyrimidinderivat wird dann mit dem $C_{1-6}$-Alkylmercaptan behandelt. Zur Entfernung der Schutzgruppe wird das 4-Acetyl-oxymethyl-6-alkylthio-pyrimidinderivat mit Natriumhydroxid in Aethanol versetzt. Schliesslich wird zur Umwandlung der in der vorangegangenen Stufe erzeugten 4-Hydroxymethyl-gruppe in die gewünschte Chlormethylgruppe mit Thionylchlorid behandelt.

Analog dem oben beschriebenen Verfahren zur Herstellung der $C_{1-6}$-Alkylthiogruppen enthaltenden Ausgangsmaterialien der Formel II können die 2-Chlor-, 6-Chlor- und 2,6-Dichlor-4-methyl-pyrimidinderivate mit einem $C_{1-4}$-Alkylamin oder Di($C_{1-4}$-alkyl)amin umgesetzt werden, um zu den entsprechenden

Ausgangsmaterialien der Formel II, in der $R^2$ und/oder $R^3$ $C_{1-4}$-Alkylamino bzw. Di($C_{1-4}$-alkyl)amino bedeutet, zu gelangen. In diesem Fall ist angesichts des basischen Charakters des $C_{1-4}$-Alkylamins bzw. Di($C_{1-6}$-alkyl)amins der Einsatz einer zusätzlichen Base unnötig.

Die Ausgangsmaterialien der Formel II, in der $R^2$ $C_{1-4}$-Alkyl, $R^3$ $C_{1-6}$-Alkyl, $C_{3-6}$-Cycloalkyl, ($C_{1-4}$-Alkoxy)-methyl, $C_{1-6}$-Alkoxy, Di($C_{1-4}$-Alkyl)amino- oder gegebenenfalls mit Methoxy substituiertes Phenyl und Z Chlor bedeuten, können dadurch hergestellt werden, dass man das entsprechende 2-substituierte 4-Alkyl-6-hydroxy-pyrimidin durch Chlorierung in das entsprechende 6-Chlorpyrimidin-derivat überführt, dieses mittels Zink/Mineralsäure zum entsprechenden 6-unsubstituierten Pyrimidinderivat reduziert, das entstandene 2-substituierte 4-Alkylpyrimidin mit Ammoniumperoxydisulfat in Gegenwart von Methanol und verdünnter Schwefelsäure behandelt und schliesslich das Produkt, das entsprechende 2-substituierte 4-Alkyl-6-hydroxymethyl-pyrimidin, mittels Phosphoroxychlorid oder Thionylchlorid zum gewünschten 2-substituierten 4-Alkyl-6-chlormethyl-pyrimidin chloriert. Die beiden letzten Stufen dieser Synthese sind in Chem. Pharm. Bull. 28, 3362-3368 (1980) spezifisch exemplifiziert.

Diejenigen Ausgangsmaterialien der Formel II, in der $R^1$ Chlor bedeutet, können durch direkte Chlorierung des entsprechenden 5-unsubstituierten Pyrimidinderivats hergestellt werden. Zu diesem Zwecke kommen als Chlorierungsmittel insbesondere elementares Chlor, Sulfurylchlorid und N-Chlorsuccinimid in Frage. Die Chlorierung wird zweckmässigerweise in Gegenwart eines inerten Verdünnungsmittels, wie eines halogenierten Kohlenwasserstoffes, z.B. Methylenchlorid, Essigsäure oder Wasser, bei Temperaturen zwischen -20°C und 60°C, vorzugsweise zwischen 0°C und 45°C, durchgeführt. Auch wenn die 2-Stellung und/oder die 4-Stellung des Pyrimidinkerns unbesetzt ist, d.h. wenn $R^3$ und/oder $R^2$ Wasserstoff bedeutet, wird unter den dem Fachmann geläufigen Reaktionsbedingungen nur die 5-Stellung chloriert. Es kann auch aus dem oben erwähnten 2-Hydroxy-, 6-Hydroxy- oder 2,6-Dihydroxy-4-methyl-pyrimidinderivat, in dem die 5-Stellung unbesetzt ist, durch eine solche Chlorierung die entsprechende 5-Chlor-Verbindung hergestellt werden, und diese dann zwecks Herstellung entsprechender Alkoxy-, 2-Propenyloxy-, 2-Propinyloxy- bzw. Alkylthiogruppen enthaltender Ausgangsmaterialien der Formel II auf oben beschriebene Weise weiter umgesetzt.

Zur Herstellung derjenigen Ausgangsmaterialien der Formel II, in der $R^1$, $R^2$ und oder $R^3$ Wasserstoff bedeutet, können die entsprechenden Verbindungen der Formel II, in der $R^1$, $R^2$ und/oder $R^3$ Chlor bedeutet, wie oben für die 6-Chlorpyrimidinderivate ausgeführt, mit Zink/Mineralsäure unter dem Fachmann geläufigen Reaktionsbedingungen reduziert werden. Steht Z in der Formel II für Chlor, Brom oder Jod, so ist die Gruppe -$CH_2Z$ vorerst wiederum zu schützen, vorzugsweise durch Austausch von Z gegen eine Acyloxygruppe, insbesondere Acetyloxy, nach der reduktiven Behandlung die Schutzgruppe zu entfernen und die Gruppe -$CH_2Z$ nach an sich bekannten Methoden zu regenerieren, wie dies oben beschrieben ist.

Zur Herstellung der Ausgangsmaterialien der Formel II, in der $R^1$ und/oder $R^2$ Fluor bedeutet, können die entsprechenden Chlor-enthaltenden Verbindungen mit Natriumoder Kaliumfluorid umgesetzt werden, wobei der Austausch von Chlor gegen Fluor erfolgt. Die Umsetzung wird zweckmässigerweise in Gegenwart eines wasserfreien Verdünnungsmittels, wie eines aromatischen Kohlenwasserstoffes, z.B. eines Xylols; eines niederen aliphatischen Nitrils, z.B. Acetonitril; Tetramethylensulfon; Dimethylsulfoxid; Dimethylformamid; oder 1,3-Dimethyl-2-imidazolidinon, bei Temperaturen zwischen ca. 70°C und dem Siedepunkt des Reaktionsgemisches, vorzugsweise zwischen ca. 80°C und 140°C, durchgeführt. Zudem wird zweckmässigerweise unter Zuhilfenahme eines Phasentransfer-Katalysators, wie einer Polyäthylenäther-Kronenverbindung, z.B. 15-Crown-5 (Polyäthylenäther-Kronenverbindung mit einem 5 Sauerstoffatome enthaltenden 15-Ring) gearbeitet.

Zur Herstellung der Ausgangsmaterialien der Formel II, in der $R^3$ Fluor bedeutet, können die entsprechenden Chlorenthaltenden Verbindungen mit wasserfreier Flußsäure behandelt werden, wobei der Austausch von Chlor gegen Fluor erfolgt. Die Umsetzung kann zweckmässigerweise unter Verwendung der Flußsäure auch als Lösungsmittel in einem Autoklaven unter erhöhtem Druck durchgeführt werden, wie z.B. dies in der DOS 3.118.700 beschrieben ist.

Weitere Möglichkeiten der Herstellung von Ausgangsmaterialien der Formel II sind die Carbamoylierung und die Phosphorylierung der oben erwähnten 6-Hydroxy-4-methyl-pyrimidinderivate mit Dialkyl(thio)carbamoylhalogeniden bzw. Thiophosphorsäureesterhalogeniden der allgemeinen Formeln

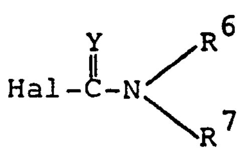

und

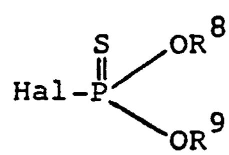

.

worin $R^6$, $R^7$, $R^8$, $R^9$ und Y die oben angegebenen Bedeutungen besitzen und Hal für Halogen, insbesondere für Chlor, steht, zu den entsprechenden Pyrimidinderivaten der Formel II, in der $R^2$ eine Gruppe (a) bzw. (b) bedeutet. Auch die Carbamoylierung und die Phosphorylierung können unter dem Fachmann geläufigen Reaktionsbedingungen durchgeführt werden.

Die bei der Herstellung dieser Ausgangsmaterialien II benötigten 2-Hydroxy-, 6-Hydroxy- und 2,6-Dihydroxy-4-methyl-pyrimidinderivate sind entweder bekannt oder können nach an sich bekannten Methoden hergestellt werden. Unter den bekannten derartigen Derivaten seien z.B. 4-Chlormethyl-uracil (siehe Angew. Chem. 80, 405-406 (1968); 2-Isopropyl-, 2-Methyl-, 2-Isopropyl-5-chlor-, 2-Phenyl- und 2-Methyl-5-chlor-4-hydroxy-6-chlormethyl-pyrimidin sowie 6-Chlor-methyl-pyrimidinol (siehe DOS 2.167.194) und 4-Chlormethyl-6-hydroxy-2-methylthio-pyrimidin (siehe J. Org. Chem. 27, 3545-3549 (1962) erwähnt.

Die 2-Alkyl-5-chlor-4-chlormethyl-6-hydroxy-pyrimidine und die entsprechenden 2- und/oder 5-unsubstituierten Pyrimidinderivate sind allgemein gemäss demin der DOS 2.167.194 beschriebenen Verfahren zugänglich. Die Herstellung von 4-Chlormethyl-6-hydroxy-2-methylthio-pyrimidin ist in J. Org. Chem. 27, 3549 (1962) beschrieben. Auf analoge Weise können schliesslich die bis anhin unbekannten 2-Alkoxy- und 2-Dialkylamino-4-chlormethyl-6-hydroxy-pyrimi-dine und das 4-Chlormethyl-6-hydroxy-pyrimidin hergestellt werden, und zwar aus dem entsprechenden O-Alkylisoharnstoff, N,N-Dialkylguanidin oder Formamidin oder einem Salz davon und γ-Chloracetessigsäurechlorid oder einem γ-Chloracetessigsäure-alkylester, insbesondere dem Methyl- oder Aethylester.

Die als Ausgangsmaterialien verwendeten Dithio- bzw. Trithiophosphate der Formel III sind entweder bekannt oder können nach an sich bekannten Methoden, z.B. gemäss Houben-Weyl, Bd. XII/2, S. 689-690 und DOS 2.506.618, hergestellt werden.

Bei den Ausgangsmaterialien der Formel I' handelt es sich um diejenigen Pyrimidinderivate der Formel I, in der X Sauerstoff bedeutet, mit Ausnahm solcher Derivate, worin $R^2$

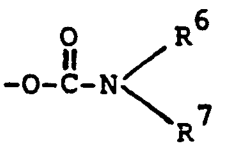 ist.

Die Verbindungen der Formel I sind ganz allgemein als Pestizide von Wert. Sie zeigen sich besonders wertvoll zur Bekämpfung von Insekten, Milben und Nematoden, insbesondere gegen

- Coleoptera wie z.B. Epilachna spp., Leptinotarsa decemlineata, Anthonomus spp., Conotrachelus nenuphar, Lema spp., Lissorhoptrus oryzaephilus, Phyllotreta spp., Psylliodes chrysocephala, Melingetes aenus, Ceutorrhynchus assimilis, Otiorhynchus sulcatus, Melolontha melolontha und Diabrotica spp.
- Lepoidoptera wie z.B. Laspeyresia spp., Adoxophyes orana, Tortrix viridana, Cheimatobia brumata, Lyonetia clerkella, Operophtera brumata, Lithocolletis blancardella, Ephestia kuehniella, Mamestra brassicae, Agrotis segetum, Plutella spp., Pieris brassicae, Choristoneura fumiferana, Heliothis spp., Spodoptera spp., Earias insulana, Pectinophora gossipiella, Chilo spp., Ostrinia nubilalis, Clysia ambiguella, Lobesia botrana.
- Diptera wie z.B. Drosophila melanogaster, Ceratitis spp., Oscinella frit, Dacus spp. und Rhagoletis spp., Leatherjacket spp., Sciara spp. und Phorbia spp.
- Homoptera, d.h. Blattläuse wie z.B. Aphis fabae, Myzus persicae und weitere Arten dieser Gattungen, Rhophalosiphon spp., Schizaphis spp. sowie Schild- und Schmierläuse wie z.B. Saissetia spp., Quadraspidiotus perniciosus, Aonidiella aurantii, Planococcus spp. sowie Zikaden wie z.B. Nephotettix spp., Laodelphax spp. und Nilaparvata spp., Aleurodidae wie z.B. Trialeurodes vaporariorum, Bemisia spp., ausserdem Trips-Arten.
- Heteroptera wie z.B. Dysdercus spp. und Lygus spp.
- Acarina wie z.B. Tetranychus urticae, Panonychus ulmi und andere Tetranychiden, Eriophyiden wie Phyllocoptruta oleivora, Aceria sheldoni, Eriophyes spp., Aceria spp. und ferner Zecken.
- Nematoda wie z.B. freilebende Nematoden (u.a. Pratylenchus spp. wie P. penetrans), blattparasitische Nematoden (u.a. Aphelenchoides) und wurzelparasitische Nematoden (u.a. Meloidogynae spp. wie M. incognita, Globodera spp. wie G. rostochiensis).

Die erfindungsgemässen Verbindungen wirken als Kontaktund Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Das erfindungsgemässe Schädlingsbekämpfungsmittel ist dadurch gekennzeichnet, dass es eine wirksame Menge mindestens einer Verbindung der allgemeinen Formel I, wie oben definiert, sowie Formulierungshilfsstoffe enthält. Das Mittel enthält zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Unter Verwendung solcher und anderer Hilfsstoffe können die Verbindungen der Formel I, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage: natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie

Cellulose, Stärke, Harnstoff und Kunstharze; und Düngemittel, wie Phosphate und Nitrate wobei solche Trägerstoffe z.B. als Stäube, Pulver oder Granulate vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-Dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsaureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber z.B. substituierte Benzophenone, Diphenylacrylnitrilsäureester und Zimtsäureester; und Deaktivatoren z.B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Herbizide, Pflanzenwachstumsregulatoren und Düngemittel.

Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Die erfindungsgemässen Schädlingsbekämpfungsmittel enthalten im allgemeinen zwischen 0,0005 und 95 Gewichtsprozent der Verbindung bzw. Verbindungen der Formel I als Wirkstoff, vorzugsweise zwischen 1 und 75 Gewichtsprozent. Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 10 bis 75 Gewichtsprozent, der Verbindung bzw. Verbindungen der Formel I. Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z.B. Konzentrationen zwischen 0,0005 und 20 Gewichtsprozent vorliegen. Im Ultra-Low-Volume-Verfahren können Spritzbrühen formuliert werden, in denen die Wirkstoffkonzentration vorzugsweise von 10 bis 20 Gewichtsprozent beträgt, während die im Low-Volume-Verfahren und im High-Volume-Verfahren formulierten Spritzbrühen vorzugsweise eine Wirkstoffkonzentration von 0,5 bis 1,0 bzw. 0,05 bis 0,1 Gewichtsprozent aufweisen. Granulate, die insbesondere in der Moskito-Bekämpfung eingesetzt werden, enthalten vorzuqsweise von 2 bis 10 Gewichtsprozent der Verbindung bzw. Verbindungen der Formel I als Wirkstoff.

Die erfindungsgemässen Schädlingsbekämpfungsmittel können dadurch hergestellt werden, dass man mindestens eine Verbindung der allgemeinen Formel I mit Formulierungshilfsstoffen vermischt.

Die Herstellung der Mittel kann in bekannter Weise durchgeführt werden, z.B. durch Vermischen des

Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt.

Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Bodenoder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Die nachfolgenden Beispiele dienen zur näheren Erläuterung der Erfindung.

**I. Herstellung der Wirkstoffe der Formel I:**

**Beispiel 1**

8,0 g (0,043 Mol) 6-Aethoxy-4-chlormethyl-2-methyl-pyrimidin und 10,8 g (0,045 Mol) O-Aethyl-S-(n-propyl)dithiophosphorsäure-Kaliumsalz werden in 20 ml Aceton suspendiert. Nach Zugabe einer kleinen Menge (Spatelspitze) Natriumjodid wird das Reaktionsgemisch während 12 Stunden bei 45°C gut gerührt. Das Reaktionsgemisch wird alsdann durch Celite filtriert, das Filtrat unter vermindertem Druck eingeengt und der so isolierte Feststoff in 100 ml Toluol aufgenommen. Man wäscht die Toluollösung je einmal mit 5% Natriumcarbonatlösung und gesättigter Natriumchloridlösung, trocknet sie über wasserfreiem Natriumsulfat und dampft die Lösungsmittel bei 60°C unter vermindertem Druck ab. Auf diese Weise erhält man den Dithiophosphor-säure-[O-äthyl-S-(6-äthoxy-2-methyl-4-pyrimidinylmethyl)-S-(n-propyl)]ester als fast farbloses Oel, $n^{20}_D$ 1,5385.

**Beispiel 2**

Zu einer Lösung von 15 g (0,070 Mol) 4-Chlormethyl-2,6-diäthoxy-pyrimidin und 18 g (0,070 Mol) O-Aethyl-S(n-propyl)-dithiophosphorsäure-Dimethylammoniumsalz in 40 ml Aethanol wird eine kleine Menge (Spatelspitze) Natriumjodid zugegeben, und das Reaktionsgemisch wird bei 45-50°C während 16 Stunden gerührt. Anschliessend wird das Reaktionsgemisch in 150 ml Toluol aufgenommen, und die Toluollösung wird je einmal mit gesättigter Natriumbicarbonatlösung und gesättigter Natriumchloridlösung gewaschen und über wasserfreiem Natriumsulfat getrocknet. Man dampft die Lösungsmittel unter vermindertem Druck ab und filtriert das entstandene leicht rötliche Oel durch Kieselgel. Auf diese Weise erhält man den Dithiophosphorsäure-[O-äthyl-S-(2,6-diäthoxy-4-pyrimidinylmethyl)-S-(n-propyl)]ester als fast farbloses Oel, $n^{26}_D$ 1,5333.

**Beispiele 3-55**

Analog dem in Beispiel 1 oder 2 beschriebenen Verfahren werden die entsprechenden Ausgangsmaterialien der Formeln II und III umgesetzt, um die in den nachstehenden Tabellen 1 und 2 aufgeführten Verbindungen der Formel I, insbesondere der unten angegebenen Formeln Ia und Ib, herzustellen.

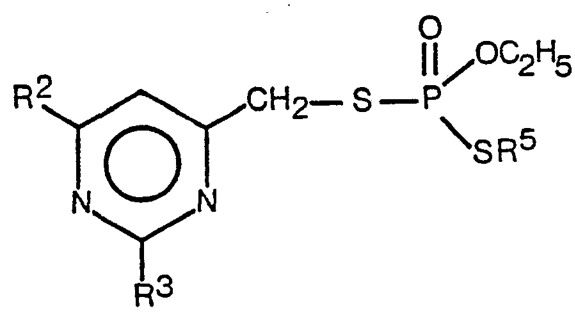

Ia

**Tabelle 1**

| Beisp. | $R^2$ | $R^3$ | $R^5$ | Physikalische Angabe (n) |
|---|---|---|---|---|
| 3 | $OC_2H_5$ | $CH_3$ | sek. $C_4H_9$ | $n_D^{25} = 1,5360$ |
| 4 | $OC_2H_5$ | 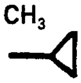 | $nC_3H_7$ | $n_D^{20} = 1,5483$ |
| 5 | Cl | tert. $C_4H_9$ | $nC_3H_7$ | $n_D^{20} = 1,5414$ |
| 6 | $OC_2H_5$ | $OC_2H_5$ | sek. $C_4H_9$ | $n_D^{20} = 1,5354$ |
| 7 | $OC_2H_5$ | tert. $C_4H_9$ | $nC_3H_7$ | $n_D^{22} = 1,5241$ |
| 8 | $OCH_3$ | tert. $C_4H_9$ | $nC_3H_7$ | $n_D^{24} = 1,5234$ |
| 9 | $N(CH_3)_2$ | Cl | $nC_3H_7$ | Oel<br>$^1$H-NMR ($CDCl_3$):<br>3,15 (s, N $(C\underline{H}_3)_2$),<br>3,96 (d, $C\underline{H}_2SP$),<br>6,52 (s, $C\underline{H}$); |
| 10 | Cl | Cl | $nC_3H_7$ | $n_D^{23} = 1,5702$ |
| 11 | $OCH_3$ | Cl | $nC_3H_7$ | $n_D^{23} = 1,5598$ |
| 12 | Cl | iso$C_3H_7$ | $nC_3H_7$ | $n_D^{21} = 1,5473$ |
| 13 | $OC_2H_5$ | iso$C_3H_7$ | $nC_3H_7$ | $n_D^{21} = 1,5290$ |
| 14 | $OCH_3$ | $OCH_3$ | $nC_3H_7$ | $n_D^{21} = 1,5469$ |
| 15 | Cl | $C_2H_5$ | $nC_3H_7$ | $n_D^{23} = 1,5513$ |
| 16 | $OCH_2CH_3$ | $C_2H_5$ | $nC_3H_7$ | $n_D^{23} = 1,5346$ |
| 17 | $OCON(CH_3)_2$ | $CH_3$ | $nC_3H_7$ | $n_D^{27} = 1,5408$ |
| 18 | $OP(S)(OCH_3)_2$ | $CH_3$ | $nC_3H_7$ | $n_D^{22} = 1,5459$ |
| 19 | $OC_2H_5$ | $SCH_3$ | $nC_3H_7$ | $n_D^{22} = 1,5612$ |
| 20 | $SC_2H_5$ | 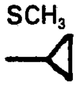 | $nC_3H_7$ | $n_D^{24} = 1,5741$ |
| 21 | iso$C_3H_7O$ | iso$C_3H_7O$ | $nC_3H_7$ | $n_D^{23} = 1,5272$ |
| 22 | iso$C_3H_7O$ | iso$C_3H_7$ | $nC_3H_7$ | $n_D^{22} = 1,5246$ |
| 23 | $OCH_2C{\equiv}CH$ | iso$C_3H_7$ | $nC_3H_7$ | $n_D^{23} = 1,5396$ |
| 24 | $OCH_2CH{=}CH_2$ | iso$C_3H_7$ | $nC_3H_7$ | $n_D^{25} = 1,5332$ |
| 25 | $OC_2H_5$ | Cl | $nC_3H_7$ | $n_D^{24} = 1,5422$ |

| Beisp. | $R^2$ | $R^3$ | $R^5$ | Physikalische Angabe (n) |
|---|---|---|---|---|
| 26 | $SC_2H_5$ | $SC_2H_5$ | $nC_3H_7$ | $n_D^{26} = 1{,}5914$ |
| 27 | $OC_2H_5$ | $-C_6H_4-Cl-p$ | $nC_3H_7$ | $n_D^{23} = 1{,}5840$ |
| 28 | $OP(S)(OC_2H_5)_2$ | $-C_6H_4-Cl-p$ | $nC_3H_7$ | oel $^1H$-NMR ($CDCl_3$): 4,23 (d,$C\underline{H}_2SP$), 7,03 (s,$C\underline{H}$), 7,47 (d,$C\underline{H}$), 8,43 (d,$C\underline{H}$) |
| 29 | $OC_2H_5$ | $OC_2H_5$ | $C_2H_5$ | $n_D^{20} = 1{,}5323$ |
| 30 | $OCSN(CH_3)_2$ | $CH_3$ | $nC_3H_7$ | Oel $^1H$-NMR ($CDCl_3$): 2,78 (s, $C\underline{H}_3$), 3,08 (s,$N(C\underline{H}_3)_2$), 4,12 (d,$C\underline{H}_2SP$), 7,87 (s, $C\underline{H}$) |
| 31 | $OC_6H_5$ | $CH_3$ | $nC_3H_7$ | $n_D^{25} = 1{,}5749$ |
| 32 | $N(CH_3)_2$ | $CH_3$ | $nC_3H_7$ | Oel $^1H$-NMR ($CDCl_3$): 2,50 (s, $C\underline{H}_3$), 3,10 (s,$N(C\underline{H}_3)_2$), 3,97 (d,$C\underline{H}_2SP$), 6,40 (s, $C\underline{H}$) |
| 33 | $OCON(CH_3)_2$ | $OC_2H_5$ | $nC_3H_7$ | $n_D^{24} = 1{,}5410$ |
| 34 | Cl | $N(C_2H_5)_2$ | $nC_3H_7$ | $n_D^{24} = 1{,}5623$ |
| 35 | Cl | $NHC_3H_7$iso | $nC_3H_7$ | $n_D^{24} = 1{,}5659$ |
| 36 | $N(C_2H_5)_2$ | Cl | $nC_3H_7$ | $n_D^{24} = 1{,}5684$ |
| 37 | $NHC_3H_7$iso | Cl | $nC_3H_7$ | $n_D^{25} = 1{,}5689$ |
| 38 | $OCH_2C{\equiv}CH$ | iso$C_3H_7$ | sek.$C_4H_9$ | $n_D^{24} = 1{,}5367$ |
| 39 | $OC_2H_5$ | $OCH_3$ | $nC_3H_7$ | $n_D^{20} = 1{,}5424$ |
| 40 | $OCH_2C{\equiv}CH$ | $OCH_3$ | $nC_3H_7$ | $n_D^{25} = 1{,}5536$ |
| 41 | $N(C_2H_5)_2$ | $OCH_3$ | $nC_3H_7$ | $n_D^{25} = 1{,}5528$ |
| 42 | $OC_2H_5$ | $N(C_2H_5)_2$ | $nC_3H_7$ | Oel $^1H$-NMR ($CDCl_3$): 3,70 (q, $N(C\underline{H}_2)-CH_3)_2$), 3,90 (d, $C\underline{H}_2SP$), 4,33 (q, $OC\underline{H}_2CH_3$), 5,95 (s, $C\underline{H}$) |
| 43 | iso$C_3H_7O$ | $OCH_3$ | $nC_3H_7$ | Oel $^1H$-NMR ($CDCl_3$): 1,37 (d, $CH(C\underline{H}_3)_2$), 4,00 (s, $OC\underline{H}_3$), 4,03 (d, $C\underline{H}_2SP$), 5,39 (m, $C\underline{H}(CH_3)_2$), 6,42 (s, $C\underline{H}$) |
| 44 | $OCH_3$ | H | $nC_3H_7$ | $n_D^{25} = 1{,}5465$ |
| 45 | $OC_2H_5$ | H | $nC_3H_7$ | $n_D^{25} = 1{,}5400$ |
| 46 | $OCH_2C{\equiv}CH$ | H | $nC_3H_7$ | $n_D^{25} = 1{,}5541$ |
| 47 | $OC_6H_5$ | H | $nC_3H_7$ | $n_D^{25} = 1{,}5784$ |
| 48 | $CH_3$ | iso$C_3H_7$ | $nC_3H_7$ | $n_D^{24} = 1{,}5353$ |

| Beisp. | $R^2$ | $R^3$ | $R^5$ | Physikalische Angabe (n) |
|---|---|---|---|---|
| 49 | $OCH_2CH_2OCH_3$ | $isoC_3H_7$ | $nC_3H_7$ | Oel<br>$^1H$-NMR ($CDCl_3$):<br>1,28 (d, $CH(CH_3)_2$),<br>3,07 (m, $CH(CH_3)_2$),<br>3,40 (s, $OCH_3$),<br>3,75 (m, $OCH_2$),<br>4,07 (d, $CH_2SP$),<br>4,56 (m,$OCH_2$),<br>6,70 (s,CH) |
| 50 | F | $isoC_3H_7$ | $nC_3H_7$ | Oel<br>$^1H$-NMR ($CDCl_3$):<br>1,33 (d, $CH(CH_3)_2$),<br>3,18 (m, $CH(CH_3)_2$),<br>4,20 (d, $CH_2SP$),<br>6,98 (d,$J_{HF}$=2Hz,H) |
| 51 | F | Cl | $nC_3H_7$ | $^1H$-NMR ($CDCl_3$):<br>4,17 (d, $CH_2SP$),<br>7,20 (d, $J_{HF}$=2Hz,<br>H); $n_D^{22}$ = 1,5410 |
| 52 | $OC_3H_7iso$ | $OC_2H_5$ | $nC_3H_7$ | $n_D^{21}$ = 1,5360 |

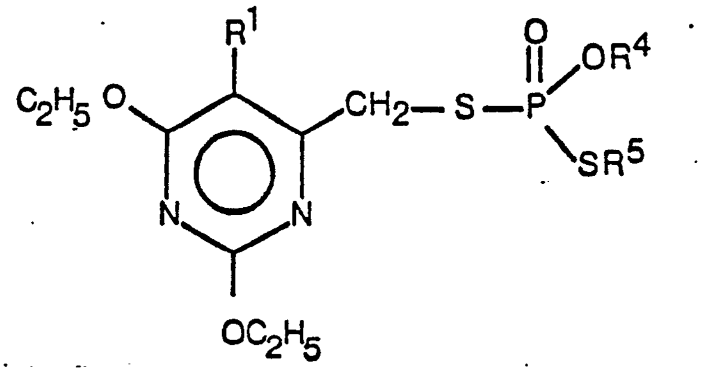

Ib

**Tabelle 2**

| Beisp. | $R^1$ | $R^4$ | $R^5$ | Physikalische Angabe (n) |
|---|---|---|---|---|
| 53 | H | $CH_3$ | $CH_3$ | Oel<br>$^1H$-NMR ($CDCl_3$): 2,10 (d, $CH_3SP$), 3,73 (d, $CH_3OP$), 4,00 (d, $CH_2SP$), 4,42 (q,$OCH_2CH_3$), 6,43 (s, CH) |
| 54 | H | $CH_3$ | $nC_3H_7$ | $n_D^{26}$ = 1,5412 |
| 55 | Cl | $C_2H_5$ | $nC_3H_7$ | $n_D^{24}$ = 1,5449 |

**Beispiel 56**

2 g (0,053 Mol) Dithiophosphorsäure-[O-äthyl-S-(2 6-diäthoxy-4-pyrimidinylmethyl)-S-(n-propyl)]ester (siehe Beispiel 2) und 0,3 g (0,0145 Mol) Phosphorpentasulfid in 10 ml Toluol werden 16 Stunden bei 60°C belassen. Man verdünnt dann das Reaktionsgemisch mit 50 ml Toluol und wäscht die Lösung einmal mit 0,5N Salzsäure und zweimal mit gesättigter Natriumchloridlösung. Anschliessend wird das Lösungsmittel unter vermindertem Druck abgedampft und das entstandene Rohprodukt an Kieselgel mit 15% Diäthyläther in n-Hexan chromatographisch gereinigt. Man erhält den Trithiophosphorsäure-[O-äthyl-S-(2,6-diäthoxy-4-pyrimidinylmethyl)-S-(n-propyl)]ester als gelbes Oel, $n_D^{23}$ 1,5648.

**Beispiel 57**

Analog dem in Beispiel 56 beschriebenen Verfahren wird Dithiophosphorsäure-[O-äthyl-S-(2,6-dichlor-4-pyrimidinylmethyl)-S-(n-propyl)]ester (siehe Beispiel 10) mit Phosphorpentasulfid geschwefelt, um den Trithiophosphor-säure-[O-äthyl-S-(2,6-dichlor-4-pyrimidinylmethyl)-S-(n-propyl)]ester, $n_D^{23}$ 1,6063, herzustellen.

**Beispiel 58**

Analog dem im Beispiel 56 beschriebenen Verfahren wird Dithiophosphorsäure-[S-(6-äthoxy-2-methyl-4-pyrimidinylmethyl)-O-äthyl-S-(sek. butyl)]ester (siehe Beispiel 3) mit Phosphorpentasulfid geschwefelt, um den Trithiophosphorsäure, [S-(6-äthoxy-2-methyl-4-pyrimidinylmethyl)-O-äthyl-S-(sek. butyl)]ester, $n^{20}_D$ 1,5691, herzustellen.

**II. Herstellung der Ausgangsmaterialien:**

**Beispiel 59**

Das als Ausgangsmaterial in den Beispielen 1 und 3 benötigte 6-Aethoxy-4-chlormethyl-2-methyl-pyrimidin kann wie folgt hergestellt werden:

15,8 g (0,1 Mol) 6-Chlormethyl-4-hydroxy-2-methyl-pyrimidin, 13 ml (0,1 Mol) Diäthylsulfat sowie 27,6 g (0,2 Mol) Kaliumcarbonat werden in 500 ml Aceton während 6 Stunden bei Rückflusstemperatur erhitzt. Alsdann wird das Reaktionsgemisch filtriert und das Filtrat zur Trockene eingeengt. Der Rückstand wird in 200 ml Methylenchlorid aufgenommen und die Methylenchloridlösung einmal mit 50 ml 2N Natronlauge und zweimal mit 50 ml halbgesättigter Natriumchloridlösung gewaschen, über wasserfreiem Natriumsulfat getrocknet und unter vermindertem Druck eingedampft. Das zurückbleibende Oel wird bei 47°C/0,07 mmHg (9,33 Pa) destilliert, worauf das Destillat erstarrt. Man erhält das 6-Aethoxy-4-chlormethyl-2-methyl-pyrimidin, Smp. 29°C.

**Beispiel 60**

Das als Ausgangsmaterial in den Beispielen 2, 6, 29, 53 und 54 benötigte 4-Chlormethyl-2,6-diäthoxy-pyrimidin kann wie folgt hergestellt werden:

16,1 g (0,1 Mol) 6-Chlormethyl-uracil und 57,0 g (0,3 Mol) Triäthyloxonium-tetrafluorborat werden in 100 ml 1,1,2-Trichloräthan suspendiert, und die Suspension wird während 18 Stunden bei 90°C belassen. Anschliessend wird das Reaktionsgemisch abgekühlt und tropfenweise mit einer 50%-igen wässrigen Lösung von 41,5 g (0,3 Mol) Kaliumcarbonat versetzt, wobei anfänglich starke Entwicklung von Kohlenstoffdioxid beobachtet wird. Man dekantiert dann vom ausgefallenen schlammartigen Rückstand ab und extrahiert diesen mehrmals mit kleinen Mengen Methylenchlorid. Die vereinigten organischen Phasen werden mit Wasser gewaschen, über wasserfreiem Natriumsulfat getrocknet und unter vermindertem Druck eingedampft. Das Rohprodukt wird in heisses n-Hexan aufgenommen und die Lösung mit Aktivkohle behandelt und durch Filtration von unlöslichen Anteilen befreit. Beim Einengen des Filtrats erhält man das 4-Chlormethyl-2,6-diäthoxy-pyrimidin, Smp. 38-39°C.

**Beispiel 61**

Das als Ausgangsmaterial in Beispiel 12 benötigte 6-Chlor-4-chlormethyl-2-isopropyl-pyrimidin kann wie folgt hergestellt werden:

12,1 g (0,065 Mol) 4-Chlormethyl-6-hydroxy-2-isopropyl-pyrimidin werden in 50 ml Phosphoroxychlorid suspendiert, und die Suspension wird während 30 Minuten unter Stickstoff auf 80°C erwärmt, wobei das kristalline Ausgangsmaterial allmählich gelöst wird. Unter vermindertem Druck wird alsdann bei 40-60°C das überschüssige Phosphoroxychlorid abdestilliert. Man giesst den Rückstand auf Eiswasser, bringt durch Zugabe von Natronlauge den pH-Wert des Gemisches auf 7 und extrahiert es dreimal mit Diäthyläther. Die vereinigten organischen Extrakte werden über wasserfreiem Natriumsulfat getrocknet und unter vermindertem Druck eingedampft, und der dunkelfarbige ölige Rückstand wird in heisses n-Hexan aufgenommen und die Lösung mit Aktivkohle behandelt. Nach Filtration des heissen Gemisches durch Celite erhält man das 6-Chlor-4-chlormethyl-2-iso-propyl-pyrimidin als oranges Oel, das gemäss Dünnschichtchromatographie rein ist. Bei der Destillation dieses Oels unter vermindertem Druck erhält man eine farblose Flüssigkeit, Sdp. 75°C/0,05 mmHg, (6,67 Pa) $n_D^{20}$ 1,5226.

**Beispiel 62**

Das als Ausgangsmaterial in Beispiel 13 benötigte 6-Aethoxy-4-chlormethyl-2-isopropyl-pyrimidin kann wie folgt hergestellt werden:

Eine Lösung von 9,6 g (0,047 Mol) 6-Chlor-4-chlor-methyl-2-isopropyl-pyrimidin (siehe Beispiel 61) in 40 ml absolutem Aethanol wird während 3 Stunden bei 0°C tropfenweise mit einer Lösung von 1,24 g (0,054 Mol) Natrium in Aethanol versetzt, und das Gemisch wird 3 Stunden bei 0°C und dann 16 Stunden bei Raumtemperatur gerührt. Man nimmt alsdann das Reaktionsgemisch in Diäthyläther auf, wäscht die Lösung neutral mit Natriumchloridlösung, trocknet sie über wasserfreiem Natriumsulfat und setzt sie unter vermindertem Druck vom Lösungsmittel frei. Nach Destillieren des öligen Rückstandes am Hochvakuum erhält man das 6-Aethoxy-4-chlormethyl-2-isopropyl-pyrimidin, Sdp. 52°C/ 0,07 mmHg (9,33 Pa), $n^{21}_D$ 1,4955.

**Beispiel 63-71**

Analog dem in Beispiel 59 oder 60 beschriebenen Verfahren oder den in den Beispielen 61 und 62 beschriebenen Verfahren werden aus dem entsprechenden 2-Alkyl-, 2-Cyclo-alkyl-, 2-Aryl- oder 2-Alkoxy-4-chlormethyl-6-hydroxy-pyrimidin bzw. aus 4-Chlormethyl-6-hydroxy-pyrimidin die in der nachstehenden Tabelle 3 aufgeführten Ausgangsmaterialien der Formel II, insbesondere der unten angegebenen Formel IIa, hergestellt. Auch die entsprechenden Endprodukte der Formel I sind in dieser Tabelle angegeben.

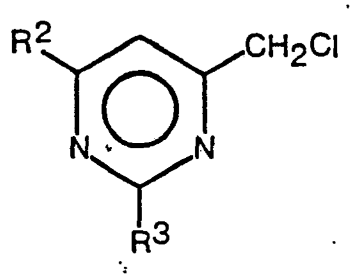

IIa

Tabelle 3

| Beisp. | $R^2$ | $R^3$ | Physikalische Angabe (n) | Beispiel-Nr. des Endproduktes der Formel I |
|---|---|---|---|---|
| 63 | $OC_2H_5$ | 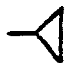 | <(1,33 Pa)> Sdp. 74°C/0,01 mmHg <->, $n_D^{20}$ = 1,5270 | 4 |
| 64 | $OC_2H_5$ | tert. $C_4H_9$ | Oel $^1$H-NMR ($CDCl_3$): 1,37 (s, $C(C\underline{H}_3)_3$, 1,40 (t, $C\underline{H}_3$), 4,44 (q, $C\underline{H}_2$), 4,50 (s, $C\underline{H}_2Cl$), 6,69 (s, $C\underline{H}$) | 7 |
| 65 | $OCH_3$ | tert. $C_4H_9$ | Sdp. 60°C/0,02 mmHg (2,67 Pa) $n_D^{23}$ = 1,4933 | 8 |
| 66 | $OC_2H_5$ | $C_2H_5$ | Oel $^1$H-NMR ($CDCl_3$): 1,30+1,39 (tt, 2×$C\underline{H}_3$), 2,75+4,42 (qq, 2×$C\underline{H}_2$), 4,50 (s, $C\underline{H}_2Cl$), 6,71 (s, $C\underline{H}$) | 16 |
| 67 | $OC_2H_5$ | $-C_6H_4$-Cl-p | Smp. 83–84,5°C | 27 |
| 68 | $OC_2H_5$ | $OCH_3$ | — (Oel) | 39 |
| 69 | iso$C_3H_7O$ | $OCH_3$ | Oel $^1$H-NMR ($CDCl_3$): 1,35 (d, $CH(C\underline{H}_3)_2$), 3,97 (s, $OC\underline{H}_3$), 4,44 (s, $C\underline{H}_2Cl$), 5,40 (m, $C\underline{H}(CH_3)_2$), 6,50 (s, $C\underline{H}$) | 43 |
| 70 | $OCH_3$ | H | Oel $^1$H-NMR ($CDCl_3$): 4,00 (s, $OC\underline{H}_3$), 4,53 (s, $C\underline{H}_2Cl$), 6,98 (s, $C\underline{H}$), 8,75 (s, $C\underline{H}$) | 44 |
| 71 | $OC_2H_5$ | H | Oel $^1$H-NMR ($CDCl_3$): 1,40 (t, $OCH_2C\underline{H}_3$), 4,45 (q, $OC\underline{H}_2CH_3$), 6,90 (s, $C\underline{H}$), 8,75 (s, $C\underline{H}$) | 45 |

**Beispiel 72-78**

Analog dem in Beispiel 61 beschriebenen Verfahren wird das entsprechende 2-Alkyl- oder 2-Alkoxy-4-chlormethyl-6-hydroxy-pyrimidin, das 6-Chlormethyl-uracil oder das 4-Chlormethyl-6-hydroxy-pyrimidin mit Phosphoroxychlorid behandelt, um die in der nachstehenden Tabelle 4 aufgeführten Ausgangsmaterialien der Formel II, insbesondere der unten angegebenen Formel IIb, herzustellen. Auch die entsprechenden Endprodukte der Formel I und die daraus hergestellten Zwischenprodukte der Formel II sind in dieser Tabelle angegeben.

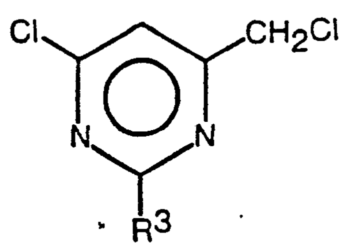

IIb

Tabelle 4

| Beisp. | $R^3$ | Physikalische Angabe(n) | Beispiel-Nr. des Endproduktes der Formel I bzw. des Zwischenproduktes der Formel II |
|---|---|---|---|
| 72 | tert. $C_4H_9$ | Oel $^1H$-NMR ($CDCl_3$): 1,40 (s, $C(CH_3)_3$), 4,63 (s,$CH_2Cl$), 7,43 (s,CH | 5 |
| 73 | Cl | Smp. 32—33°C Sdp. 90°C/0,1 mmHg (13,3 Pa) | 10 |
| 74 | $C_2H_5$ | Sdp. 60°C/0,1 mmHg (13,3 Pa) | 15 |
| 75 | $CH_3$ | Oel $n_D^{22,5}$ = 1,5453 | 81,100 |
| 76 | $OC_2H_5$ | Oel $n_D^{22,5}$ = 1,5362 | |
| 77 | $OCH_3$ | — (Feststoff) | 82,103 |
| 78 | H | — (Oel) | 83,84 |

**Beispiel 79**

Analog dem in Beispiel 62 beschriebenen Verfahren wird das 6-Chlor-4-chlormethyl-2-isopropyl-pyrimidin (siehe Beispiel 61) mit Isopropanol und Natrium umgesetzt, um das als Ausgangsmaterial in Beispiel 22 benötigte 4-Chlormethyl-2-isopropyl-6-isopropoxy-pyrimidin herzustellen. Man erhält dieses Produkt in Form eines Oels, $^1H$-NMR ($CDCl_3$): 1,30 (d, 2x$CH_3$), 1,37 (d, 2x$CH_3$), 3,10 (m, CH), 4,50 (s, CH), 5,40 (m, CH), 6,65 (s, CH).

**Beispiel 80**

Das als Ausgangsmaterial in den Beispielen 23 und 38 benötigte 4-Chlormethyl-2-isopropyl-6-(2-propinyloxy)-pyrimidin kann wie folgt hergestellt werden:

21,5 g 50%ige Natriumhydrid-Dispersion in Oel (0,45 Mol NaH) werden kurz unter Stickstoff in 200 ml absolutem n-Pentan aufgeschlämmt. Danach wird das Natriumhydrid durch Abdekantieren vom Oel freigesetzt und mit 400 ml trockenem Tetrahydrofuran überschichtet. Alsdann werden unter gutem Rühren 25,4 ml (0,43 Mol) 2-Propinol eingetragen, wobei die Temperatur des Gemisches auf 40°C ansteigt. Nach beendeter Wasserstoffentwicklung kühlt man das Gemisch auf 0°C ab und tropft während 30 Minuten eine Lösung von 6-Chlor-4-chlormethyl-2-isopropyl-pyrimidin (siehe Beispiel 61) in 100 ml Tetrahydrofuran zu. Man rührt das Reaktionsgemisch während 16 Stunden nach und dampft dann unter vermindertem Druck den grössten Teil des Lösungsmittels ab. Anschliessend wird der Rückstand in 300 ml Methylenchlorid aufgenommen und die Lösung zweimal mit je 300 ml Wasser neutral gewaschen. Man trocknet die organische Phase über wasserfreiem Magnesiumsulfat, engt sie ein und reinigt den Rückstand an Kieselgel mit Diäthyläther/n-Hexan (1:9). Auf diese Weise erhält man das 4-Chlormethyl-2-isopropyl-6-(2-propinyloxy)-pyrimidin in Form eines gelblichen Oels, $n_D^{24}$ 1,5142.

**Beispiele 81-85**

Analog dem in Beispiel 80 beschriebenen Verfahren wird aus dem entsprechenden 2-Alkyl- oder 2-Alkoxy-6-chlor-4-chlormethyl-pyrimidin bzw. 6-Chlor-4-chlormethyl-pyrimidin und Phenol, 2-Propinol bzw. Aethylenglykol-monomethyläther die in der nachstehenden Tabelle 5 aufgeführten Ausgangsmaterialien der Formel II, insbesondere der oben angegebenen Formel IIa, hergestellt. Auch die entsprechenden Endprodukte der Formel I sind in dieser Tabelle angegeben.

**Tabelle 5**

| Beisp. | $R^2$ | $R^3$ | Physikalische Angabe(n) | Beispiel-Nr. des Endproduktes der Formel I |
|---|---|---|---|---|
| 81 | $OC_6H_5$ | $CH_3$ | Smp. 48—50°C | 31 |
| 82 | $OCH_2C{\equiv}CH$ | $OCH_3$ | — (Oel) | 40 |
| 83 | $OCH_2C{\equiv}CH$ | H | Smp. 40—41°C | 46 |
| 84 | $OC_6H_5$ | H | Oel $^1$H-NMR ($CDCl_3$): 4,62 (s, $C\underline{H}_2Cl$), 7,0—7,7 (breites Signal, $C_6\underline{H}_5$+5-$\underline{H}$ in Pyrimidinkern), 8,80 (s, $C\underline{H}$) | 47 |
| 85 | $OCH_2CH_2OCH_3$ | iso$C_3H_7$ | Oel $^1$H-NMR ($CDCl_3$): 1,30 (d, $CH(C\underline{H}_3)_2$), 3,06 (m, $C\underline{H}(CH_3)_2$), 3,44 (s, $OC\underline{H}_3$), 3,75 (m, $OC\underline{H}_2$), 4,52 (s, $C\underline{H}_2Cl$), 4,58 (m, $OC\underline{H}_2$), 6,80 (s, $C\underline{H}$) | 49 |

**Beispiel 86**

Der als Ausgangsmaterial in Beispiel 17 benötigte N,N-Dimethylcarbaminsäure-(4-chlormethyl-2-methyl-6-pyrimidinyl)ester kann wie folgt hergestellt werden:

5 g (0 0315 Mol) 4-Chlormethyl-6-hydroxy-2-methyl-pyrimidin werden unter Stickstoff portionenweise in eine Suspension von 0,80 g (0,0331 Mol) Natriumhydrid (durch zweimaliges Extrahieren mit n-Pentan aus einer 50%-igen Dispersion in Oel freigesetzt) in 50 ml absolutes Tetrahydrofuran eingetragen. Nach beendeter Wasserstoffentwicklung tropft man 2 9 ml (0,0315 Mol) Dimethylcarbamoylchlorid zu und erhitzt das Reaktionsgemisch während 16 Stunden bei Rückflusstemperatur. Anschliessend wird das abgekühlte Reaktionsgemisch mit 150 ml Toluol verdünnt, der Reihe nach mit je 50 ml 5%-iger Natriumbicarbonatlösung, 0,1N Salzsäure und gesättigter Natriumchloridlösung gewaschen, über wasserfreiem Natriumsulfat getrocknet und unter vermindertem Druck eingeengt. Das zurückbleibende Oel wird mit 90% Diäthyläther in n-Hexan kurz an Kieselgel filtriert. Man erhält den N,N-Dimethylcarbaminsäure-(4-chlormethyl-2-methyl-6-pyrimidinyl)ester als farbloses Oel, $n^{22}_D$ 1,5265.

**Beispiel 87**

Der als Ausgangsmaterial in Beispiel 17 benötigte N,N-Dimethylcarbaminsäure-(4-chlormethyl-2-methyl-6-pyrimidinyl)ester kann auch wie folgt hergestellt werden:

1,9 kg (12 Mol) 4-Chlormethyl-6,hydroxy-2-methyl-pyrimidin werden in 14 l Toluol suspendiert und der Reihe nach mit 1,353 kg (12,6 Mol) Dimethylcarbamoylchlorid und 3,48 kg (25,2 Mol) Kaliumcarbonat versetzt. Anschliessend wird die hellbeige Suspension während 72 Stunden bei 60°C gerührt. Man filtriert das Reaktionsgemisch durch Celite und wäscht die Toluolphase viermal mit je 5 l 1%iger Schwefelsäure und zweimal mit Wasser neutral, trocknet sie über wasserfreiem Natriumsulfat, destilliert das Losungsmittel unter

vermindertem Druck ab und erhält somit den N,N-Dimethylcarbaminsäure-(4-chlormethyl-2-methyl-6-pyrimidinyl)ester als gelbes Oel. Dieses Rohprodukt wird an 12 kg Kieselgel mit Aethylacetat/n-Hexan (1:4) chromatographisch gereinigt und aus Diäthyläther/n-Hexan kristallisiert. Man erhält das reinere Produkt als weisse Kristalle, Smp. 49,5-50° C.

**Beispiel 88**

Analog dem in Beispiel 86 beschriebenen Verfahren wird das 4-Chlormethyl-6-hydroxy-2-isopropyl-pyrimidin mit Allylbromid umgesetzt, um das als Ausgangsmaterial in Beispiel 24 benötigte 4-Chlormethyl-2-isopropyl-6-(2-propenyloxy)-pyrimidin herzustellen. Man erhält dieses Produkt in Form eines Oels, H-NMR ($CDCl_3$): 1,30 (d, $2xC\underline{H}_3$), 3,15 (m, $C\underline{H}$), 4,55 (s, $C\underline{H}_2$), 4,93 (m, $C\underline{H}_2$), 5,37 (m, $C\underline{H}_2$), 6,12 (m, $C\underline{H}$), 6,75 (s, $C\underline{H}$).

**Beispiel 89**

Analog dem in Beispiel 86 beschriebenen Verfahren wird das 4-Chlormethyl-6-hydroxy-2-methyl-pyrimidin mit N,N-Dimethylthiocarbamoylchlorid umgesetzt, um den als Ausgangsmaterial in Beispiel 30 benötigten N,N-Dimethyl-thiocarbaminsäure-(4-chlormethyl-2-methyl-6-pyrimidinyl)-ester herzustellen. Man erhält dieses Produkt in Form eines gelben Oels.

**Beispiel 90**

Analog dem in Beispiel 87 beschriebenen Verfahren wird das 2-Aethoxy-4-chlormethyl-6-hydroxy-pyrimidin mit N,N-Dimethylcarbamoylchlorid umgesetzt, um den als Ausgangsmaterial in Beispiel 33 beschriebenen N,N-Dimethyl-carbaminsäure-(2-äthoxy-4-chlormethyl-6-pyrimidinyl)ester herzustellen. Man erhält dieses Produkt in Form eines gelben Oels, $^1$H-NMR ($CDCl_3$): 1,43 (t, $OCH_2C\underline{H}_3$), 3,08 und 3,14 (2 x s, $CON(C\underline{H}_3)_2$), 4,40 (q, $O\underline{C}H_2C\underline{H}_3$), 4,50 (s, $C\underline{H}_2Cl$), 7,00 (s, $C\underline{H}$).

**Beispiel 91**

Analog dem in Beispiel 87 beschriebenen Verfahren wird das 4-Chlormethyl-2-(p-chlorphenyl)-6-hydroxy-pyrimidin mit O,O-Diäthyl-thiophosphorsäurechlorid umgesetzt, um den als Ausgangsmaterial in Beispiel 28 benötigten Thiophos-phorsäure-[O,O-diäthyl-O-[4-chlormethyl-2-(p-chlorphenyl)-6-pyrimidinyl]]ester herzustellen. Man erhält dieses Produkt in Form eines weissen Feststoffes.

**Beispiel 92**

Das als Ausgangsmaterial in Beispiel 10 benötigte 4-Chlormethyl-2,6-dichlor-pyrimidin (siehe Beispiel 73) kann auch wie folgt hergestellt werden:

200 g (1,25 Mol) 6-Chlormethyl-uracil werden in 600 ml Phosphoroxychlorid suspendiert, und die Suspension wird während 6 Stunden unter Stickstoff bei 80° C erhitzt. Alsdann wird das Reaktionsgemisch auf 21 1 Wasser bei 3O-40° C getropft und das wässrige Gemisch zweimal mit je 5 1 Methylenchlorid extrahiert. Man wäscht die organische Phase mit Wasser und neutral mit Kaliumcarbonatlösung, trocknet sie über wasserfreiem Natriumsulfat und dampft sie unter vermindertem Druck ein. Nach Destillieren des öligen Rückstandes am Hochvakuum erhält man das 4-Chlormethyl-2,6-dichlor-pyrimidin, Sdp. 90° C/0,1 mm Hg (93,3 Pa), Smp. 32-33° C.

**Beispiel 93**

Das als Ausgangsmaterial in den Beispielen 2, 6, 29, 53 und 54 benötigte 4-Chlormethyl-2,6-diäthoxy-pyrimidin (siehe Beispiel 60) kann auch wie folgt hergestellt werden:

Zu einer Lösung von 50 g (0,255 Mol) 4-Chlormethyl-2,6-dichlor-pyrimidin (siehe Beispiele 73 und 92) in 170 ml absolutem Aethanol wird unter Eiskühlung und Rühren während einer Stunde eine Lösung von 11,73 g (0,51 Mol) Natrium in 260 ml Aethanol zugetropft. Man rührt das Reaktionsgemisch weitere 5 Stunden bei

Raumtemperatur, giesst es auf Wasser und extrahiert das wässrige Gemisch zweimal mit je 1 l Methylenchlorid. Die organische Phase wird unter vermindertem Druck eingeengt und der ölige Rückstand am Hochvakuum destilliert Man erhält so das 4-Chlormethyl-2,6-diäthoxy-pyrimidin Sdp 90° C/0,1 mm Hg und Smp. 38-39° C.

**Beispiele 94-97**

Analog dem in Beispiel 93 beschriebenen Verfahren werden aus 4-Chlormethyl-2,6-dichlor-pyrimidin (siehe Beispiele 73 und 92), dem entsprechenden Alkohol und Natrium die in der nachstehenden Tabelle 6 aufgeführten Ausgangsmaterialien der Formel II, insbesondere der oben angegebenen Formel IIa, hergestellt. Auch die entsprechenden Endprodukte der Formel I sind in dieser Tabelle angegeben.

**Tabelle 6**

| Beisp. | $R^2$ | $R^3$ | Physikalische Angabe(n) | Beispiel-Nr. des Endproduktes der Formel I |
|---|---|---|---|---|
| 94 | $OCH_3$ | Cl | – (Oel) | 11 |
| 95 | $OCH_3$ | $OCH_3$ | – (Oel) | 14 |
| 96 | iso$C_3H_7O$ | iso$C_3H_7O$ | Oel <br> $^1$H-NMR ($CDCl_3$): 1,34 (d, 2 × C<u>H</u>$_3$), 1,40 (d, 2 × C<u>H</u>$_3$), 4,42 (s, C<u>H</u>$_2$), 5,22 (m, C<u>H</u>), 5,36 (m, C<u>H</u>), 6,44 (s, C<u>H</u>) | 21 |
| 97 | $OC_2H_5$ | Cl | – (Oel) | 25 |

**Beispiel 98**

Die als Ausgangsmaterialien in den Beispielen 34 und 36 benötigte 6-Chlor-4-chlormethyl-2-diäthylamino-pyrimidin und 2-Chlor-4-chlormethyl-6-diäthylamino-pyrimidin konnen wie folgt hergestellt werden:

Eine Lösung von 5,92 g (0,03 Mol) 4-Chlormethyl-2,6-dichlor-pyrimidin (siehe Beispiele 73 und 92) in 60 ml Toluol wird bei 0° C tropfenweise mit 12,5 ml (0,12 Mol) Diäthylamin während 30 Minuten versetzt. Anschliessend lässt man das Reaktionsgemisch langsam auf Raumtemperatur kommen und rührt weitere 24 Stunden nach. Man verdünnt das Gemisch mit weiteren 150 ml Toluol, wäscht das Ganze mit 50 ml verdünnter Natronlauge und mit Wasser, trocknet die organische Phase über wasserfreiem Magnesiumsulfat und dampft das Lösungsmittel unter vermindertem Druck ab.

Durch Chromatographie des Rohprodukts an Kieselgel unter Verwendung von Diäthyläther/n-Hexan (1:19) als Laufmittel erhält man als erste Fraktion das als Ausgangsmaterial in Beispiel 34 benötigte 6-Chlor-4-chlormethyl-2-diäthylamino-pyrimidin in Form eines nahezu farblosen Oels, $^1$H-NMR ($CDCl_3$): 1,17 (t, N($CH_2$C<u>H</u>$_3$)$_2$), 3,60 (q, N(C<u>H</u>$_2$$CH_3$)$_2$), 4,37 (s, C<u>H</u>$_2$Cl), 6,67 (s, CH).

Durch nachfolgende Chromatographie mit Diäthyläther/ n-Hexan (1:4) erhält man als Hauptfraktion das als Ausgangsmaterial in Beispiel 36 benötigte 2-Chlor-4-chlor-methyl-6-diäthylamino-pyrimidin in Form eines gelben Oels, $^1$H-NMR ($CDCl_3$): 1,20 (t, N($CH_2$C<u>H</u>$_3$)$_2$), 3,54 (q, N(C<u>H</u>$_2$C<u>H</u>$_3$)$_2$), 4,44 (s, C<u>H</u>$_2$Cl), 6,48 (s, C<u>H</u>).

**Beispiel 99-103**

Analog dem in Beispiel 98 beschriebenen Verfahren wird das entsprechende 2-Alkyl- oder 2-Alkoxy-6-chlor-4-chlormethyl-pyrimidin oder das 2-Chlormethyl-2,6-dichlor-chlormethyl-pyrimidin oder das 2-Chlormethyl-2,6-dichlorpyrimidin mit dem entsprechenden Mono- oder Dialkylamin umgesetzt, um die in der nachstehenden Tabelle 7 aufgeführten Ausgangsmaterialien der Formel II, insbesondere der oben angegebenen Formel IIa, herzustellen. Auch die entsprechenden Endprodukte der Formel I sind in dieser Tabelle angegeben.

Tabelle 7

| Beisp. | $R^2$ | $R^3$ | Physikalische Angabe(n) | Beispiel-Nr. des Endproduktes der Formel I |
|---|---|---|---|---|
| 99 | $N(CH_3)_2$ | Cl | — (Oel) | 9 |
| 100 | $N(CH_3)_2$ | $CH_3$ | — (Oel) | 32 |
| 101 | Cl | $NHC_3H_7$iso | Oel<br>$^1$H-NMR ($CDCl_3$): 1,22 (d,$CH(C\underline{H}_3)_2$), 4,22 (m,$C\underline{H}(CH_3)_2$), 4,37 (s,$C\underline{H}_2Cl$), 5,18 (breites Signal, N$\underline{H}$), 6,70 (s, C$\underline{H}$) | 35 |
| 102 | $NHC_3H_7$iso | Cl | Oel<br>$^1$H-NMR ($CDCl_3$): 1,27 (d,$CH(C\underline{H}_3)_2$), 4,10 (m,$C\underline{H}(CH_3)_2$), 4,44 (s,$C\underline{H}_2Cl$), 5,28 (breites Signal, N$\underline{H}$), 6,42 (s, C$\underline{H}$) | 37 |
| 103 | $N(C_2H_5)_2$ | $OCH_3$ | Oel<br>$^1$H-NMR ($CDCl_3$): 1,20 (t,$N(CH_2C\underline{H}_3)_2$), 3,53 (q,$N(C\underline{H}CH_3)_2$), 3,92 (s,$OC\underline{H}_3$), 4,40 (s, $C\underline{H}_2Cl$), 6,22 (s, C$\underline{H}$) | 41 |

**Beispiel 104**

Analog dem in Beispiel 98 beschriebenen Verfahren wird 4-Aethoxy-2-chlor-6-chlormethyl-pyrimidin (siehe Beispiel 97) mit Diäthylamin umgesetzt, um das als Ausgangsmaterial in Beispiel 42 benötigte 4-Aethoxy-6-chlormethyl-2-diäthylamino-pyrimidin herzustellen. Man erhält dieses Produkt in Form eines gelben Oels, H-NMR ($CDCl_3$): 1,20 (t, $N(CH_2C\underline{H}_3)_2$), 1,37 (t, $OCH_2C\underline{H}_3$), 3,60 (q, $N(C\underline{H}_2CH_3)_2$) 4,33 (s, $C\underline{H}_2Cl$), 4,35 (q, $OC\underline{H}_2CH_3$), 6,06 (s, C$\underline{H}$).

**Beispiel 105**

Das als Ausgangsmaterial in Beispiel 26 benötigte 4-Chlormethyl-2,6-diäthylthio-pyrimidin kann wie folgt hergestellt werden:

Ein Gemisch von 16,05 g (0,10 Mol) 6-Chlormethyl-uracil, 9,85 g (0,12 Mol) Natriumacetat sowie 0,3 g Natriumjodid in 50 ml Eisessig wird während 20 Minuten bei Rückflusstemperatur erhitzt. Anschliessend wird das Gemisch auf Raumtemperatur gekühlt und filtriert. Das Filtrat wird mit 250 ml Diäthyläther versetzt und die resultierenden Kristalle abfiltriert. Die vereinigten Kristallisate werden zu 200 ml Wasser gegeben und so die noch verbleibenden Salze ausgewaschen. Man isoliert durch Filtrieren 11,3 g 6-Acetoxy-methyl-uracil.

Das bei 50°C getrocknet Produkt der ersten Verfahrensstufe wird in 73 ml (0,8 Mol) Phosphoroxychlorid gelöst und die Lösung eine Stunde bei 80°C erhitzt. Man destilliert alsdann das überschüssige Phosphoroxychlorid unter vermindertem Druck ab, giesst den Rückstand auf 200 ml Wasser, extrahiert das wässrige Gemisch zweimal mit je 200 ml Diäthyläther, trocknet die vereinigten Extrakte über wasserfreiem Natriumsulfat und dampft die organische Lösung ein. Der ölige Rückstand wird mit 25% Diäthyläther in n-Hexan chromatographisch aufgetrennt. Man isoliert so das 4-Acetoxymethyl-2,6-dichlor-pyrimidin, $^1$H-NMR ($CDCl_3$): 2,23 (s, $C\underline{H}_3$), 5,19 (s, $C\underline{H}_2$), 7,35 (s, C$\underline{H}$).

1,73 g (0,036 Mol) einer 50%igen Dispersion von Natriumhydrid in Oel wird zu 40 ml absolutes Tetrahydrofuran gegeben und die resultierende Suspension bei Raumtemperatur mit 2,54 ml (0,034 Mol) Aethylmercaptan behandelt. Nach Beendigung der Wasserstoffentwicklung wird bei 0°C unter Kühlung eine Lösung von 3,8 g Acetoxymethyl-2,6-dichlor-pyrimidin in 10 ml Tetrahydrofuran zugetropft. Man rührt das

Reaktionsgemisch während 24 Stunden bei Raumtemperatur, verdünnt es anschliessend mit 100 ml Diäthyläther und wäscht das verdünnte Gemisch mit gesättigter Natriumchloridlösung. Dann trocknet man die organische Phase über wasserfreiem Magnesiumsulfat und dampft sie ein. Der ölige Rückstand wird an Kieselgel mit 85% Diäthylather in n-Hexan chromatographisch aufgetrennt. Man isoliert so das 4-Acetoxymethyl-2,6-diäthylthio-pyrimidin, $^1$H-NMR ($CDCl_3$): 1,39 (t, 2x$C\underline{H}_3$), 2,19 (s, $C\underline{H}_3$), 3,15 und 3,20 (qq, 2x$C\underline{H}_2$), 5,01 (s, $CH_2$), 6,79 (s, CH).

Eine Lösung von 1,6 g (0,0059 Mol) 4-Acetoxymethyl-2,6-diäthylthio-pyrimidin in 3 ml Aethanol wird bei 0°C mit einer Lösung von 0,86 g (0,0065 Mol) Natriumhydroxid in 2,5 ml Aethanol versetzt. Nach 5 Minuten wird mit 150 ml Diäthyläther verdünnt und mit Wasser neutral gewaschen. Man trocknet die organische Phase über wasserfreiem Magnesiumsulfat und dampft sie ein. Auf diese Weise erhält man als Rückstand das 2,6-Diäthylthio-4-hydroxy-methyl-pyrimidin.

Der Rückstand (1,35 g) wird dann bei 0°C tropfenweise mit 8,5 ml Thionylchlorid versetzt. Nach 10 Minuten wird das überschüssige Thionylchlorid unter vermindertem Druck bei 25°C abdestilliert und der Rückstand mit 150 ml Diäthyläther und 50 ml Wasser ausgeschüttelt. Man trocknet die organische Phase über wasserfreiem Magnesiumsulfat und entfernt unter vermindertem Druck das Lösungsmittel. Man erhält so 1,45 g 4-Chlormethyl-2,6-diäthylthio-pyrimidin als leicht gelbliches Oel.

**Beispiel 106**

Analog dem in Beispiel 105 beschriebenen fünfstufigen Verfahrens wird aus 4-Chlormethyl-2-cyclopropyl-6-hydroxypyrimidin das als Ausgangsmaterial in Beispiel 20 benötigte 6-Aethylthio-4-chlormethyl-2-cyclopropyl-pyrimidin hergestellt. Dieses Produkt wird in Form eines Oels erhalten.

**Beispiel 107**

Analog dem in Beispiel 59 beschriebenen Verfahren wird das 4-Chlormethyl-6-hydroxy-2-methylthio-pyrimidin (aus S. Cohen et al., J. Org. Chem. <u>27</u>, 3545-3549 (1962) bekannt) mit Diäthylsulfat behandelt, un das als Ausgangsmaterial in Beispiel 19 benötigte 6-Aethoxy-4-chlormethyl-2-methylthio-pyrimidin herzustellen. Smp. (nach Kristallisation aus Wasser) 167-171°C; H-NMR ($CDCl_3$): 1,40 (t, $CH_3$), 2,56 (s, $S\underline{CH}_3$), 4,45 (q, $C\underline{H}_2$), 4,47 (s, $C\underline{H}_2Cl$), 6,55 (s, $C\underline{H}$).

**Beispiel 108**

Das als Ausgangsmaterial in Beispiel 48 benötigte 4-Chlormethyl-2-isopropyl-6-methyl-pyrimidin kann wie folgt hergestellt werden:

Ein Gemisch von 6,5 g (0,036 Mol) 4-Hydroxy-2-iso-propyl-6-methyl-pyrimidin und 16,8 g (0,108 Mol) Phosphoroxychlorid in 40 ml Chloroform wird während 2 Stunden auf Rückflusstemperatur erhitzt. Man engt dann die Reaktionslösung unter vermindertem Druck ein, giesst den Rückstand auf Wasser und extrahiert die wässrige Phase zweimal mit je 100 ml n-Hexan. Die organische Phase wird über wasserfreiem Natriumsulfat getrocknet und anschliessend eingedampft. Man erhält das 4-Chlor-2-isopropyl-6-methyl-pyri-midin in Form eines orangen Oels, H-NMR ($CDCl_3$): 1,32 (d, $CH(C\underline{H}_3)_2$), 2,48 (s, $C\underline{H}_3$), 3,17 (m, $C\underline{H}(CH_3)_2$), 7,03 (s, $C\underline{H}$).

3,05 g (0,018 Mol) des obigen Produktes und 5,9 g (0,09 Mol) Zinkpulver werden in 90 ml Wasser auf Rückflusstemperatur erhitzt und danach tropfenweise mit 1,4 ml (0,019 Mol) 25%iger Ammoniaklösung versetzt. Nach siebenstündiger Reaktion wird das Gemisch auf Raumtemperatur gekühlt und zweimal mit je 100 ml Diäthyläther extrahiert. Man trocknet die organische Phase über wasserfreiem Natriumsulfat und destilliert den Diäthyläther über eine Vigreux-Kolonne unter Normaldruck ab. Man erhält das 2-Isopropyl-4-methyl-pyrimidin in Form eines gelblichen Oels.

1,7 g (0,0125 Mol) des obigen Produktes werden in einem Gemisch von 9 ml Wasser, 22 ml Methanol und 0,91 ml konzentrierter Schwefelsäure gelöst, und anschliessend werden 5,6 g (0,0245 Mol) Ammoniumperoxydisulfat zugefügt. Man erhitzt das Gemisch während 4 Stunden auf Rückflusstemperatur, engt danach das Gemisch unter vermindertem Druck etwas ein, fügt zum Rückstand Wasser und extrahiert mit Chloroform und dampft das Chloroform von der organischen Phase unter vermindertem Druck ab. Man erhält das 4-Hydroxy-2-isopropyl-6-methyl-pyrimidin in Form eines braunen Oels.

Ein Gemisch von 1,7 g (0,0097 Mol) des obigen Produktes, 10 ml Phosphoroxychlorid und 10 ml Chloroform wird während 2 Stunden auf Rückflusstemperatur erhitzt. Das Lösungsmittel und das überschüssige Phosphoroxychlorid werden dann unter vermindertem Druck abdestilliert, und der Rückstand wird auf Wasser gegossen. Man bringt die wässrige Lösung unter Zugabe von verdünnter Natronlauge auf einen pH-Wert von 7, extrahiert sie zweimal mit n-Hexan, trocknet die organische Phase mit wasserfreiem Natriumsulfat und dampft diese ein. Man erhält das 4-Chlormethyl-2-isopropyl-6-methyl-pyrimidin als gelbliches Oel, $^1$H-NMR ($CDCl_3$): 1,32 (d, $CH(C\underline{H}_3)_2$), 2,53 (s, $CH_3$), 3,17 (m, $C\underline{H}(CH_3)_2$), 4,53 (s, $C\underline{H}_2Cl$), 7,20 (s, $C\underline{H}$).

**Beispiel 109**

Das als Ausgangsmaterial in Beispiel 50 benötigte 4-Chlormethyl-6-fluor-2-isopropyl-pyrimidin kann wie folgt hergestellt werden:

Ein Gemisch von 0,8 g (0,0039 Mol) 6-Chlor-4-chlor-methyl-2-isopropyl-pyrimidin (siehe Beispiel 61), 0,51 g (0,0089 Mol) wasserfreies Kaliumfluorid und 0,14 g 18-Crown-6 (0,0004 Mol) in 7 ml Acetonitril wird 48 Stunden auf Rückflusstemperatur erhitzt. Das Reaktionsgemisch wird alsdann in Diäthyläther aufgenommen und die Lösung mit gesättigter Natriumchloridlösung gewaschen, über wasserfreiem Natriumsulfat getrocknet und unter vermindertem Druck vom Lösungsmittel freigesetzt. Man erhält in Form eines gelben Oels das 4-Chlormethyl-6-fluor-2-isopropyl-pyrimidin, H-NMR ($CDCl_3$): 1,32 (d, $CH(CH_3)_2$), 3,18 (m, $CH(CH_3)_2$), 4,62 (s, $C\underline{H}_2Cl$), 6,99 (d, $C\underline{H}$).

**Beispiel 110**

Das als Ausgangsmaterial in Beispiel 51 benötigte 2-Chlor-4-chlormethyl-6-fluor-pyrimidin kann wie folgt hergestellt werden:

5 g (0,027 Mol) 4-Chlormethyl-2,6-dichlor-pyrimidin (siehe Beispiele 73 und 92) und 3,3 g (0,056 Mol) wasserfreies Kaliumfluorid werden in 8 ml Sulfolan während 24 Stunden auf 140°C erhitzt. Man kühlt dann das Reaktionsgemisch auf 0°C ab und filtriert es unter Verwendung von Diäthyläther/n-Hexan (1:4) an Kieselgel. Nach Abdampfen des Lösungsmittels erhält man das 2-Chlor-4-chlormethyl-6-fluor-pyrimidin in Form eines gelben Oels, $^1$H-NMR ($CDCl_3$): 4,70 (s, $C\underline{H}_2Cl$), 7,30 (d, $J_{FH}$=2 Hz, H).

**Beispiel 111**

Analog dem in Beispiel 59 oder 60 beschriebenen Verfahren oderdemjenigender Beispiele 61 und 62 wird das 2-Aethoxy-5-chlor-4-chlormethyl-6-hydroxy-pyrimidin in das als Ausgangsmaterial in Beispiel 55 benötigte 5-Chlor-chlormethyl-2,6-diäthoxy-pyrimidin übergeführt. Man erhält dieses Produkt in Form eines Oels.

**Beispiel 112**

Das als Ausgangsmaterial in Beispiel 111 benötigte 2-Aethoxy-5-chlor-4-chlormethyl-6-hydroxy-pyrimidin kann wie folgt hergestellt werden:

Zu einer Lösung von 6 g (0,032 Mol) 2-Aethoxy-4-chlormethyl-6-hydroxy-pyrimidin in 90 ml Methylenchlorid werden 2,8 ml (0,035 Mol) Sulfurylchlorid zugetropft, und das Reaktionsgemisch wird anschliessend während 4 Stunden bei Raumtemperatur gerührt. Das Reaktionsgemisch wird dann mit 100 ml Wasser versetzt und mit 30%iger Natriumhydroxidlösung auf pH 7 gestellt. Man trennt die organische Phase ab, extrahiert die vereinigten organischen Phasen mit Wasser, trocknet sie über wasserfreiem Natriumsulfat und dampft sie ein. Man erhält kristallines 2-Aethoxy-5-chlor-4-chlormethyl-6-hydroxy-pyrimidin, $^1$H-NMR ($CDCl_3$): 1,45 (t, $OCH_2C\underline{H}_3$), 4,54 (q, $OC\underline{H}_2CH_3$), 4,54 (s, $C\underline{H}_2Cl$), 9,80 (breites Signal, OH).

**Beispiel 113**

Das als Ausgangsmaterial in den Beispielen 76, 90 und 112 sowie zur direkten Synthese (durch Aethoxylierung) eines 6-Alkoxy-2-äthoxy-4-chlormethyl-pyrimidins (z.B. das Endprodukt der Beispiele 60 und 93) benötigte 2-Aethoxy-4-chlormethyl-6-hydroxy-pyrimidin kann wie folgt hergestellt werden:

Ein Gemisch von 24,9 g (0,2 Mol) O-Aethylisoharnstoffhydrochlorid und 21,5 g (0,14 Mol) 4-Chloracetessigsauremethylester in 100 ml Methanol wird bei O-5°C während 10 Minuten mit einer Lösung von 7,55 g (0,33 Mol) Natrium in 100 ml Methanol versetzt. Man rührt das Reaktionsgemisch 2 Stunden bei 0°C und 18 Stunden bei Raumtemperatur nach und dampft dann das Lösungsmittel unter vermindertem Druck ab. Der Rückstand wird in 300 ml Wasser gelöst und die Lösung auf einen pH-Wert von 6 gestellt. Das entstandene kristalline Produkt wird abfiltriert, mit Diäthyläther gewaschen und bei 40°C getrocknet. Man erhält auf diese Weise das 2-Aethoxy-4-chlormethyl-6-hydroxy-pyrimidin, Smp. 157-159°C. Durch dreimaliges Nachextrahieren des wässrigen Filtratsmit je 150 ml Chloroform erhält man eine weitere Menge dieses Produkts, Smp. 159-161°C.

**Beispiel 114**

Das 2-Aethoxy-4-chlormethyl-6-hydroxy-pyrimidin kann auch wie folgt hergestellt werden:

Eine Emulsion von 20,9 g (0,135 Mol) 4-Chloracetessig-säure-methylester und 23,5 g (0,189 Mol) O-Aethylisoharnstoff-hydrochlorid wird bei -20 bis -10°C während 5 Minuten unter gutem Rühren mit einer Lösung von 41,3 g (0,31 Mol) Natriumhydroxid in 40 ml Wasser versetzt. Nach zweistündigem Nachrühren bei Raumtemperatur wird das entstandene kristalline Produkt abfiltriert und getrocknet. Die Kristalle, Smp. 156-158°C, werden mit Diäthyläther gut gewaschen. Man erhält 2-Aethoxy-4-chlormethyl-6-hydroxy-pyrimidin, Smp. 161-163°C.

**Beispiel 115**

Analog dem in Beispiel 113 beschriebenen Verfahren setzt man O-Methylisoharnstoff-hydrochlorid oder -hydrosulfat mit 4-Chloracetessigsäure-methylester um, um das als Ausgangsmaterial in den Beispielen 68, 69 und 77 benötigte 4-Chlormethyl-6-hydroxy-2-methoxy-pyrimidin' Smp-154-155°C, herzustellen.

**Beispiel 116**

Analog dem in Beispiel 113 beschriebenen Verfahren setztman Formamidin-hydroacetatoder -hydrochlorid mit 4-Chloracetessigsäure-methylester um, um das als Ausgangsmaterial in den Beispielen 70, 71 und 78 benötigte 4-Chlormethyl-6-hydroxy-pyrimidin, Smp. 164-165,5°C (aus Aethanol kristallisiert) herzustellen.

**III. Formulierungsbeispiele:**

**Beispiel 117**

Ein emulgierbares Konzentrat hat folgender Zusammensetzung:

| | | g/Liter |
|---|---|---|
| Verbindung der Formel I (Wirkstoff) | | 500 |
| Emulgator-Mischung, bestehend aus Calcium-Alkylarylsulfonat, Alkylphenoläthoxylat und einem Aethylenoxid/Propylenoxid-Blockpoly-merisat | | 50 |
| Calcium-Dodecylbenzolsulfonat | | 25 |
| Lösungsmittel (Gemisch aus Mono-, Di- und Tri(nieder alkyl)benzolen) | ad | 1000 ml |

**Patentansprüche** für die Vertragsstaaten BE, CH, DE, FR, GB, IT, LI, LU, NL, SE

1. Verbindungen der allgemeinen Formel

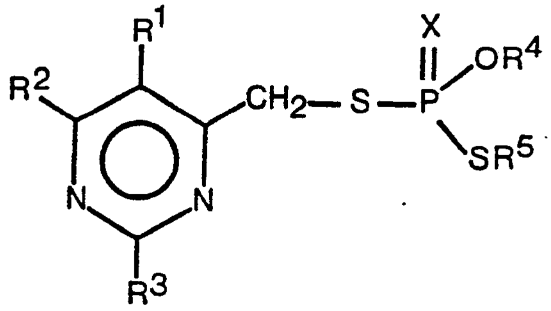

I

worin $R^1$ Wasserstoff, Fluor oder Chlor,
$R^2$ Wasserstoff, Fluor, Chlor, $C_{1-4}$-Alkyl, $C_{1-6}$-Alkoxy, 2-Propenyloxy, 2-Propinyloxy, 2-($C_{1-4}$-Alkoxy)-äthoxy, unsubstituiertes oder mit Halogen mono- oder disubstituiertes Phenoxy, $C_{1-6}$-Alkylthio, $C_{1-4}$-Alkylamino, Di($C_{1-4}$-alkyl)amino oder eine Gruppe (a) oder (b)

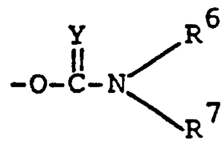

(a)

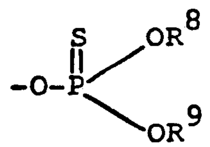

(b)

$R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkyl-thio, $C_{1-4}$-Alkylamino, Di($C_{1-4}$-alkyl)amino oder unsubstituiertes, monosubstituiertes oder disubstituiertes Phenyl, wobei die Substituenten Fluor, Chlor, Trifluormethyl und/oder Methoxy sind,
$R^4$ $C_{1-3}$-Alkyl,
$R^5$ $C_{1-6}$-Alkyl,
$R^6$ und $R^7$ bzw. $R^8$ und $R^9$ unabhängig voneinander $C_{1-3}$-Alkyl
und X und Y unabhängig voneinander Sauerstoff oder Schwefel bedeuten.

2. Verbindungen nach Anspruch 1, worin $R^2$ Wasserstoff, Fluor, Chlor, $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, Di($C_{1-4}$-alkyl)amino oder unsubstituiertes, monosubstituiertes oder disubstituiertes Phenyl bedeuten, wobei die Substituenten Fluor, Chlor, Trifluormethyl und(oder Methoxy sind, und $R^1$, $R^4$, $R^5$ und X die in Anspruch 1 angegebenen Bedeutungen besitzen.

3. Verbindungen nach Anspruch 2, worin $R^1$ Wasserstoff bedeutet.

4. Verbindungen nach Anspruch 2 oder 3, worin $R^2$ Methoxy, Aethoxy, Isopropoxy oder 2-Propinyloxy bedeutet.

5. Verbindungen nach einem der Ansprüche 2 bis 4, worin $R^3$ Wasserstoff, Methyl, Isopropyl, tert.Butyl, Cyclopropyl, Methoxy, Aethoxy, Methylthio, Aethylthio, Dimethylamino oder Diäthylamino bedeutet.

6. Verbindungen nach Anspruch 1, worin $R^3$ Isopropylamino bedeutet.

7. Verbindungen nach einem der Ansprüche 2 bis 5, worin $R^4$ Aethyl bedeutet.
8. Verbindungen nach einem der Ansprüche 2 bis 5 und 7, worin $R^5$ n-Propyl bedeutet.
9. Verbindungen nach einem der Ansprüche 2 bis 5, 7 und 8, worin X Sauerstoff bedeutet.
10. Verbindungen nach Anspruch 2, worin $R^2$ und/oder $R^3$ $C_{1-6}$-Alkoxy, 2-Propenyloxy oder 2-Propinyloxy bedeutet.
11. Verbindungen nach Anspruch 2, worin $R^1$, $R^2$ und/ oder $R^3$ Chlor bedeutet.
12. Verbindungen nach Anspruch 1, worin $R^2$ 2-($C_{1-4}$-Alkoxy)-äthoxy oder unsubstituiertes oder mit Halogen monooder disubstituiertes Phenoxy bedeutet.
13. Verbindungen nach Anspruch 2, worin $R^2$ und/oder $R^3$ $C_{1-6}$-Alkylthio bedeutet.
14. Verbindungen nach Anspruch 1, worin $R^2$ und/oder $R^3$ $C_{1-4}$-Alkylamino oder Di($C_{1-4}$-alkyl)amino bedeutet.
15. Verbindungen nach Anspruch 2, worin $R^2$ $C_{1-4}$-Alkyl und $R^3$ $C_{1-6}$-Alkyl, $C_{3-6}$-Cycloalkyl, ($C_{1-4}$-Alkoxy)methyl, $C_{1-6}$-Alkoxy, Di($C_{1-4}$-alkyl)amino oder gegebenenfalls mit Methoxy substituiertes Phenyl bedeuten.
16. Verbindungen nach Anspruch 2, worin $R^1$, $R^2$ und/ oder $R^3$ Wasserstoff bedeutet.
17. Verbindungen nach Anspruch 2, worin $R^1$, $R^2$ und/ oder $R^3$ Fluor bedeutet.
18. Verbindungen nach Anspruch 2, worin $R^2$ eine Gruppe (a) oder (b) bedeutet.
19. Dithiophosphorsäure-[O-äthyl-S-(2,6-diäthoxy-4-pyrimidinylmethyl)-S-(n-propyl)]ester.
20. Dithiophosphorsäure-[O-äthyl-S-[2-isopropyl-6-(2-propinyloxy)-4-pyrimidinylmethyl]-S-(n-propyl)]ester.
21. Eine Verbindung nach Anspruch 2, ausgewählt aus:
Dithiophosphorsäure-[O-äthyl-S-(6-äthoxy-2-methyl-4-pyrimidinylmethyl)-S-(n-propyl)]ester,
Dithiophosphorsäure-[O-äthyl-S-(sek. butyl)-S-(6-äthoxy-2-methyl-4-pyrimidinylmethyl)]ester,
Dithiophosphorsäure-[O-äthyl-S-(6-äthoxy-2-cyclo-propyl-4-pyrimidinylmethyl)-S-(n-propyl)]ester,
Dithiophosphorsäure-[O-äthyl-S-[2-(tert. butyl)-6-chlor-4-pyrimidinylmethyl]-S-(n-propyl)] ester,
Dithiophosphorsäure-[O-äthyl-S-(sek. butyl)-S-(2,6-diäthoxy-4-pyrimidinylmethyl)]ester,
Dithiophosphorsäure-[O-äthyl-S-[6-äthoxy-2-(tert. butyl)-4-pyrimidinylmethyl]-S-(n-propyl)]ester,
Dithiophosphorsäure-[O-äthyl-S-[2-(tert. butyl)-6-methoxy-4-pyrimidinylmethyl]-S-(n-propyl)]ester,
Dithiophosphorsäure-[O-äthyl-S-(2-chlor-6-dimethyl-amino-4-pyrimidinylmethyl)-S-(n-propyl)]ester,
Dithiophosphorsäure-[O-äthyl-S-(2,6-dichlor-4-pyrimidinylmethyl)-S-(n-propyl)]ester,
Dithiophosphorsäure-[O-äthyl-S-(2-chlor-6-methoxy-4-pyrimidinylmethyl)-S-(n-propyl)]ester,
Dithiophosphorsäure-[O-äthyl-S-(6-chlor-2-isopropyl-4-pyrimidinylmethyl)-S-(n-propyl)]ester,
Dithiophosphorsäure-[O-äthyl-S-(6-äthoxy-2-isopropyl-4-pyrimidinylmethyl)-S-(n-propyl)]ester,
Dithiophosphorsäure-[O-äthyl-S-(2,6-dimethoxy-4-pyrimidinylmethyl)-S-(n-propyl)]ester,
Dithiophosphorsäure-[O-äthyl-S-(2-äthyl-6-chlor-4-pyrimidinylmethyl)-S-(n-propyl)]ester,
Dithiophosphorsäure-[O-äthyl-S-(6-äthoxy-2-äthyl-4-pyrimidinylmethyl)-S-(n-propyl)]ester,
Dithiophosphorsäure-[O-äthyl-S-(6-dimethylcarbamoyloxy-2-methyl-4-pyrimidinylmethyl)-S-(n-propyl)]ester,
Dithiophosphorsäure-[O-äthyl-S-[6-(O,O-dimethyl-thiophosphoro)-2-methyl-4-pyrimidinylmethyl]-S-(n-propyl)]ester,
Dithiophosphorsäure-[O-äthyl-S-(6-äthoxy-2-methylthio-4-pyrimidinylmethyl)-S-(n-propyl)]ester,
Dithiophosphorsäure-[O-äthyl-S-(6-äthylthio-2-cyclo-propyl-4-pyrimidinylmethyl)-S-(n-propyl)]ester,
Dithiophosphorsäure-[O-äthyl-S-(2,6-diisopropoxy-4-pyrimidinylmethyl)-S-(n-propyl)]ester,
Dithiophosphorsäure-[O-äthyl-S-(6-isopropoxy-2-iso-propyl-4-pyrimidinylmethyl)-S-(n-propyl)]ester,
Dithiophosphorsäure-[O-äthyl-S-[2-isopropyl-6-(2-propenyloxy)-4-pyrimidinylmethyl]-S-(n-propyl)]ester,
Dithiophosphorsäure-[O-äthyl-S-(6-äthoxy-2-chlor,4-pyrimidinylmethyl)-S-(n-propyl)]ester,
Dithiophosphorsäure-[O-äthyl-S-(2,6-diäthylthio-4-pyrimidinylmethyl)-S-(n-propyl)]ester,
Dithiophosphorsäure-[O-äthyl-S-[6-äthoxy-2-(p-chlor-phenyl)-4-pyrimidinylmethyl]-S-(n-propyl)]ester,
Trithiophosphorsäure-[O-äthyl-S-(2,6-diäthoxy-4-pyrimidinylmethyl)-S-(n-propyl)]ester,
Trithiophosphorsäure-[O-äthyl-S-(2,6-dichlor-4-pyrimidinylmethyl)-S-(n-propyl)]ester und
Trithiophosphorsäure-[O-äthyl-S-(6-äthoxy-2-methyl-4-pyrimidinylmethyl)-S-(sek. butyl)]ester.
22. Eine Verbindung nach Anspruch 1, ausgewählt aus:
Dithiophosphorsäure-[O-äthyl-S-[2-(p-chlorphenyl)-6-O,O-diäthyl-thiophosphoro)-4-pyrimidinylmethyl]-S-(n-propyl)]ester,
Dithiophosphorsäure-[O-äthyl-S-äthyl-S-(2,6-diäthoxy-4-pyrimidinylmethyl]ester,
Dithiophosphorsäure-[O-äthyl-S-(6-dimethylthiocarbamoyl-oxy-2-methyl-4-pyrimidinylmethyl)-S-(n-propyl)]ester,
Dithiophosphorsäure-[O-äthyl-S-(2-methyl-6-phenoxy-4-pyrimidinylmethyl)-S-(n-propyl)]ester,
Dithiophosphorsäure-[O-äthyl-S-(6-dimethylamino-2-methyl-4-pyrimidinylmethyl)-S-(n-propyl)]ester,
Dithiophosphorsäure-[O-äthyl-S-(2-äthoxy-6-dimethyl-carbamoyloxy-4-pyrimidinylmethyl)-S-(n-propyl)]ester,
Dithiophosphorsäure-[O-äthyl-S-(2-diäthylamino-6-chlor-4-pyrimidinylmethyl)-S-(n-propyl)]ester,
Dithiophosphorsäure-[O-äthyl-S-(6-chlor-2-isopropyl-amino-4-pyrimidinylmethyl)-S-(n-propyl)]ester,
Dithiophosphorsäure-[O-äthyl-S-(2-chlor-6-diäthylamino-4-pyrimidinylmethyl)-S-(n-propyl)]ester,
Dithiophosphorsäure-[O-äthyl-S-(2-chlor-6-isopropyl-amino-4-pyrimidinylmethyl)-S-(n-propyl)]ester,
Dithiophosphorsäure-[O-äthyl-S-(sek. butyl)-S-[2-isopropyl-6-(2-propinyloxy)-4-pyrimidinylmethyl]]ester,
Dithiophosphorsäure-[O-äthyl-S-(6-äthoxy-2-methoxy-4-pyrimidinylmethyl)-S-(n-propyl)]ester,
Dithiophosphorsäure-[O-äthyl-S-[2-methoxy-6-(2-pro-pinyloxy)-4-pyrimidinylmethyl]-S-(n-propyl)]ester,
Dithiophosphorsäure-[O-äthyl-S-(6-diäthylamino-2-methoxy-4-pyrimidinylmethyl)-S-(n-propyl)]ester,

Dithiophosphorsäure-[O-äthyl-S-(6-äthoxy-2-diäthyl-amino-4-pyrimidinylmethyl)-S-(n-propyl)]ester, Dithiophosphorsäure-[O-äthyl-S-(6-isopropoxy-2-methoxy-4-pyrimidinylmethyl)-S-(n-propyl)]ester, Dithiophosphorsäure-[O-äthyl-S-(6-methoxy-4-pyrimi-dinylmethyl)-S-(n-propyl)]ester, Dithiophosphorsäure-[O-äthyl-S-(6-äthoxy-4-pyrimi-dinylmethyl)-S-(n-propyl)]ester, Dithiophosphorsäure-[O-äthyl-S-[6-(2-propinyloxy)-4-pyrimidinylmethyl]-S-(n-propyl)]ester, Dithiophosphorsäure-[O-äthyl-S-(6-phenoxy-4-pyrimi-dinylmethyl)-S-(n-propyl)]ester, Dithiophosphorsäure-[O-äthyl-S-(2-isopropyl-6-methyl-4-pyrimidinylmethyl)-S-(n-propyl)]ester, Dithiophosphorsäure-[O-äthyl-S-[2-isopropyl-6-(2-methoxyäthoxy)-4-pyrimidinylmethyl]-S-(n-propyl)]ester, Dithiophosphorsäure-[O-äthyl-S-(6-fluor-2-isopropyl-4-pyrimidinylmethyl)-S-(n-propyl)]ester, Dithiophosphorsäure-[O-äthyl-S-(2-chlor-6-fluor-4-pyrimidinylmethyl)-S-(n-propyl)]ester, Dithiophosphorsäure-[O-äthyl-S-(2-äthoxy-6-isopropoxy-4-pyrimidinylmethyl)-S-(n-propyl)]ester, Dithiophosphorsäure-[S-(2,6-diäthoxy-4-pyrimidinyl-methyl)-O-methyl-S-methyl]ester, Dithiophosphorsäure-[S-(2,6-diäthoxy-4-pyrimidinyl-methyl)-O-methyl-S-(n-propyl)]ester und Dithiophosphorsäure-[O-äthyl-S-(5-chlor-2,6-diäthoxy-4-pyrimidinylmethyl)-S-(n-propyl)]ester.

23. Eine Verbindung nach Anspruch 1 als Schädlingsbekämpfungsmittel.

24. Schädlingsbekämpfungsmittel, dadurch gekennzeichnet, dass es eine wirksame Menge mindestens einer Verbindung der allgemeinen Formel

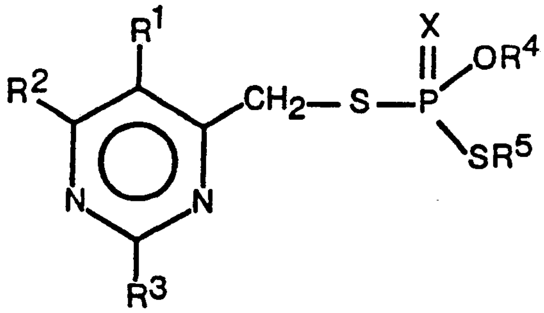

I

worin $R^1$ Wasserstoff, Fluor oder Chlor,

$R^2$ Wasserstoff, Fluor, Chlor, $C_{1-4}$-Alkyl, $C_{1-6}$-Alkoxy, 2-Propenyloxy, 2-Propinyloxy, 2-($C_{1-4}$-Alkoxy)-äthoxy, unsubstituiertes oder mit Halogen mono- oder disubstituiertes Phenoxy, $C_{1-6}$-Alkylthio, $C_{1-4}$-Alkylamino, Di($C_{1-4}$-alkyl)amino oder eine Gruppe (a) oder (b)

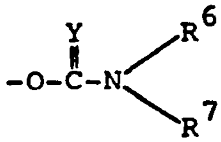

(a)

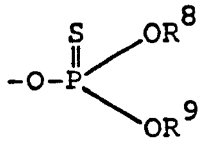

(b)

$R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkyl-thio, $C_{1-4}$-Alkylamino, Di($C_{1-4}$-alkyl)amino oder unsubstituiertes, monosubstituiertes oder disubstituiertes Phenyl, wobei die Substituenten Fluor, Chlor, Trifluormethyl und/oder Methoxy sind,

$R^4$ $C_{1-3}$-Alkyl,

$R^5$ $C_{1-6}$-Alkyl,

$R^6$ und $R^7$ bzw- $R^8$ und $R^9$ unabhängig voneinanfer $C_{1-3}$-Alkyl

und X und Y unabhängig voneinander Sauerstoff oder Schwefel bedeuten,

sowie Formulierungshilfsstoffe enthält.

25. Schädlingsbekämpfungsmittel nach Anspruch 24, worin $R^2$ Wasserstoff, Fluor, Chlor; $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-

Propenyloxy, 2-Propinyloxy, $C_{1-6}$-Alkylthio, Di($C_{1-4}$-alkyl)amino oder unsubstituiertes, monosubstituiertes oder disubstituiertes Phenyl bedeuten, wobei die Substituenten Fluor, Chlor, Trifluormethyl und/oder Methoxy sind, und $R^1$, $R^4$, $R^5$ und X die in Anspruch 24 angegebenen Bedeutungen besitzen.

26. Schädlingsbekämpfungsmittel nach Anspruch 25, dadurch gekennzeichnet, dass es eine wirksame Menge von Dithiophosphorsäure-[O-äthyl-S-(2,6-diäthoxy-4-pyrimidinyl-methyl)-S-(n-propyl)]ester sowie Formulierungshilfsstoffe enthält.

27. Schädlingsbekämpfungsmittel nach Anspruch 25, dadurch gekennzeichnet, dass es eine wirksame Menge von Dithiophosphorsäure-[O-äthyl-S-[2-isopropyl-6-(2-pro-pinyloxy)-4-pyrimidinylmethyl]-S-(n-propyl)]ester sowie Formulierungshilfsstoffe enthält.

28. Verfahren zur Herstellung von Verbindungen der allgemeinen Formel

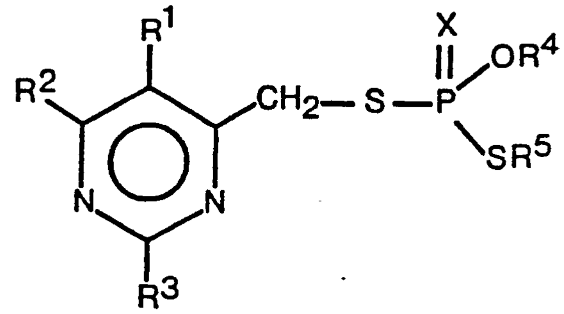

I

worin $R^1$ Wasserstoff, Flour oder Chlor

$R^2$ Wasserstoff, Flour, Chlor, $C_{1-4}$-Alkyl, $C_{1-6}$-Alkoxy 2-Propenyloxy, 2-Propinyloxy, 2-($C_{1-4}$-Alkoxy)-äthoxy, unsubstituiertes oder mit Halogen mono- oder disubstituiertes Phenoxy, $C_{1-6}$-Alkylthio, $C_{1-4}$-Alkylamino, Di($C_{1-4}$-alkyl)amino oder eine Gruppe (a) oder (b)

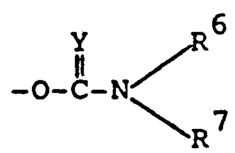

(a)

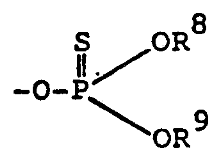

(b)

$R^3$ Wasserstoff, Fluor, Chlor, $C_{1-6}$ -Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkyl-thio, $C_{1-4}$-Alkylamino, Di($C_{1-4}$-alkyl)amino oder unsubstituiertes, monosubstituiertes oder disubstituiertes Phenyl, wobei die Substituenten Fluor Chlor Trifluormethyl und/oder Methoxy sind,

$R^4$ $C_{1-3}$-Alkyl,

$R^5$ $C_{1-6}$-Alkyl,

$R^6$ und $R^7$ bzw. $R^8$ und $R^9$ unabhängig voneinander $C_{1\ 3}$-Alkyl

und X und Y unabhängig voneinander Sauerstoff oder Schwefel bedeuten,

dadurch gekennzeichnet, dass man

a) ein Pyrimidinderivat der allgemeinen Formel

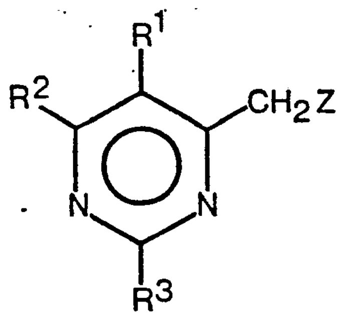

II

worin $R^1$, $R^2$ und $R^3$ die oben angegebenen Bedeutungen besitzen und Z eine Abgangruppe bedeutet, mit einem Dithio- bzw. Trithiophosphat der allgemeinen Formel

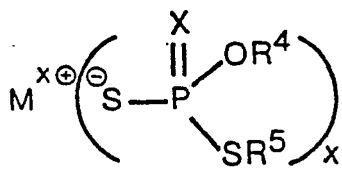

III

worin $R^4$, $R^5$ und X die oben angegebenen Bedeutungen besitzen, $M^{x\oplus}$ für ein Alkalimetall-, Erdalkalimetalloder gegebenenfalls substituiertes Ammoniumion steht und x die Wertigkeit des Kations $M^{x\oplus}$ bedeutet, umsetzt oder

b) ein Pyrimidinderivat der allgemeinen Formel

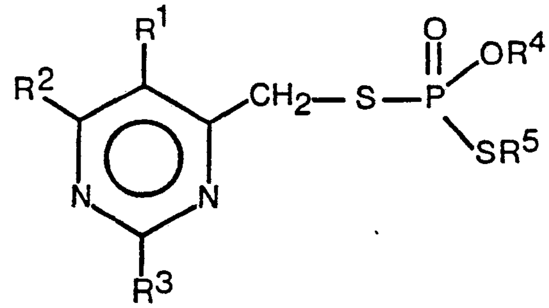

I'

worin $R^1$, $R^2$, $R^3$, $R^4$ und $R^5$ die oben angegebenen Bedeutungen besitzen, mit Ausnahme der Derivate I', worin $R^2$

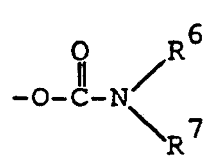

ist, mit einem Schwefelungsmittel behandelt.

29. Verfahren nach Anspruch 28, worin $R^2$ Wasserstoff, Fluor, Chlor, $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, Di($C_{1-4}$-alkyl)amino oder unsubstituiertes, monosubstituiertes oder disubstituiertes Phenyl bedeuten, wobei die Substituenten Fluor, Chlor, Trifluormethyl und/oder Methoxy sind, und $R^1$, $R^4$, $R^5$ und X die in Anspruch 28 angegebenen Bedeutungen besitzen.

30. Verfahren zur Bekämpfung von Schädlingen, dadurch gekennzeichnet, dass man das zu schützende Gut oder die Schädlinge selbst mit einer wirksamen Menge einer der in den Ansprüchen 1, 6, 12,14 und 22 genannten Verbindungen bzw. eines in Anspruch 24 genannten Mittels behandelt.

31. Verfahren zur Bekämpfung von Schädlingen, dadurch gekennzeichnet, dass man das zu schützende Gut oder die Schädlinge selbst mit einer wirksamen Menqe einer der in den Ansprüchen 2 bis 5,7 bis 11, 13 und 15

bis 21 genannten Verbindungen bzw. eines in Anspruch 25 genannten Mittels behandelt.

32 Verwendung einer der in den Ansprüchen 1, 6, 12, 14 und 22 genannten Verbindungen bzw. eines in Anspruch 24 genannten Mittels zur Bekämpfung von Schädlingen.

33. Verwendung einerder inden Ansprüchen 2 bis 5, 7 bis 11, 13 und 15 bis 21 genannten Verbindungen bzw. eines in Anspruch 25 genannten Mittels zur Bekämpfung von Schädlingen.

**Patentansprüche** für den Vertragsstaat AT

1. Schädlingsbekämpfungsmittel, dadurch gekennzeichnet, dass es eine wirksame Menge mindestens einer Verbindung der allgemeinen Formel

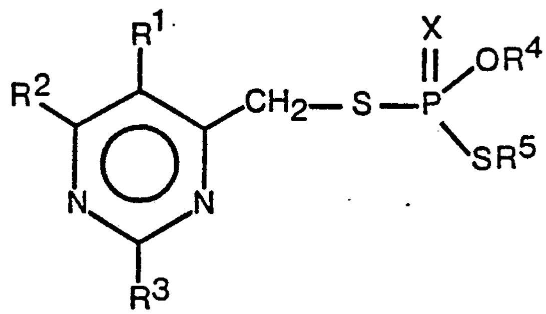

I

worin $R^1$ Wasserstoff, Fluor oder Chlor,

$R^2$ Wasserstoff, Fluor, Chlor, $C_{1-4}$-Alkyl, $C_{1-6}$-Alkoxy, 2-Propenyloxy, 2-Propinyloxy, 2-($C_{1-4}$-Alkoxy)-äthoxy, unsubstituiertes oder mit Halogen mono- oder disubstituiertes Phenoxy, $C_{1-6}$-Alkylthio, $C_{1-4}$-Alkylamino, Di($C_{1-4}$-alkyl)amino oder eine Gruppe (a) oder (b)

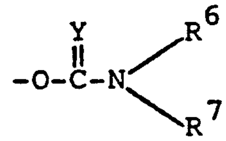

(a)

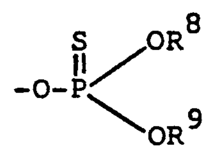

(b)

$R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, $C_{1-4}$-Alkylamino, Di($C_{1-4}$-alkyl)amino oder unsubstituiertes, monosubstituiertes oder disubstituiertes Phenyl, wobei die Substituenten Fluor, Chlor, Trifluormethyl und/oder Methoxy sind,

$R^4$ $C_{1-3}$-Alkyl,

$R^5$ $C_{1-6}$-Alkyl,

$R^6$ $R^7$ bzw. $R^8$ und $R^9$ unabhängig voneinander $C_{1-3}$-Alkyl

und X und Y unabhängig voneinander Sauerstoff oder Schwefel bedeuten,

sowie Formulierungshilfsstoffe enthält.

2. Schädlingsbekämpfungsmittel nach Anspruch 1, worin $R^2$ Wasserstoff, Fluor, Chlor, $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, Di($C_{1-4}$-alkyl)-amino oder unsubstituiertes, monosubstituiertes oder disubstituiertes Phenyl bedeuten, wobei die Substituenten Fluor, Chlor, Trifluormethyl und/oder Methoxy sind, und $R^1$, $R^4$, $R^5$ und X die in Anspruch 1 angegebenen Bedeutungen besitzen.

3. Schädlingsbekämpfungsmittel nach Anspruch 2, worin $R^1$ Wasserstoff bedeutet.

4. Schädlingsbekämpfungsmittel nach Anspruch 2 oder 3, worin $R^2$ Methoxy, Aethoxy, Isopropoxy oder 2-Propinyloxy bedeutet.

5. Schädlingsbekämpfungsmittel nach einem der Ansprüche 2 bis 4, worin $R^3$ Wasserstoff, Methyl, Isopropyl, tert.Butyl, Cyclopropyl, Methoxy, Aethoxy, Methylthio, Aethylthio, Dimethylamino oder Diäthylamino bedeutet.

6. Schädlingsbekämpfungsmittel nach Anspruch 1, worin $R^3$ Isopropylamino bedeutet.

7. Schädlingsbekämpfungsmittel nach einem der Ansprüche 2 bis 5, worin $R^4$ Aethyl bedeutet.

8. Schädlingsbekämpfungsmittel nach einem der Ansprüche 2 bis 5 und 7, worin $R^5$ n-Propyl bedeutet.

9. Schädlingsbekämpfungsmittel nach einem der Ansprüche 2 bis 5, 7 und 8, worin X Sauerstoff bedeutet.

10. Schädlingsbekämpfungsmittel nach Anspruch 2, worin $R^2$ und/oder $R^3$ $C_{1-6}$-Alkoxy, 2-Propenyloxy oder 2-Propinyloxy bedeutet.

11. Schädlingsbekämpfungsmittel nach Anspruch 2, worin $R^1$, $R^2$ und/ oder $R^3$ Chlor bedeutet.

12. Schädlingsbekämpfungsmittel nach Anspruch 1, worin $R^2$ 2-($C_{1-4}$-Alkoxy)-äthoxy oder unsubstituiertes oder mit Halogen mono- oder disubstituiertes Phenoxy bedeutet.

13. Schädlingsbekämpfungsmittel nach Anspruch 2, worin $R^2$ und/oder $R^3$ $C_{1-6}$-Alkylthio bedeutet.

14. Schädlingsbekämpfungsmittel nach Anspruch 1, worin $R^2$ und/oder $R^3$ $C_{1-4}$-Alkylamino oder Di($C_{1-4}$-alkyl)-amino bedeutet.

15. Schädlingsbekämpfungsmittel nach Anspruch 2, worin $R^2$ $C_{1-4}$-Alkyl und $R^3$ $C_{1-6}$-Alkyl, $C_{3-6}$-Cycloalkyl, ($C_{1-4}$-Alkoxy)methyl, $C_{1-6}$-Alkoxy, Di($C_{1-4}$-alkyl)amino oder gegebenenfalls mit Methoxy substituiertes Phenyl bedeuten.

16. Schädlingsbekämpfungsmittel nach Anspruch 2, worin $R^1$, $R^2$ und/ oder $R^3$ Wasserstoff bedeutet.

17. Schädlingsbekämpfungsmittel nach Anspruch 2, worin $R^1$, $R^2$ und/ oder $R^3$ Fluor bedeutet.

18. Schädlingsbekämpfungsmittel nach Anspruch 2, worin $R^2$ eine Gruppe (a) oder (b) bedeutet.

19. Schädlingsbekämpfungsmittel nach Anspruch 2, dadurch gekennzeichnet, dass es eine wirksame Menge von Dithiophosphorsäure-[O-äthyl-S-(2,6-diäthoxy-4-pyrimidinyl-methyl)-S-(n-propyl)]ester sowie Formulierungshilfsstoffe enthält.

20. Schädlingsbekämpfungsmittel nach Anspruch 2, dadurch gekennzeichnet, dass es eine wirksame Menge von Dithiophosphorsäure-[O-äthyl-S-[2-isopropyl-6-(2-propinyl-oxy)-4-pyrimidinylmethyl]-S-(n-propyl)]ester sowie Formulierungshilfsstoffe enthält.

21. Schädlingsbekämpfungsmittel nach Anspruch 2, dadurch gekennzeichnet, dass es eine wirksame Menge mindestens einer Verbindung ausgewählt aus:

Dithiophosphorsäure-[O-äthyl-S-(6-äthoxy-2-methyl-4-pyrimidinylmethyl)-S-(n-propyl)]ester,
Dithiophosphorsäure-[O-äthyl-S-(sek. butyl)-S-(6-äthoxy-2-methyl-4-pyrimidinylmethyl)]ester,
Dithiophosphorsäure-[O-äthyl-S-(6-äthoxy-2-cyclo-propyl-4-pyrimidinylmethyl)-S-(n-propyl)]ester,
Dithiophosphorsäure-[O-äthyl-S-[2-(tert. butyl)-6-chlor-4-pyrimidinylmethyl]-S-(n-propyl)]ester,
Dithiophosphorsäure-[O-äthyl-S-(sek. butyl)-S-(2,6-diäthoxy-4-pyrimidinylmethyl)]ester,
Dithiophosphorsäure-[O-äthyl-S-[6-äthoxy-2-(tert. butyl)-4-pyrimidinylmethyl]-S-(n-propyl)]ester,
Dithiophosphorsäure-[O-äthyl-S-[2-(tert. butyl)-6-methoxy-4-pyrimidinylmethyl]-S-(n-propyl)]ester,
Dithiophosphorsäure-[O-äthyl-S-(2-chlor-6-dimethyl-amino-4-pyrimidinylmethyl)-S-(n-propyl)]ester,
Dithiophosphorsäure-[O-äthyl-S-(2,6-dichlor-4-pyrimidinylmethyl)-S-(n-propyl)]ester,
Dithiophosphorsäure-[O-äthyl-S-(2-chlor-6-methoxy-4-pyrimidinylmethyl)-S-(n-propyl)]ester,
Dithiophosphorsäure-[O-äthyl-S-(6-chlor-2-isopropyl-4-pyrimidinylmethyl)-S-(n-propyl)]ester,
Dithiophosphorsäure-[O-äthyl-S-(6-äthoxy-2-isopropyl-4-pyrimidinylmethyl)-S-(n-propyl)]ester,
Dithiophosphorsäure-[O-äthyl-S-(2,6-dimethoxy-4-pyrimidinylmethyl)-S-(n-propyl)]ester,
Dithiophosphorsäure-[O-äthyl-S-(2-äthyl-6-chlor-4-pyrimidinylmethyl)-S-(n-propyl)]ester,
Dithiophosphorsäure-[O-äthyl-S-(6-äthoxy-2-äthyl-4-pyrimidinylmethyl)-S-(n-propyl)]ester,
Dithiophosphorsäure-[O-äthyl-S-(6-dimethylcarbamoyloxy-2-methyl-4-pyrimidinylmethyl)-S-(n-propyl)]ester,
Dithiophosphorsäure-[O-äthyl-S-[6-(O,O-dimethyl-thiophosphoro)-2-methyl-4-pyrimidinylmethyl]-S-(n-propyl)]ester,
Dithiophosphorsäure-[O-äthyl-S-(6-äthoxy-2-methylthio-4-pyrimidinylmethyl)-S-(n-propyl)]ester,
Dithiophosphorsäure-[O-äthyl-S-(6-äthylthio-2-cyclo-propyl-4-pyrimidinylmethyl)-S-(n-propyl)]ester,
Dithiophosphorsäure-[O-äthyl-S-(2,6-diisopropoxy-4-pyrimidinylmethyl)-S-(n-propyl)]ester,
Dithiophosphorsäure-[O-äthyl-S-(6-isopropoxy-2-iso-propyl-4-pyrimidinylmethyl)-S-(n-propyl)]ester,
Dithiophosphorsäure-[O-äthyl-S-[2-isopropyl-6-(2-propenyloxy)-4-pyrimidinylmethyl]-S-(n-propyl)]ester,
Dithiophosphorsäure-[O-äthyl-S-(6-äthoxy-2-chlor-4-pyrimidinylmethyl)-S-(n-propyl)]ester,
Dithiophosphorsäure-[O-äthyl-S-(2,6-diäthylthio-4-pyrimidinylmethyl)-S-(n-propyl)]ester,
Dithiophosphorsäure-[O-äthyl-S-[6-äthoxy-2-(p-chlor-phenyl)-4-pyrimidinylmethyl]-S-(n-propyl)]ester,
Trithiophosphorsäure-[O-äthyl-S-(2,6-diäthoxy-4-pyrimidinylmethyl)-S-(n-propyl)]ester,
Trithiophosphorsäure-[O-äthyl-S-(2,6-dichlor-4-pyrimidinylmethyl)-S-(n-propyl)]ester und
Trithiophosphorsäure-[O-äthyl-S-(6-äthoxy-2-methyl-4-pyrimidinylmethyl)-S-(sek. butyl)]ester, sowie Formulierungshilfsstoffe enthält.

22. Schädlingsbekämpfungsmittel nach Anspruch 1, dadurch gekennzeichnet, dass es eine wirksame Menge mindestens einer Verbindung ausgewählt aus:

Dithiophosphorsäure-[O-äthyl-S-[2-(p-chlorphenyl)-6-(O,O-diäthyl-thiophosphoro)-4-pyrimidinylmethyl]-S-(n-propyl)]ester,

Dithiophosphorsäure-[O-äthyl-S-äthyl-S-(2,6-diäthoxy-4-pyrimidinylmethyl]ester, Dithiophosphorsäure-[O-äthyl-S-(6-dimethylthiocarbamoyl-oxy-2-methyl-4-pyrimidinylmethyl)-S-(n-propyl)]ester, Dithiophosphorsäure-[O-äthyl-S-(2-methyl-6-phenoxy-4-pyrimidinylmethyl)-S-(n-propyl)]ester, Dithiophosphorsäure-[O-äthyl-S-(6-dimethylamino-2-methyl-4-pyrimidinylmethyl)-S-(n-propyl)]ester, Dithiophosphorsäure-[O-äthyl-S-(2-äthoxy-6-dimethyl-carbamoyloxy-4-pyrimidinylmethyl)-S-(n-propyl)]ester, Dithiophosphorsäure-[O-äthyl-S-(2-diäthylamino-6-chlor-4-pyrimidinylmethyl)-S-(n-propyl)]ester, Dithiophosphorsäure-[O-äthyl-S-(6-chlor-2-isopropyl-amino-4-pyrimidinylmethyl)-S-(n-propyl)]ester, Dithiophosphorsäure-[O-äthyl-S-(2-chlor-6-diäthylamino-4-pyrimidinylmethyl)-S-(n-propyl)]ester, Dithiophosphorsäure-[O-äthyl-S-(2-chlor-6-isopropyl-amino-4-pyrimidinylmethyl)-S-(n-propyl)]ester, Dithiophosphorsäure-[O-äthyl-S-(sek. butyl)-S-[2-isopropyl-6-(2-propinyloxy)-4-pyrimidinylmethyl]]ester, Dithiophosphorsäure-[O-äthyl-S-(6-äthoxy-2-methoxy-4-pyrimidinylmethyl)-S-(n-propyl)]ester, Dithiophosphorsäure-[O-äthyl-S-[2-methoxy-6-(2-pro-pinyloxy)-4-pyrimidinylmethyl]-S-(n-propyl)]ester, Dithiophosphorsäure-[O-äthyl-S-(6-diäthylamino-2-methoxy-4-pyrimidinylmethyl)-S-(n-propyl)]ester, Dithiophosphorsäure-[O-äthyl-S-(6-äthoxy-2-diäthyl-amino-4-pyrimidinylmethyl)-S-(n-propyl)]ester, Dithiophosphorsäure-[O-äthyl-S-(6-isopropoxy-2-methoxy-4-pyrimidinylmethyl)-S-(n-propyl)]ester, Dithiophosphorsäure-[O-äthyl-S-(6-methoxy-4-pyrimi-dinylmethyl)-S-(n-propyl)]ester, Dithiophosphorsäure-[O-äthyl – S-(6-äthoxy-4-pyrimi-dinylmethyl)-S-(n-propyl)]ester, Dithiophosphorsäure-[O-äthyl-S-[6-(2-propinyloxy)-4-pyrimidinylmethyl]-S-(n-propyl)]ester, Dithiophosphorsäure-[O-äthyl-S-(6-phenoxy-4-pyrimi-dinylmethyl)-S-(n-propyl)]ester, Dithiophosphorsäure-[O-äthyl-S-(2-isopropyl-6-methyl-4-pyrimidinylmethyl)-S-(n-propyl)]ester, Dithiophosphorsäure-[O-äthyl-S-[2-isopropyl-6-(2-methoxyäthoxy)-4-pyrimidinylmethyl]-S-(n-propyl)]ester, Dithiophosphorsäure-[O-äthyl-S-(6-fluor-2-isopropyl-4-pyrimidinylmethyl)-S-(n-propyl)]ester, Dithiophosphorsäure-[O-äthyl-S-(2-chlor-6-fluor-4-pyrimidinylmethyl)-S-(n-propyl)]ester, Dithiophosphorsäure-[O-äthyl-S-(2-äthoxy-6-isopropoxy-4-pyrimidinylmethyl)-S-(n-propyl)]ester, Dithiophosphorsäure-[S-(2,6-diäthoxy-4-pyrimidinyl-methyl)-O-methyl-S-methyl]ester, Dithiophosphorsäure-[S-(2,6-diäthoxy-4-pyrimidinyl-methyl)-O-methyl-S-(n-propyl)]ester und Dithiophosphorsäure-[O-äthyl-S-(5-chlor-2,6-diäthoxy-4-pyrimidinylmethyl)-S-(n-propyl)]ester, sowie Formulierungshilfsstoffe enthält.

23. Verfahren zur Herstellung von Verbindungen der allgemeinen Formel

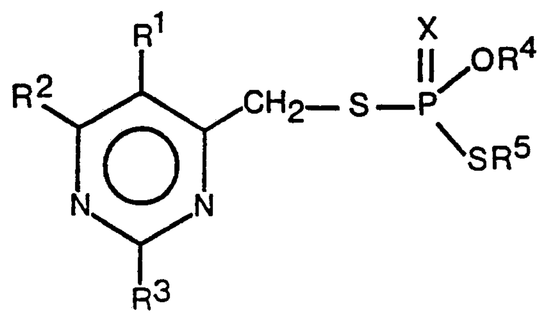

I

worin $R^1$ Wasserstoff Fluor oder Chlor,

$R^2$ Wasserstoff Fluor, Chlor $C_{1-4}$-Alkyl, $C_{1-6}$-Alkoxy, 2-Propenyloxy, 2-Propinyloxy, 2-($C_{1-4}$-Alkoxy)-äthoxy, unsubstituiertes oder mit Halogen mono- oder disubstituiertes Phenoxyt, $C_{1-6}$-Alkylthio $C_{1-4}$-Alkylamino, Di($C_{1-4}$-alkyl)amino oder eine Gruppe (a) oder (b)

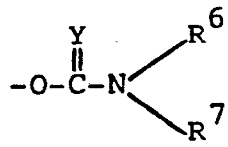

(a)

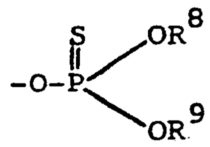

(b)

$R^3$ Wasserstoff Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkyl-thio, $C_{1-4}$-Alkylamino, Di($C_{1-4}$-alkyl)amino oder unsubstituiertes, monosubstituiertes oder disubstituiertes Phenyl, wobei die Substituenten Fluor, Chlor, Trifluormethyl und/oder Methoxy sind,
$R^4$ $C_{1-3}$-Alkyl,
$R^5$ $C_{1-6}$-Alkyl,
$R^6$ und $R^7$ bzw. $R^8$ und $R^9$ unabhängig voneinander $C_{1-3}$-Alkyl
und X und Y unabhängig voneinander Sauerstoff oder Schwefel bedeuten,
dadurch gekennzeichnet, dass man
a) ein Pyrimidinderivat der allgemeinen Formel

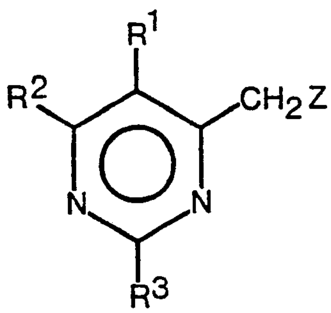

II

worin $R^1$ $R^2$ und $R^3$ die oben angegebenen Bedeutungen besitzen
und Z eine Abgangsgruppe bedeutet,
mit einem Dithio- bzw. Trithiophosphat der allgemeinen Formel

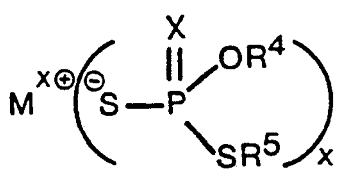

III

worin $R^4$, $R^5$ und X die oben angegebenen Bedeutungen besitzen,
$M^{x\oplus}$ für ein Alkalimetall, Erdalkalimetall- oder gegebenenfalls substituiertes Ammoniumion steht
und x die Wertigkeit des Kations $M^{x\oplus}$ bedeutet, umsetzt oder
b) ein Pyrimidinderivat der allgemeinen Formel

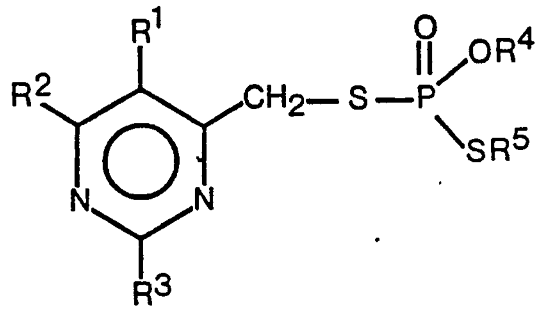

I'

worin $R^1$, $R^2$, $R^3$, $R^4$ und $R^5$ die oben angegebenen Bedeutungen besitzen, mit Ausnahme der Derivate I', worin $R^2$

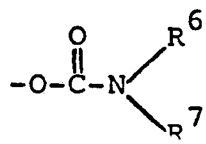

ist, mit einem Schwefelungsmittel behandelt.

24. Verfahren nach Anspruch 23, worin $R^2$ Wasserstoff, Fluor, Chlor, $C_{1-4}$-Alkyl, $C_{1-6}$-Alkoxy, 2-Propenyloxy, 2-Propinyloxy, $C_{1-6}$-Alkylthio, Di($C_{1-4}$-alkyl)amino oder eine Gruppe (a) oder (b) und $R^3$ Wasserstoff, Fluor, Chlor, $C_{1-6}$-Alkyl, $C_{3-6}$-Cycloalkyl, ($C_{1-4}$-Alkoxy)methyl, ($C_{1-4}$-Alkylthio)methyl, $C_{1-6}$-Alkoxy, 2-Propenyloxy, 2-Propinyloxy, $C_{1-6}$-Alkylthio, Di($C_{1-4}$-alkyl)amino oder unsubstituiertes, monosubstituiertes oder disubstituiertes Phenyl bedeuten, wobei die Substituenten Fluor, Chlor, Trifluormethyl und/oder Methoxy sind, und $R^1$, $R^4$, $R^5$ und X die in Anspruch 23 angegebenen Bedeutungen besitzen.

25. Verfahren zur Herstellung eines Schädlingsbekämpfungsmittels, dadurch gekennzeichnet, dass man mindestens eine der in den Ansprüchen 1 bis 22 genannten Verbindungen mit Formulierungshilfsstoffen vermischt.

26. Verfahren zur Bekämpfung von Schädlingen, dadurch gekennzeichnet, dass man das zu schützende Gut oder die Schädlinge selbst mit einer wirksamen Menge einer bzw. eines der in den Ansprüchen 1 bis 22 genannten Verbindungen bzw. Schädlingsbekämpfungsmittel behandelt.

27. Verwendung einer bzw. eines der in den Ansprüchen 1 bis 22 genannten Verbindungen bzw. Mittel zur Bekämpfung von Schädlingen.

**Revendications** pour les Etats Contractants BE, CH, DE, FR, GB, IT, LI, LU, NL, SE

1. Composés répondant à la formule générale

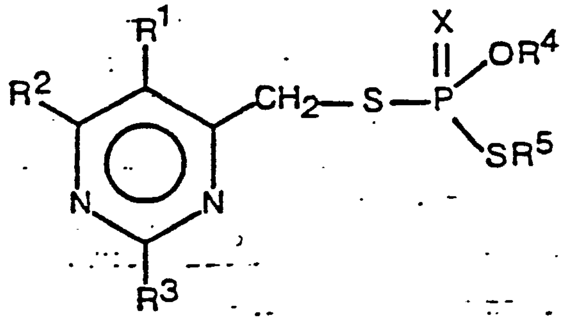

I

dans laquelle $R^1$ représente l'hydrogène, le fluor ou le chlore,

$R^2$ représente l'hydrogène, le fluor, le chlore, un alkyle en $C_1$ à $C_4$, un alcoxy en $C_1$ à $C_6$, un 2-propényloxy, un 2-propinyloxy, un (alcoxy $C_{1-4}$)-2 éthoxy un phénoxy non substitué ou mono- ou disubstitué par un halogène, un alkyl $C_{1-6}$thio, un alkyl $C_{1-4}$amino, un di(alkyl $C_{1-4}$)amino ou un groupe (a) ou (b)

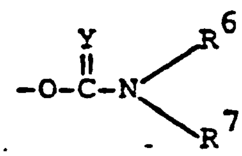

(a)

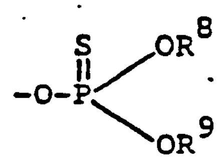

(b)

$R^3$ représente l'hydrogène, le fluor, le chlore, un alkyle en $C_1$ à $C_6$ un cycloalkyle $C_{3-6}$, un (alcoxy $C_{1-4}$) méthyle, un (alkyl $C_{1-4}$ thio)méthyle, un alcoxy $C_{1-6}$, un 2-propényloxy, un 2-propinyloxy, un alkyl $C_{1-6}$thio, un alkyl $C_{1-4}$amino, un di(alkyl $C_{1-4}$)amino ou un phényle non substitué, monosubstitué ou disubstitué, les substituants étant le fluor, le chlore, un trifluorométhyle et/ou un méthoxy,

$R^4$ représente un alkyle en $C_1$ à $C_3$

$R^5$ représente un alkyle en $C_1$ à $C_6$

$R^6$ et $R^7$, respectivement $R^8$ et $R^9$ représentent, indépendamment l'un de l'autre, un alkyle en $C_1$ à $C_3$

et X et Y représentent, indépendamment l'un de l'autre, l'oxygène ou le soufre.

2. Composés selon la revendication 1, dans lesquels $R^2$ représente l'hydrogène, le fluor, le chlore, un alkyle en $C_1$ à $C_4$, un alcoxy en $C_1$ à $C_6$, un 2-propényloxy, un 2-propi-nyloxy, un alkyl $C_{1-6}$thio, un di(alkyl $C_{1-4}$)amino ou un groupe (a) ou (b) et $R^3$ représente l'hydrogène, le fluor, le chlore, un alkyle en $C_1$ à $C_6$, un cycloalkyle $C_3$-$C_6$, un (alcoxy $C_{1-4}$) méthyle, un (alkyl $C_{1-4}$thio)méthyle, un alcoxy en $C_1$ à $C_6$, un 2-propényloxy, un 2-propinyloxy, un alkyl $C_{1-6}$thio, un di(alkyl $C_{1-4}$)amino, un phényle non substitué, monosubstitué ou disubstitué, les substituants étant le fluor, le chlore, un trifluorométhyle et/ou un méthoxy et $R^1$, $R^4$, $R^5$ et X ayant les significations indiquées dans la revendication 1.

3. Composés selon la revendication 2, dans lesquels $R^1$ représente l'hydrogène.

4. Composés selon la revendication 2 ou 3, dans lesquels $R^2$ représente un méthoxy, un éthoxy, un isopropoxy ou un 2-propinyloxy.

5. Composés selon l'une des revendications 2 à 4, dans lesquels $R^3$ représente l'hydrogène, un méthyle, un isopropyle, un tert.butyle, un cyclopropyle, un méthoxy, un éthoxy, un méthylthio, un éthylthio, un diméthylamino ou un diéthylamino.

6. Composés selon la revendication 1, dans lesquels $R^3$ représente un isopropylamino.

7. Composés selon l'une des revendications 2 à 5, dans lesquels $R^4$ représente un éthyle.

8. Composés selon l'une des revendications 2 à 5 et 7, dans lesquels $R^5$ représente un n-propyle.

9. Composés selon l'une des revendications 2 à 5, 7 et 8, dans lesquels X représente l'oxygène.

10. Composés selon la revendication 2, dans lesquels $R^2$ et/ou $R^3$ représente un alcoxy en $C_{1-6}$, un 2-propényloxy ou un 2-propinyloxy.

11. Composés selon la revendication 2, dans lesquels $R^1$, $R^2$ et/ou $R^3$ représentent le chlore.

12. Composés selon la revendication 1, dans lesquels $R^2$ représente un (alcoxy $C_{1-4}$)-2-éthoxy ou un phénoxy non substitué ou mono- ou disubstitué par un halogène.

13. Composes selon la revendication 2, dans lesquels $R^2$ et/ou $R^3$ représentent un alkyl $C_{1-6}$thio.

14. Composés selon la revendication 1, dans lesquels $R^2$ et/ou $R^3$ représentent un alkyl $C_{1-4}$amino ou un di(alkyl $C_{1-4}$)amino.

15. Composés selon la revendication 2, dans lesquels $R^2$ représente un alkyle en $C_{1-4}$ et $R^3$ représente un allyle en $C_{1-6}$, un cycloalkyle $C_{3-6}$, un (alcoxy $C_{1-4}$)méthyle, un alcoxy en $C_{1-6}$, un di(alkyl $C_{1-4}$)amino ou un phényle éventuellement substitué par un méthoxy.

16. Composés selon la revendication 2, dans lesquels $R^1$, $R^2$ et/ou $R^3$ représentent l'hydrogène.

17. Composés selon la revendication 2, dans lesquels $R^1$, $R^2$ et/ou $R^3$ représentent le fluor.

18. Composés selon la revendication 2, dans lesquels $R^2$ représente un groupe (a) ou (b).

19. Dithiophosphate de O-éthyle et de S-(diétho-xy-2,6 pyrimidinyl-4 méthyle), S-(n-propyle).

20. Dithiophosphate de O-éthyle et de S-[isopro-pyl-2 (2 propinyloxy)-6 pyrimidinyl-4 méthyle], S-(n-propyle).

21. Un composé selon la revendication 2, choisi parmi:
le dithiophosphate de O-éthyle et de S-(éthoxy-6 méthyl-2 pyrimidinyl-4 méthyle), S-(n-propyle),
le dithiophosphate de O-éthyle et de S-(sec.butyle), S-(éthoxy-6 méthyl-2 pyrimidinyl-4 méthyle),
le dithiophosphate de O-éthyle et de S-(éthoxy-6 cyclopropyl-2 pyrimidinyl-4 méthyle), S-(n-propyle),
le dithiophosphate de O-éthyle et de S-[(tert.butyl)-2 chlo-ro-6 pyrimidinyl-4 méthyle], S-(n-propyle), le dithiophosphate de O-éthyle et de S-(sec.butyle), S-(diétho-xy-2,6 pyrimidinyl-4 méthyle),
le dithiophosphate de O-éthyle et de S-[éthoxy-6 (tert.butyl)-2 pyrimidinyl-4 méthyle], S-(n-propyle),
le dithiophosphate de O-éthyle et de S-[(tert.bu-tyl)-2 méthoxy-6 pyrimidinyl-4 méthyle],S-(n-propyle),
le dithiophosphate de O-éthyle et de S-(chloro-2 diméthylamino-6 pyrimidinyl-4 méthyle), S-(n-propyle),
le dithiophosphate de O-éthyle et de S-(dichloro-2,6 pyrimidinyl-4 méthyle), S-(n-propyle),
le dithiophosphate de O-éthyle et de S-(chloro-2 méthoxy-6 pyrimidinyl-4 méthyle), S-(n-propyle),
le dithiophosphate de O-éthyle et de S-(chloro-6 isopropyl-2 pyrimidinyl-4 méthyle), S-(n-propyle),
le dithiophosphate de O-éthyle et de S-(éthoxy-6 isopropyl-2 pyrimidinyl-4 méthyle), S-(n-propyle),
le dithiophosphate de O-éthyle et de S-(dimétho-xy-2,6 pyrimidinyl-4 méthyle), S-(n-propyle),
le dithiophosphate de O-éthyle et de S-(éthyl-2 chloro-6 pyrimidinyl-4 méthyle), S-(n-propyle),
le dithiophosphate de O-éthyle et de S-(éthoxy-6 éthyl-2 pyrimidinyl-4 méthyle), S-(n-propyle),
le dithiophosphate de O-éthyle et de S-(diméthylcarbamoyloxy- méthyl-2 pyrimidinyl-4 méthyl), S-(n-propyle),
le dithiophosphate de O-éthyle et de S-[(O,O-dime-thylthiophosphoro)-6 méthyle-2 pyrimidinyl-4 méthyle], S(n-propyle),
le dithiophosphate de O-éthyle et de S-(éthoxy-6 méthylthio-2 pyrimidinyl-4 méthyle), S-(n-propyle),
le dithiophosphate de O-éthyle et de S-(éthylthio-6 cyclopropyl-2 pyrimidinyl-4 méthyle), S-(n-propyle),
le dithiophosphate de O-éthyle et de S-(diisopro-poxy-2,6 pyrimidinyl-4 méthyle), S-(n-propyle),
le dithiophosphate de O-éthyle et de S-(isopropo-xy-6 isopropyl-2 pyrimidinyl-4 méthyle), S-(n-propyle),
le dithiophosphate de O-éthyle et de S-[isopropyl-2(2-propényloxy)-6 pyrimidinyl-4 méthyle], S-(n-propyle),
le dithiophosphate de O-éthyle et de S-(éthoxy-6 chloro-2 pyrimidinyl-4 méthyle), S-(n-propyle),
le dithiophosphate de O-éthyle et de S-(diéthyl-thio-2,6 pyrimidinyl-4 méthyle), S-(n-propyle),
le dithiophosphate de O-éthyle et de S-[éthoxy-6 (p-chlorophényl)-2 pyrimidinyl-4 méthyle], S-(n-propyle),
le trithiophosphate de O-éthyle et de S-(diéthoxy-2,6 pyrimidinyl-4 méthyle), S-(n-propyle),
le trithiophosphate de O-éthyle et de S-(dichloro-2,6 pyrimidinyl-4 méthyle), S-(n-propyle) et
le trithiophosphate de O-éthyle et de S-(éthoxy-6 méthyl-2 pyrimidinyl-4 méthyle), S-(sec.butyle).

22. Un composé selon la revendication 1, choisi parmi:
le dithiophosphate de O-éthyle et de S-[(p-chloro-phényl)-2 (O,O-diéthylthiophosphoro)-6 pyrimidinyl-4 méthyle], S-(n-propyle),
le dithiophosphate de O-éthyle et de S-éthyle, S-(diéthoxy-2,6 pyrimidinyl-4 méthyle),
le dithiophosphate de O-éthyle et de S-(diméthyl-thiocarbamoyloxy-6 méthyle-2 pyrimidinyl-4 méthyle), S-(n-propyle),
le dithiophosphate de O-éthyle et de S-(méthyl-2 phénoxy-6 pyrimidinyl-4 méthyle), S-(n-propyle),
le dithiophosphate de O-éthyle et de S-(diméthyl-amino-6 méthyl-2 pyrimidinyl-4 méthyle), S-(n-propyle),
le dithiophosphate de O-éthyle et de S-(éthoxy-2 diméthylcarbamoyloxy-6 pyrimidinyl-4 méthyle), S-(n-propyle),
le dithiophosphate de O-éthyle et de S-(diéthyl-amino-2 chloro-6 pyrimidinyl-4 méthyle), S-(n-propyle),
le dithiophosphate de O-éthyle et de S-(chloro-6 isopropylamino-2 pyrimidinyl-4 méthyle), S-(n-propyle),
le dithiophosphate de O-éthyle et de S-(chloro-2 diéthylamino-6 pyrimidinyl-4 méthyle), S-(n-propyle),
le dithiophosphate de O-éthyle et de S-(chloro-2 isopropylamino-6 pyrimidinyl-4 méthyle), S-(n-propyle),
le dithiophosphate de O-éthyle et de S-(sec.butyle), S-[isopropyl-2 (2-propinyloxy)-6 pyrimidinyl-4 méthyle],
le dithiophosphate de O-éthyle et de S-(éthoxy-6 méthoxy-2 pyrimidinyl-4 méthyle), S-(n-propyle),
le dithiophosphate de O-éthyle et de S-[méthoxy-2 (2-propinyloxy)-6 pyrimidinyl-4 méthyle], S-(n-propyle),

le dithiophosphate de O-éthyle et de S-(diéthyla-mino-6 méthoxy-2 pyrimidinyl-4 méthyle), S-(n-propyle),
le dithiophosphate de O-éthyle et de S-(éthoxy-6 diéthylamino-2 pyrimidinyl-4 méthyle), S-(n-propyle),
le dithiophosphate de O-éthyle et de S-(isopropoxy-6 méthoxy-2 pyrimidinyl-4 méthyle), S-(n-propyle),
le dithiophosphate de O-éthyle et de S-(méthoxy-6 pyrimidinyl-4 méthyle), S-(n-propyle),
le dithiophosphate de O-éthyle et de S-(éthoxy-6 pyrimidinyl-4 méthyle), S-(n-propyle),
le dithiophosphate de O-éthyle et de S-[(2-propiny-loxy)-6 pyrimidinyl-4 méthyle], S-(n-propyle),
le dithiophosphate de O-éthyle et de S-(phénoxy-6 pyrimidinyl-4 méthyle), S-(n-propyle),
le dithiophosphate de O-éthyle et de S-(isopropyl-2 méthyl-6 pyrimidinyl-4 méthyle), S-(n-propyle),
le dithiophosphate de O-éthyle et de S-[isopropyl-2 (méthoxy-2 éthoxy)-6 pyrimidinyl-4 méthyle], S-(n-propyle),
le dithiophosphate de O-éthyle et de S-(fluoro-6 isopropyl-2 pyrimidinyl-4 méthyle), S-(n-propyle),
le dithiophosphate de O-éthyle et de S-(chloro-2 fluoro-6 pyrimidinyl-4 méthyle), S-(n-propyle),
le dithiophosphate de O-éthyle et de S-(éthoxy-2 isopropoxy-6 pyrimidinyl-4 méthyle), S-(n-propyle),
le dithiophosphate de S-(diéthoxy-2,6 pyrimidi-nyl-4 méthyle) et de O-méthyle, S-méthyle,
le dithiophosphate de S-(diéthoxy-2,6 pyrimidinyl-4 méthyle) et de O-méthyle, S-(n-propyle) et
le dithiophosphate de O-éthyle et de S-(chloro-5 diéthoxy-2,6 pyrimidinyl-4 méthyle), S-(n-propyle).

23. Un composé selon la revendication 1 en tant que pesticide.

24. Pesticide, caractérisé en ce qu'il contient une quantité efficace d'au moins un composé de formule générale

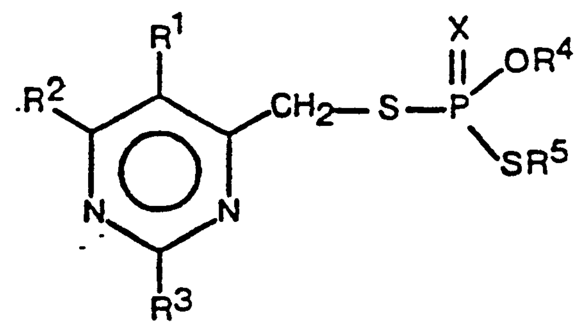

I

dans laquelle $R^1$ représente l'hydrogène, le fluor ou le chlore,

$R^2$ représente l'hydrogène, le fluor, le chlore, un alkyle en $C_1$ à $C_4$, un alcoxy en $C_1$ à $C_6$, un 2-propényloxy, un 2-propinyloxy, un (alcoxy $C_{1-4}$)-2 éthoxy, un phénoxy non substitué ou mono- ou disubstitué par un halogène, un alkyl $C_{1-6}$thio, un alkyl $C_{1-4}$amino, un di(alkyl $C_{1-4}$)amino ou un groupe (a) ou (b)

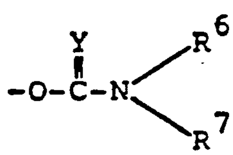

(a)

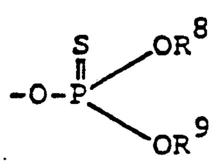

(b)

$R^3$ représente l'hydrogène, le fluor, le chlore, un alkyle en $C_1$ à $C_6$, un cycloalkyle $C_{3-6}$, un (alcoxy $C_{1-4}$) méthyle, un (alkyl $C_{1-4}$thio)méthyle, un alcoxy $C_{1-6}$, un 2-pro-pényloxy, un 2-propinyloxy, un alkyl $C_{1-6}$thio, un alkyl $C_{1-4}$amino, un di(alkyl $C_{1-4}$)amino ou un phényle non substitué, monosubstitué ou disubstitué, les substituants étant le fluor, le chlore, un trifluorométhyle et/ou un méthoxy,

$R^4$ représente un alkyle en $C_1$ à $C_3$

$R^5$ représente un alkyle en $C_1$ à $C_6$

$R^6$ et $R^7$, respectivement $R^6$ et $R^9$ représentent, indépendamment l'un de l'autre, un alkyle en $C_1$ à $C_3$

et X et Y représentent, indépendamment l'un de l'autre, l'oxygène ou le soufre, ainsi que des adjuvants de

formulation.

25. Pesticide selon la revendication 24, dans lequel $R^2$ représente l'hydrogène, le fluor, le chlore, un alkyle en $C_1$ à $C_4$ un alcoxy en $C_1$ à $C_6$, un 2-propényloxy,un 2-propinyloxy, un alkyl $C_{1-6}$thio, um di(alkyl $C_{1-4}$)amino ou un groupe (a) ou (b) et $R^3$ représente l'hydrogène, le fluor, le chlore, un alkyle en $C_1$ à $C_6$, un cycloalkyle $C_3$-$C_6$, un (alcoxy $C_{1-4}$)méthyle, un (alkyl $C_{1-4}$thio)méthyle, un alcoxy en $C_1$ à $C_6$, un 2-propényloxy, un 2-propinyloxy, un alkyl $C_{1-6}$thio, un di(alkyl $C_{1-4}$)amino, um phényle non substitué, monosubstitué ou disubstitué, les substituants étant le fluor, le chlore, un trifluorométhyle et/ou un méthoxy et $R^1$, $R^4$, $R^5$ et X ayant les significations indiquées dans la revendication 24.

26. Pesticide selon la revendication 25, caractérisé en ce qu'il contient une quantité efficace de dithiophosphate de O-éthyle et de S-(diéthoxy-2,6 pyrimidinyl-4 méthyle), S-(n-propyle), ainsi que des adjuvants de formulation.

27. Pesticide selon la revendication 25, caractérisé en ce qu'il contient une quantité efficace de dithiophosphate de O-éthyle et de S-[isopropyl-2(2-propinyloxy)-6 pyrimidinyl-4 méthyle], S-(n-propyle) ainsi que des adjuvants de formulation.

28. Procédé de préparation de composés de formule générale

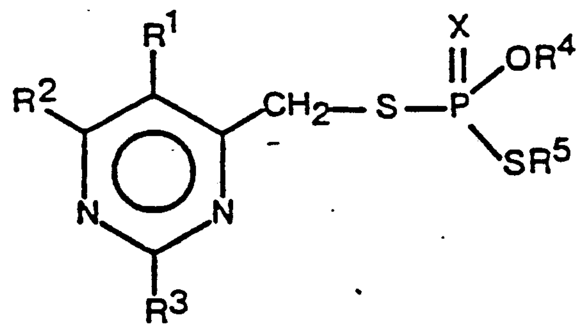

I

dans laquelle $R^1$ représente l'hydrogène, le fluor ou le chlore,

$R^2$ représente l'hydrogène, le fluor, le chlore, un alkyle en $C_1$ à $C_4$, un alkoxyen $C_1$ à $C_6$, un 2-propényloxy, un 2-propinyloxy, un (alcoxy $C_{1-4}$)-2 éthoxy, un phénoxy non substitué ou mono- ou disubstitué par un halogène, un alkyl $C_{1-6}$thio, un alkyl $C_{1-4}$amino, un di(alkyl $C_{1-4}$)amino ou un groupe (a) ou (b)

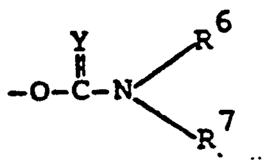

(a)

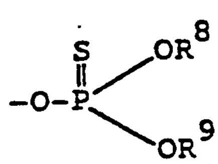

(b)

$R^3$ représente l'hydrogène, le fluor, le chlore, un alkyle en $C_1$ à $C_6$, un cycloalkyle $C_{3-6}$, un (alcoxy $C_{1-4}$) méthyle, un (alkyl $C_{1-4}$ thio)méthyle, un alcoxy $C_{1-6}$, un 2-pro-pényloxy, un 2-propinyloxy, un alkyl $C_{1-6}$thio, un alkyl $C_{1-4}$ amino, un di(alkyl $C_{1-4}$)amino ou un phényle non substitué, monosubstitué ou disubstitué, les substituants étant le fluor, le chlore, un trifluorométhyle et/ou un méthoxy,

$R^4$ représente un alkyle en $C_1$ à $C_3$

$R^5$ représente un alkyle en $C_1$ à $C_6$

$R^6$ et $R^7$, respectivement $R^8$ et $R^9$ représentent, indépendamment l'un de l'autre, un alkyle en $C_1$ à $C_3$

et X et Y représentent, indépendamment l'un de l'autre, l'oxygène ou le soufre, caractérisé en ce que

a) on fait réagir un dérivé de la pyrimidine de formule genérale

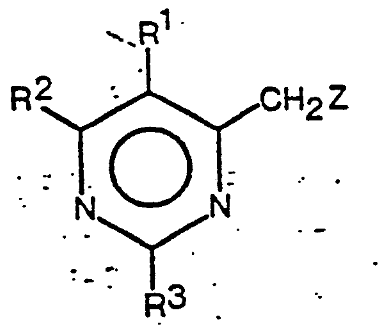

II

dans laquelle $R^1$, $R^2$ et $R^3$ ont significations précédentes et Z représente un groupe éliminable, avec un dithioou trithiophosphate de formule générale

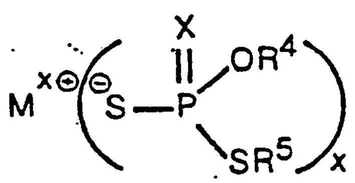

III

dans laquelle $R^4$, $R^5$ et X ont les significations précédentes, $M^x \oplus$ est un ion de métal alcalin, de métal alcalinoterreux ou d'ammonium éventuellement substitué et
x représente la valence du cation $M^x \oplus$, ou
b) on traite un dérivé de la pyrimidine de formule générale

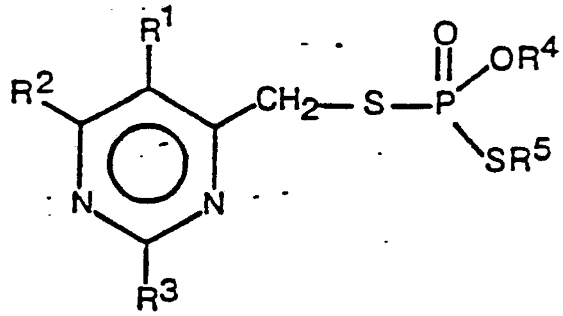

I'

dans laquelle $R^1$, $R^2$, $R^3$, $R^4$ et $R^5$ ont les significationsprécédentes,
à l'exception du dérivé I' où $R^2$ est

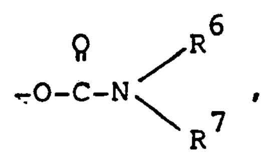

avec un agent de sulfuration.

29. Procédé delon la revendication 28, dans lequel $R^2$ représente l'hydrogène, le fluor, le chlore, un alkyle en $C_1$ à $C_4$, un alcoxy en $C_1$ à $C_6$, un 2-propényloxy, un 2-pro-pinyloxy, un alkoxy en $C_{1-6}$thio, un di(alkyl $C_{1-4}$)amino ou un groupe (a) ou (b) et $R^3$ représente l'hydrogène, le fluor, le chlore, un alkyle en $C_1$ à $C_6$, un cycloalkyle $C_3$-$C_6$, un (alcoxy $C_{1-4}$) méthyle, un (alkyl $C_{1-4}$ thio)méthyle, un alcoxy en $C_1$ à $C_6$, un 2-propényloxy, un 2-propinyloxy, un alkyl $C_{1-6}$thio, un di (alkyl $C_{1-4}$)amino, un phényle non substitué, monosubstitué ou disubstitué, les substituants étant le fluor, le chlore, un trifluorométhyle et/ou un méthoxy et $R^1$, $R^4$, $R^5$ et X ayant les sigmifications indiquées dans la revendication 28.

30. Procédé pour la lutte contre les parasites, caractérisé en ce que l'on traite le produit à protéger ou les parasites eux mêmes avec une quantité efficace d'un des composés cités dans les revendications 1, 6, 12, 14 et 22 ou d'un agent cité dans la revendication 24.

31. Procede pour la lutte contre les parasites, caractérisé en ce que l'on traite le produit à protéger ou les parasites eux-mêmes avec une quantité efficace d'un des composés cités dans les revendications 2 à 5, 7 à 11,

13 et 15 à 21 ou d'un agent cité dans la revendication 25.

32. Utilisation d'un des composés cités dans les revendications 1, 6, 12, 14 et 22 ou d'un agent cité dans la revendication 24 pour la lutte contre les parasites.

33. Utilisation d'un des composés cités dans les revendications 2 à 5, 7 à 11, 13 et 15 à 21 ou d'un agent cité dans la revendication 25 pour la lutte contre les parasites.

**Patent Claims** for the Contracting States

BE; CH, DE, FR, GE, IT, LI, LU, NL, SE

1. Compounds of the general formula

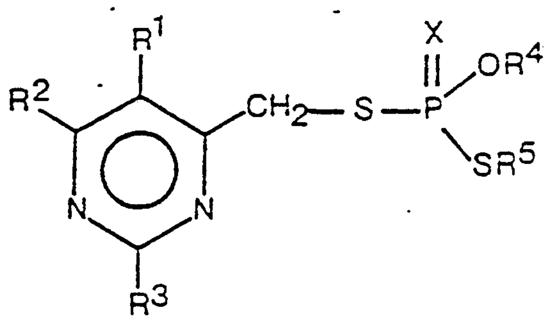

I

wherein $R^1$ signifies hydrogen, fluorine or chlorine,

$R^2$ signifies hydrogen, fluorine, chlorine $C_{1-4}$-alkoxy, 2-pro-penyloxy, 2-propynyloxy, -($C_{1-4}$-alkoxy)-ethoxy, unsubstituted phenoxy, phenoxy monosubstituted or disubstitu-, red with halogen, $C_{1-6}$-alkythio, $C_{1-4}$-alkylamino, di($C_{1-4}$-alkyl)-amino or a group (a) or (b)

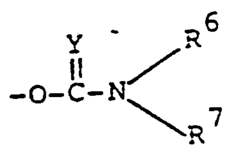

(a)

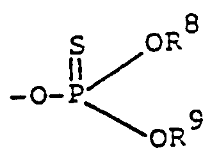

(b)

$R^3$ signifies hydrogen fluorine, chlorine, $C_{1-6}$-alkyl $C_{3-}$-cycloalkyl, ($C_{1-4}$-alkoxy)methyl, ($C_{1-4}$-alkyl-thio)methyl, $C_{1-6}$-alkoxy, 2-propenyl-oxy 2-propynyloxy $C_{1-6}$-alkylthio, $C_{1-4}$-alkylamino, di($C_{1-4}$-alkyl)-amino or unsubstituted, monosubstituted or disubstituted phenyl, the substituents being fluorine, chlorine, trifluoromethyl and/or methoxy,

$R^4$ signifies $C_{1-3}$-alkyl,

$R^5$ signifies $C_{1-6}$-alkyl,

$R^6$ and $R^7$ or $R^8$ and $R^9$ each independently signify $C_{1-3}$-alkyl

and X and Y each independently signify oxygen or sulphur.

2. Compounds according to claim 1, wherein $R^2$ signifies hydrogen, fluorine, chlorine, $C_{1-4}$-alkyl, $C_{1-6}$-alkoxy, 2-propenyloxy, 2-propynyloxy, $C_{1-6}$-alkylthio, di($C_{1-4}$-alkyl)amino or a group (a) or (b) and $R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyloxy, 2-pro-pynyloxy, $C_{1-6}$-alkylthio, di($C_{1-4}$-alkyl)amino or unsubstituted, monosubstituted or disubstituted phenyl, the substituents being fluorine, chlorine, trifluoromethyl and/or methoxy, and $R^1$, $R^4$, $R^5$ and X have the significances given in claim 1.

3. Compounds according to claim 2, wherein $R^1$ signifies hydrogen.

4. Compounds according to claim 2 or3, wherein $R^2$ signifies methoxy, ethoxy, isopropoxy or 2-propynyloxy.

5. Compounds according to any one of claims 2 to 4, wherein $R^3$ signifies hydrogen, methyl, isopropyl, tert.butyl, cyclopropyl, methoxy, ethoxy, methylthio, ethylthio, dimethylamino or diethylamino.

6. Compounds according to claim 1, wherein $R^3$ signifies isopropylamino.

7. Compounds according to any one of claims 2 to 5, wherein $R^4$ signifies ethyl.

8. Compounds according to any one of claims 2 to 5 and 7, wherein $R^5$ signifies n-propyl.

9. Compounds according to any one of claims 2 to 5, 7 and 8, wherein X signifies oxygen.

10. Compounds according to claim 2, wherein $R^2$ and/or $R^3$ signifies $C_{1-6}$-alkoxy, 2-propenyloxy or 2-propynyloxy.

11. Compounds according to claim 2, wherein $R^1$, $R^2$ and/or $R^3$ signifies chlorine.

12. Compounds according to claim 1, wherein $R^2$ signifies 2-($C_{1-4}$-alkoxy)-ethoxy or unsubstituted phenoxy or phenoxy monosubstituted or disubstituted with halogen.

13. Compounds according to claim 2 wherein $R^2$ and/or $R^3$ signifies $C_{1-6}$-alkylthio.

14. Compounds according to claim 1, wherein $R^2$ and/or $R^3$ signifies $C_{1-4}$-alkylamino or di($C_{1-4}$-alkyl)amino.

15. Compounds according to claim 2, wherein $R^2$ signifies $C_{1-4}$-alkyl and $R^3$ signifies $C_{1-6}$-alkyl, $C_{3-6}$-cycloalkyl, ($C_{1-4}$-alkoxy)methyl, $C_{1-6}$-alkoxy, di($C_{1-4}$-alkyl)amino or phenyl optionally substituted with methoxy.

16. Compounds according to claim 2, wherein $R^1$, $R^2$ and/or $R^3$ signifies hydrogen.

17. Compounds according to claim 2, wherein $R^1$, $R^2$ and/or $R^3$ signifies fluorine.

18. Compounds according to claim 2, wherein $R^2$ signifies a group (a) or (b).

19. O-Ethyl S-(2,6-diethoxy-4-pyrimidinylmethyl) S-(n-propyl) dithiophosphate.

20. O-Ethyl S-[2-isopropyl-6-(2-propynyloxy)-4-pyrimidinylmethyl] S-(n-propyl) dithiophosphate.

21. A compound according to claim 2, selected from:
O-Ethyl S-(6-ethoxy-2-methyl-4-pyrimidinylmethyl) S-(n-propyl) dithiophosphate,
O-ethyl S-(sec.butyl) S-(6-ethoxy-2-methyl-4-pyrimi-dinylmethyl) dithiophosphate,
O-ethyl S-(6-ethoxy-2-cyclopropyl-4-pyrimidinylmethyl) S-(n-propyl) dithiophosphate,
O-ethyl S-[2-(tert.butyl)-6-chloro-4-pyrimidinyl-methyl] S-(n-propyl) dithiophosphate,
O-ethyl S-(sec.butyl) S-(2,6-diethoxy-4-pyrimidinyl-methyl) dithiophosphate,
O-ethyl S-[6-ethoxy-2-(tert.butyl)-4-pyrimidinyl-methyl] S-(n-propyl) dithiophosphate,
O-ethyl S-[2-(tert.butyl)-6-methoxy-4-pyrimidinyl-methyl] S-(n-propyl) dithiopnosphate,
O-ethyl S-(2-chloro-6-dimethylamino-4-pyrimidinyl-methyl) S-(n-propyl) dithiophosphate,
O-etnyl S-(2,6-dichloro-4-pyrimidinylmethyl) S-(n-propyl) dithiopnosphate,
O-ethyl S-(2-chloro-6-methoxy-4-pyrimidinylmetnyl) S-(n-propyl) dithiophosphate,
O-ethyl S-(6-chloro-2-isopropyl-4-pyrimidinylmethyl) S-(n-propyl) dithiophosphate,
O-ethyl S-(6-ethoxy-2-isopropyl-4-pyrimidinylmethyl) S-(n-propyl) dithiophosphate,
O-ethyl S-(2,6-dimethoxy-4-pyrimidinylmethyl) S-(n-propyl) dithiophosphate,
O-ethyl S-(2-ethyl-6-chloro-4-pyrimidinylmethyl) S-(n-propyl) dithiophosphate,
O-ethyl S-(6:ethoxy-2-ethyl-4-pyrimidinylmethyl) S-(n-propyl) dithiophosphate,
O-ethyl S-(6-dimethylcarbamoyloxy-2-methyl-4-pyrimidinylmethyl) S-(n-propyl) dithiophosphate,
O-ethyl S-[6-(O,O-dimethylthiophosphoro)-2-methyl-4-pyrimidinylmethyl] S-(n-propyl) dithiophosphate,
O-ethyl S-(6-ethoxy-2-methylthio-4-pyrimidinylmethyl) S-(n-propyl) dithiophosphate,
O-ethyl S-(6-ethylthio-2-cyclopropyl-4-pyrimidinyl-methyl) S-(n-propyl) dithiophosphate,
O-ethyl S-(2,6-diisopropoxy-4-pyrimidinylmethyl) S-(n-propyl) dithiophosphate,
O-ethyl S-(6-isopropoxy-2-isopropyl-4-pyrimidinyl-methyl) S-(n-propyl) dithiophosphate,
O-ethyl S-[2-isopropyl-6-(2-propenyloxy)-4-pyrimi-dinylmethyl] S-(n-propyl) dithiophosphate,
O-ethyl S-(6-ethoxy-2-chloro-4-pyrimidinylmethyl) S-(n-propyl) dithiophosphate,
O-ethyl S-(2,6-diethylthio-4-pyrimidinylmethyl) S-(n-propyl) dithiophosphate,
O-ethyl S-[6-ethoxy-2-(p-chlorophenyl)-4-pyrimidinyl-methyl] S-(n-propyl) dithiophosphate,
O-ethyl S-(2,6-diethoxy-4-pyrimidinylmethyl) S-(n-propyl) trithiophosphate,
O-ethyl S-(2,6-dichloro-4-pyrimidinylmethyl) S-(n-propyl) trithiophosphate and
O-ethyl S-(6-ethoxy-2-methyl-4-pyrimidinylmethyl) S-(sec.butyl) trithiophosphate.

22. A compound according to claim 1, selected from:
O-Ethyl S-[2-(p-chlorophenyl)-6-(O,O-diethyl-thiophos-phoro)-4-pyrimidinylmethyl] S-(n-propyl) dithiophosphate,
O-ethyl S-ethyl S-(2,6-diethoxy-4-pyrimidinylmethyl) dithiophosphate,
O-ethyl S-(6-dimethylthiocarbamoyloxy-2-methyl-4-pyrimidinylmethyl) S-(n-propyl) dithiophosphate,
O-ethyl S-(2-methyl-6-phenoxy-4-pyrimidinylmethyl) S-(n-propyl) dithiophosphate,
O-ethyl S-(6-dimethylamino-2-methyl-4-pyrimidinyl-methyl) S-(n-propyl) dithiophosphate,
O-ethyl S-(2-ethoxy-6-dimethylcarbamoyloxy-4-pyrimi-dinylmethyl) S-(n-propyl) dithiophosphate,
O-ethyl S-(2-diethylamino-6-chloro-4-pyrimidinyl-methyl) S-(n-propyl) dithiophosphate,
O-ethyl S-(6-chloro-2-isopropylamino-4-pyrimidinyl-methyl) S-(n-propyl) dithiophosphate,
O-ethyl S-(2-chloro-6-diethylamino-4-pyrimidinyl-methyl) S-(n-propyl) dithiophosphate,
O-ethyl S-(2-chloro-6-isopropylamino-4-pyrimidinyl-methyl) S-(n-propyl) dithiophosphate,
O-ethyl S-(sec.butyl) S-[2-isopropyl-6-(2-propynyloxy)-4-pyrimidinylmethyl] dithiophosphate,
O-ethyl S-(6-ethoxy-2-methoxy-4-pyrimidinylmethyl) S-(n-propyl) dithiophosphate,
O-ethyl S-[2-methoxy-6-(2-propynyloxy)-4-pyrimidinyl-methyl] S-(n-propyl) dithiophosphate,
O-ethyl S-(6-diethylamino-2-methoxy-4-pyrimidinyl-methyl) S-(n-propyl) dithiophosphate,
O-ethyl S-(6-ethoxy-2-diethylamino-4-pyrimidinyl-methyl) S-(n-propyl) dithiophosphate,
O-ethyl S-(6-isopropoxy-2-methoxy-4-pyrimidinylmethyl) S-(n-propyl) dithiophosphate,
O-ethyl S-(6-methoxy-4-pyrimidinylmethyl) S-(n-propyl) dithiophosphate,
O-ethyl S-(6-ethoxy-4-pyrimidinylmethyl) S-(n-propyl) dithiophosphate,
O-ethyl S-[6-(2-propynyloxy)-4-pyrimidinylmethyl] S-(n-propyl) dithiophosphate,

O-ethyl S-(6-phenoxy-4-pyrimidinylmethyl) S-(n-propyl) dithiophosphate,
O-ethyl S-(2-isopropyl-6-methyl-4-pyrimidinylmethyl) S-(n-propyl) dithiophosphate,
O-ethyl S-[2-isopropyl-6-(2-methoxyethoxy)-4-pyrimi-dinylmethyl] S-(n-propyl) dithiophosphate,
O-ethyl S-(6-fluoro-2-isopropyl-4-pyrimidinylmethyl) S-(n-propyl) dithiophosphate,
O-ethyl S-(2-chloro-6-fluoro-4-pyrimidinylmethyl) S-(n-propyl) dithiophosphate,
O-ethyl S-(2-ethoxy-6-isopropoxy-4-pyrimidinylmethyl) S-(n-propyl) dithiophosphate,
S-(2,6-diethoxy-4-pyrimidinylmethyl) O-methyl S-methyl dithiophosphate,
S-(2,6-diethoxy-4-pyrimidinylmethyl) O-methyl S-(n-propyl) dithiophosphate and
O-ethyl S-(5-chloro-2,6-diethoxy-4-pyrimidinylmethyl) S-(n-propyl) dithiophosphate.

23. A compound according to claim 1 as a pest control agent.

24. A pest control composition, characterized in that it contains an effective amount of at least one compound of the general formula

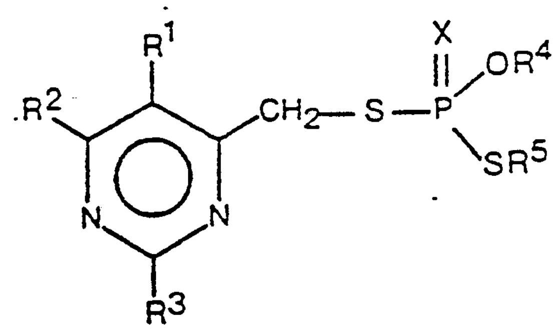

I

wherein $R^1$ signifies hydrogen, fluorine or chlorine,

$R^2$ signifies hydrogen, fluorine, chlorine, $C_{1-4}$-alkyl, $C_{1-6}$-alkoxy, 2-pro-penyloxy, 2-propynyloxy, 2-($C_{1-4}$-alkoxy)-ethoxy, unsubstituted phenoxy, phenoxy monosubstituted or disubstituted with halogen, $C_{1-6}$-alkylthio, $C_{1-4}$-alkylamino, di($C_{1-4}$-alkyl)-amino or a group (a) or (b)

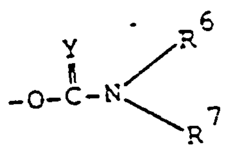

(a)

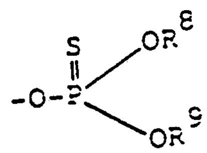

(b)

$R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyl-oxy, 2-propynyloxy, $C_{1-6}$-alkylthio, $C_{1-4}$-alkylamino, di($C_{1-4}$-alkyl)-amino or unsubstituted, monosubstituted or disubstituted phenyl, the substituents being fluorine, chlorine, trifluoromethyl and/or methoxy,

$R^4$ signifies $C_{1-3}$-alkyl,

$R^5$ signifies $C_{1-6}$-alkyl,

$R^6$ and $R^7$ or $R^8$ and $R^9$ each independently signify $C_{1-3}$-alkyl

and X and Y each independently signify oxygen or sulphur,

as well as formulation adjuvants.

25. A pest control composition according to claim 24, wherein $R^2$ signifies hydrogen, fluorine, chlorine, $C_{1-4}$-alkyl, $C_{1-6}$-alkoxy, 2-propenyloxy, 2-propynyl-oxy, $C_{1-6}$-alkylthio, di($C_{1-4}$-alkyl)amino or a group (a) or (b) and $R^3$ signifies hydrogen, fluorine, chlorine, $C_{1-6}$-alkyl, $C_{3-6}$-cycloalkyl, ($C_{1-4}$-alkoxy)-methyl, ($C_{1-4}$-alkylthio)methyl, $C_{1-6}$-alkoxy, 2-pro-penyloxy, 2-propynyloxy, $C_{1-6}$-alkylthio, di-($C_{1-4}$-alkyl)amino or unsubstituted, monosubstituted or disubstituted phenyl, the substituents being fluorine, chlorine, trifluoromethyl and/or methoxy, and $R^1$, $R^4$, $R^5$ and X have the significances given in claim 24.

26. A pest control composition according to claim 25, characterized in that it contains an effective amount of O-ethyl S-(2,6-diethoxy-4-pyrimidinylmethyl) S-(n-propyl) dithiophosphate as well as formulation adjuvants.

27. A pest control composition according to claim 25, characterized in that it contains an effective amount of O-ethyl S-[2-isopropyl-6-(2-propynyloxy)-4-pyrimidinyl-methyl] S-(n-propyl) dithiophosphate as well as formulation adjuvants.

28. A process for the manufacture of compounds of the general formula

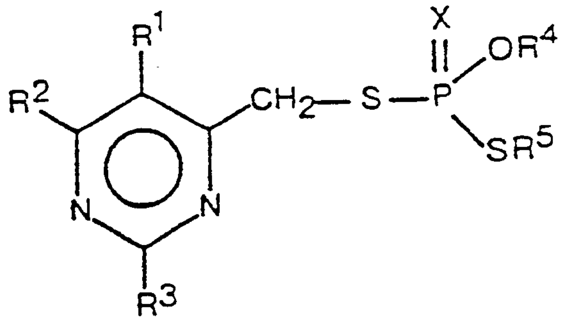

I

.

wherein $R^1$ signifies hydrogen, fluorine or chlorine,
$R^2$ signifies hydrogen, fluorine, chlorine, $C_{1-4}$-alkyl, $C_{1-6}$-alkoxy, 2-pro-penyloxy, 2-propynyloxy, 2-($C_{1-4}$-alkoxy)-ethoxy, unsubstituted phenoxy, phenoxy monosubstituted or disubstituted with halogen, $C_{1-6}$-alkylthio, $C_{1-4}$-alkylamino, di($C_{1-4}$-alkyl)-amino or a group (a) or (b)

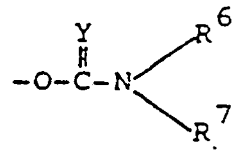

(a)

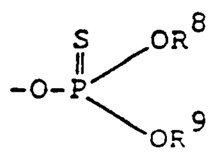

(b)

$R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyl-oxy, 2-propynyloxy, $C_{1-6}$-alkylthio, $C_{1-4}$-alkylamino, di($C_{1-4}$-alkyl)-amino or unsubstituted, monosubstituted or disubstituted phenyl, the substituents being fluorine, chlorine, trifluoromethyl and/or methoxy,
$R^4$ signifies $C_{1-3}$-alkyl,
$R^5$ signifies $C_{1-6}$-alkyl,
$R^6$ and $R^7$ or $R^8$ and $R^9$ each independently signify $C_{1-3}$-alkyl
and X and Y each independently signify oxygen or sulphur,
characterized by
a) reacting a pyrimidine derivative of the general formula

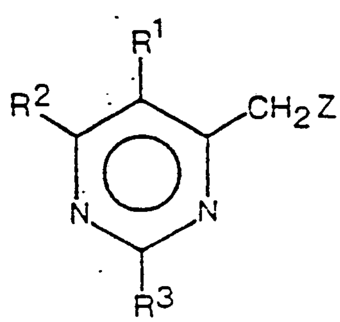

II

wherein $R^1$, $R^2$ and $R^3$ have the significances given above
and Z signifies a leaving group,
with a dithiophosphate or trithiophosphate of the general formula

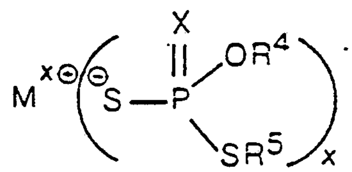

III

wherein $R^4$, $R^5$ and X have the significances given above,
$M^{x\oplus}$ stands for an alkali metal, alkaline earth metal or optionally substituted ammonium ion and x signifies the valency of the cation $M^{x\oplus}$,
or
b) treating a pyrimidine derivative of the general formula

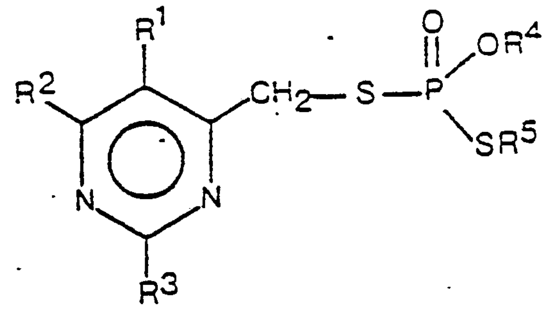

I'

wherein $R^1$, $R^2$, $R^3$, $R^4$ and $R^5$ have the significances given above,
with the exception of the derivatives I' in which $R^2$ is

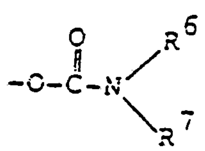

, with a sulphurizing agent.

29. A process according to claim 28, wherein $R^2$ signifies hydrogen, fluorine, chlorine, $C_{1-4}$-alkyl, $C_{1-6}$-alkoxy, 2-propenyloxy, 2-propynyloxy, $C_{1-6}$-alkylthio, di($C_{1-4}$-alkyl)amino or a group (a) or (b) and $R^3$ signifies hydrogen, fluorine, chlocine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyloxy, 2-pro-pynyloxy, $C_{1-6}$-alkylthio, di($C_{1-4}$-alkyl)amino or unsubstituted, monosubstituted or disubstituted phenyl, the substituents being fluorine, chlorine, trifluoromethyl and/or methoxy, and $R^1$, $R^4$, $R^5$ and X have the significances given in claim 28.

30. A method for the control of pests, characterized by treating the locus to be protected or the pests themselves with an effective amount of one of the compounds set forth in claims 1, 6, 12, 14 and 22 or of a composition set forth in claim 24.

31. A method for the control of pests, characterized by treating the locus to be protected or the pests themselves with an effective amount of one of the compounds set forth in claims 2 to 5, 7 to 11, 13 and 15 to 21 or of a composition set forth in claim 25.

32. The use of one of the compounds set forth in claims 1, 6, 12, 14 and 22 or of a composition set forth in claim 24 for the control of pests.

33. The use of one of the compounds set forth in claims 2 to 5, 7 to 11, 13 and 15 to 21 or of a composition set forth in claim 25 for the control of pests.

**Revendications** pour l'Etat Contractant AT

1. Pesticide caractérisé en ce qu'il contient une quantité efficace d'au moins un composé de formule générale

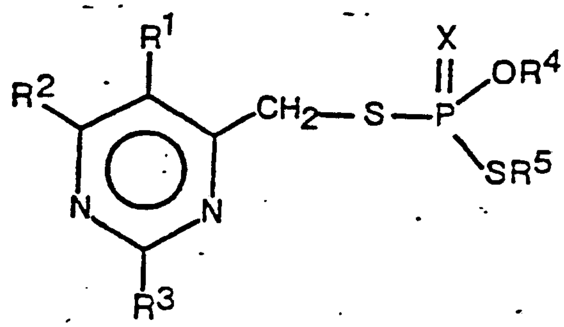

I

dans laquelle $R^1$ représente l'hydrogène, le fluor ou le chlore, $R^2$ représente l'hydrogène, le fluor, le chlore, un alkyle en $C_1$ à $C_4$, un alcoxy en $C_1$ à $C_6$, un 2-propényloxy, un 2-propinyloxy un (alcoxy $C_{1-4}$)-2 ethoxy, un phénoxy non subsitué ou mono- ou disubstitue par un halogène un alkyl $C_{1-6}$thio, un alkyl $C_{1-4}$amino, un di(alkyl $C_{1-4}$)amino ou un groupe (a) ou (b)

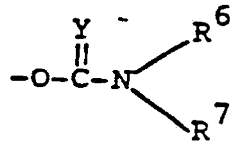

(a)

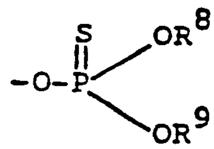

(b)

$R^3$ représente l'hydrogène, le fluor, le chlore, un alkyle en $C_1$ à $C_6$ un cycloalkyle $C_{3-6}$ un (alcoxy $C_{1-4}$) méthyle un (alkyl $C_{1-4}$thio)méthyle un alcoxy $C_{1-6}$, un 2-pr-opényloxy, un 2-propinyloxy, un alkyl $C_{1-6}$thio, un alkyl $C_{1-4}$amino, un di(alkyl $C_{1-4}$)amino ou un phényle non substitué, monosubstitué ou disubstitué, les substituants étant le fluor, le chlore, un trifluorométhyle et/ou un méthoxy,

$R^4$ représente un alkyle en $C_1$ à $C_3$

$R^5$ représente un alkyle en $C_1$ à $C_6$

$R^6$ et $R^7$, respectivement $R^8$ et $R^9$ représentent, indépendamment l'un de l'autre, un alkyle en $C_1$ à $C_3$ et X et Y représentent, indépendamment l'un de l'autre, l,oxygène ou le soufre, ainsi que des adjuvants de formulation.

2. Pesticide selon la revendication 1, dans lequel $R^2$ représente l'hydrogène, le fluor, le chlore, un alkyle en $C_1$ à $C_4$, un alcoxy en $C_1$ à $C_6$, un 2-propényloxy, un 2-propiny-loxy, un alkyl $C_{1-6}$thio, un di(alkyl $C_{1-4}$)amino ou un groupe (a) ou (b) et $R^3$ représente l'hydrogène, le fluor, le chlore, un alkyle en $C_1$ à $C_6$, un cycloalkyle $C_3$-$C_6$, un (alcoxy $C_{1-4}$) méthyle, un (alkyl $C_{1-4}$thio)méthyle, un alcoxy en $C_1$ à $C_6$, un 2-propényloxy, un 2-propinyloxy, un alkyl $C_{1-6}$thio, un di (alkyl $C_{1-4}$)amino, un phényle non substitué, monosubstitué ou disubstitué, les substituants étant le fluor, le chlore, un trifluorométhyle et/ou un méthoxy et $R^1$, $R^4$, $R^5$ et X ayant les significations indiquées dans la revendication 1.

3. Pesticide selon la revendication 2, dans lequel $R^1$ représente l'hydrogène.

4. Pesticide selon la revendication 2 ou 3, dans lequel $R^2$ représente un méthoxy, un éthoxy, un isopropoxy ou un 2-propinyloxy.

5. Pesticide selon l'une des revendications 2 à 4, dans lequel $R^3$ représente l'hydrogène, un méthyle, un isopropyle, un tert.butyle, un cyclopropyle, un méthoxy, un éthoxy, un méthylthio, un éthylthio, un dimèthylamino ou un diéthylamino.

6. Pesticide selon la revendication 1, dans lequel $R^3$ représente un isopropylamino.

7. Pesticide selon l'une des revendications 2 à 5, dans lequel $R^4$ représente un éthyle.

8. Pesticide selon l'une des revendications 2 à 5 et 7, dans lequel $R^5$ représente un n-propyle.

9. Pesticide selon l'une des revendications 2 à 5, 7 et 6, dans lequel X représente l'oxygène.

10. Pesticide selon la revendication 2, dans lequel $R^2$ et/ou $R^3$ représentent un alcoxy en $C_1$ à $C_6$, un 2-propényloxy ou un 2-propinyloxy.

11.Pesticide selon la revendication 2, dans lequel $R^1$, $R^2$ et/ou $R^3$ représentent le chlore.

12. Pesticide selon la revendication 1, dans lequel $R^2$ représente un (alcoxy $C_{1\ 4}$)-2 éthoxy ou un phénoxy non substitué ou mono- ou disubstitué par un halogène.

13. Pesticide selon la revendication 2, dans lequel $R^2$ et/ou $R^3$ représentent un alkyl $C_{1-6}$thio.

14. Pesticide selon la revendication 1, dans lequel $R^2$ et/ou $R^3$ représentent un alkyl $C_{1-4}$amino ou un di(alkyl

$C_{1-4}$) amino.

15. Pesticide selon la revendication 2, dans lequel $R^2$ représente un alkyle en $C_1$ à $C_4$ et $R^3$ représente un alkyle en $C_1$ à $C_6$, un cycloalkyle $C_{3\ 6}$, un (alcoxy $C_{1-4}$)méthyle, un alcoxy en $C_1$ à $C_6$, un di(alkyl $C_{1-4}$)amino ou un phényle éventuellement substitué par un méthoxy.

16. Pesticide selon la revendication 2, dans lequel $R^1$, $R^2$ et/ou $R^3$ représentent l'hydrogène.

17. Pesticide selon la revendication 2, dans lequel $R^1$, $R^2$ et/ou $R^3$ représentent le fluor.

18. Pesticide selon la revendication 2, dans lequel $R^2$ représente un groupe (a) ou (b).

19. Pesticide selon la revendication 2, caractérisé en ce qu'il contient une quantité efficace de dithiophosphate de O-éthyle et de S-(diéthoxy-2,6 pyrimidinyl-4 méthyle), S(n-propyle) ainsi que des adjuvants de formulation.

20. Pesticide selon la revendication 2, caractérisé en ce qu'il contient une quantité efficace de dithiophosphate de O-éthyle et de S-[isopropyl-2 (2-propinyloxy)-6 pyrimidinyl-4 méthyle], S-(n-propyle) ainsi que des adjuvants de formulation.

21. Pesticide selon la revendication 2, caractérisé en ce qu'il contient une quantité efficace d'au moins un composé choisi parmi:
le dithiophosphate de O-éthyle et de S-(éthoxy-6 mé-thyl-2 pyrimidinyl-4 méthyle), S-(n-propyle),
le dithiophosphate de O-éthyle et de S-(sec.butyle), S-(éthoxy-6 méthyl-2 pyrimidinyl-4 méthyle), le dithiophosphate de O-éthyle et de S-(éthoxy-6 cyclopropyl-2 pyrimidinyl-4 méthyle), S-(n-propyle),
le dithiophosphate de O-éthyle et de S-[(tert.bu-tyl)-2 chloro-6 pyrimidinyl-4 méthyle], S-(n-propyle),
le dithiophosphate de O-éthyle et de S-(sec.butyle), S-(diéthoxy-2,6 pyrimidinyl-4 méthyle),
le dithiophosphate de O-éthyle et de S-[éthoxy-6 (tert.butyl)-2 pyrimidinyl-4 méthyle], S-(n-propyle),
le dithiophosphate de O-éthyle et de S-[(tert.bu-tyl)-2 méthoxy-6 pyrimidinyl-4 methyle], S-(n-propyle),
le dithiophosphate de O-éthyle et de S-(chloro-2 diméthylamino-6 pyrimidinyl-4 méthyle), S-(n-propyle),
le dithiophosphate de O-éthyle et de S-(dichloro-2,6 pyrimidinyl-4 méthyle), S-(n-propyle),
le dithiophosphate de O-éthyle et de S-(chloro-2 méthoxy-6 pyrimidinyl-4 méthyle), S-(n-propyle),
le dithiophosphate de O-éthyle et de S-(chloro-6 isopropyl-2 pyrimidinyl-4 méthyle), S-(n-propyle),
le dithiophosphate de O-éthyle et de S-(éthoxy-6 isopropyl-2 pyrimidinyl-4 méthyle), S-(n-propyle),
le dithiophosphate de O-éthyle et de S-(dimétho-xy-2,6 pyrimidinyl-4 méthyle), S-(n-propyle),
le dithiophosphate de O-éthyle et de S-(éthyl-2 chloro-6 pyrimidinyl-4 méthyle), S-(n-propyle),
le dithiophosphate de O-éthyle et de S-(éthoxy-6 éthyl-2 pyrimidinyl-4 méthyle), S-(n-propyle),
le dithiophosphate de O-éthyle et de S-(diméthyl-carbamoyloxy-6 méthyle-2 pyrimidinyl-4 méthyle), S-(n-propyle),
le dithiophosphate de O-éthyle et de S-[(O,O-di-méthylthiophosphoro)-6 méthyle-2 pyrimidinyl-4 méthyl], S(n-propyle),
le dithiophosphate de O-éthyle et de S-(éthoxy-6 méthylthio-2 pyrimidinyl-4 méthyle), S-(n-propyle),
le dithiophosphate de O-éthyle et de S-(éthylthio-6 cyclopropyl-2 pyrimidinyl-4 méthyle), S-(n-propyle),
le dithiophosphate de O-éthyle et de S-(diisopro-poxy-2,6 pyrimidinyl-4 méthyle), S-(n-propyle), le dithiophosphate de O-éthyle et de S-(isopropo-xy-6 isopropyl-2 pyrimidinyl-4 méthyle), S-(n-propyle),
le dithiophosphate de O-éthyle et de S-[isopropyl-2(2-propényloxy)-6 pyrimidinyl-4 méthyle], S-(n-propyle),
· le dithiophosphate de O-éthyle et de S-(éthoxy-6 chloro-2 pyrimidinyl-4 méthyle), S-(n-propyle),
le dithiophosphate de O-éthyle et de S-(diéthyl-thio-2,6 pyrimidinyl-4 méthyle), S-(n-propyle),
le dithiophosphate de O-éthyle et de S-[éthoxy-6 (p-chlorophényl)-2 pyrimidinyl-4 méthyle], S-(n-propyle),
le trithiophosphate de O-éthyle et de S-(diétho-xy-2,6 pyrimidinyl-4 méthyle), S-(n-propyle),
le trithiophosphate de O-éthyle et de S-(dichloro-2,6 pyrimidinyl-4 méthyle), S-(n-propyle) et
le trithiophosphate de O-éthyle et de S-(éthoxy-6 méthyl-2 pyrimidinyl-4 méthyle), S-(sec.butyle), ainsi que des adjuvants de formulation.

22. Pesticide selon la revendication 1, caractérisé en ce qu'il contient une quantité efficace d'au moins un composé choisi parmi:
le dithiophosphate de O-éthyle et de S-[(p-chlo-rophenyl)-2 (O,O-diéthylthiophosphoro)-6 pyrimidinyl-4 methyle], S-(n-propyle),
le dithiophosphate de O-éthyle et de S-éthyle, S-(diéthoxy-2,6 pyrimidinyl-4 méthyle),
le dithiophosphate de O-éthyle et de S-(diméthyl-thiocarbamoyloxy-6 méthyl-2 pyrimidinyl-4 méthyle), S-(n-propyle),
le dithiophosphate de O-éthyle et de S-(méthyl-2 phénoxy-6 pyrimidinyl-4 méthyle), S-(n-propyle),
le dithiophosphate de O-éthyle et de S-(diméthyl-amino-6 méthyl-2 pyrimidinyl-4 méthyle), S-(n-propyle),
le dithiophosphate de O-éthyle et de S-(éthoxy-2 diméthylcarbamoyloxy-6 pyrimidinyl-4 méthyle), S-(n-propyle),
le dithiophosphate de O-éthyle et de S-(diéthyl-amino-2 chloro-6 pyrimidinyl-4 méthyle), S-(n-propyle),
le dithiophosphate de O-éthyle et de S-(chloro-6 isopropylamino-2 pyrimidinyl-4 méthyle), S-(n-propyle), le dithiophosphate de O-éthyle et de S-(chloro-2 diéthylamino-6 pyrimidinyl-4 méthyle), S-(n-propyle),
le dithiophosphate de O-éthyle et de S-(chloro-2 isopropylamino-6 pyrimidinyl-4 méthyle), S-(n-propyle),
le dithiophosphate de O-éthyle et de S-(sec.butyle), S-[isopropyl-2 (2-propinyloxy)-6 pyrimidinyl-4 méthyle],
le dithiophosphate de O-éthyle et de S-(éthoxy-6 méthoxy-2 pyrimidinyl-4 méthyle), S-(n-propyle),
le dithiophosphate de O-éthyle et de S-[méthoxy-2 (2-propinyloxy)-6 pyrimidinyl-4 méthyle], S-(n-propyle),
le dithiophosphate de O-éthyle et de S-(diéthyl-amino-6 méthoxy-2 pyrimidinyl-4 méthyle), S-(n-propyle),

le dithiophosphate de O-éthyle et de S-(éthoxy-6 diéthylamino-2 pyrimidinyl-4 méthyle), S-(n-propyle),
le dithiophosphate de O-éthyle et de S-(isopro-poxy-6 méthoxy-2 pyrimidinyl-4 méthyle), S-(n-propyle),
le dithiophosphate de O-éthyle et de S-(métho-xy-6 pyrimidinyl-4 méthyle), S-(n-propyle),
le dithiophosphate de O-éthyle et de S-(éthoxy-6 pyrimidinyl-4 méthyle), S-(n-propyle),
le dithiophosphate de O-éthyle et de S-[(2-pro-pinyloxy)-6 pyrimidinyl-4 méthyle], S-(n-propyle),
le dithiophosphate de O-éthyle et de S-(phéno-xy-6 pyrimidinyl-4 méthyle), S-(n-propyle),
le dithiophosphate de O-éthyle et de S-(isopro-pyl-2 méthyl-6 pyrimidinyl-4 méthyle), S-(n-propyle),
le dithiophosphate de O-éthyle et de S-[isopro-pyl-2 (méthoxy-2 éthoxy)-6 pyrimidinyl-4 méthyle], S-(n-propyle),
le dithiophosphate de O-éthyle et de S-(fluoro-6 isopropyl-2 pyrimidinyl-4 méthyle), S-(n-propyle),
le dithiophosphate de O-éthyle et de S-(chloro-2 fluoro-6 pyrimidinyl-4 méthyle), S-(n-propyle),
le dithiophosphate de O-éthyle et de S-(éthoxy-2 isopropoxy-6 pyrimidinyl-4 méthyle), S-(n-propyle),
le dithiophosphate de S-(diéthoxy-2,6 pyrimidi-nyl-4 méthyle) et de O-méthyle, S-méthyle,
le dithiophosphate de S-(diéthoxy-2,6 pyrimidi-nyl-4 méthyle) et de O-méthyle, S-(n-propyle) et
le dithiophosphate de O-éthyle et de S-(chloro-5 diéthoxy-2,6 pyrimidinyl-4 méthyle), S-(n-propyle), ainsi que des adjuvants de formulation.

23. Procédé de préparation de composés de formule générale

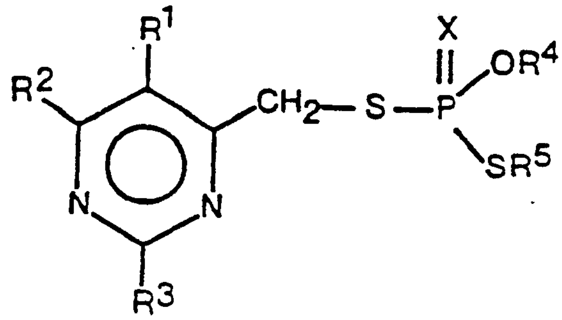

I

dans laquelle $R^1$ représente l'hydrogène, le fluor ou le chlore,

$R^2$ représente l'hydrogène, le fluor, le chlore, un alkyle en $C_1$ à $C_4$ un alcoxy en $C_1$ à $C_6$, un 2-propényloxy, un 2-propinyloxy, un (alcoxy $C_{1-4}$)-2 éthoxy, un phénoxy non substitué ou mono- ou disubstitué par un halogène, un alkyl $C_{1-6}$thio, un alkyl $C_{1-4}$amino, un di(alkyl $C_{1-4}$)amino ou un groupe (a) ou (b)

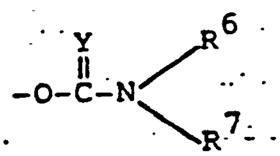

(a)

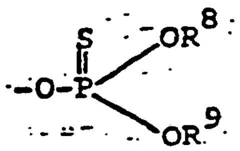

(b)

$R^3$ représente l'hydrogène, le fluor, le chlore, un alkyle en $C_1$ à $C_6$, un cycloalkyle $C_{3-6}$, un (alcoxy $C_{1-4}$) méthyle, un(alkyl $C_{1-4}$thio)méthyle, un alcoxy $C_{1-6}$, un 2-propényloxy, un 2-propinyloxy, un alkyl $C_{1-6}$thio, un alkyl $C_{1-4}$amino, un di(alkyl $C_{1-4}$)amino ou un phényle non substitué, monosubstitué ou disubstitué, les substituants étant le fluor, le chlore, un trifluorométhyle et/ou un méthoxy,

$R^4$ représente un alkyle en $C_1$ à $C_3$,

$R^5$ représente un alkyle en $C_1$ à $C_6$

$R^6$ et $R^7$, respectivement $R^8$ et $R^9$ représentent, indépendamment l'un de l'autre, un alkyle en $C_1$ à $C_3$

et X et Y représentent, indépendamment l'un de l'autre, l'oxygène ou le soufre, caractérisé en ce que

a) on fait réagir un dérivé de la pyrimidine de formule générale

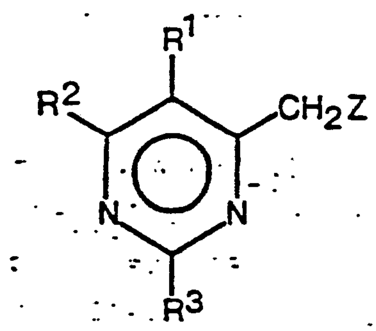

II

dans laquelle $R^1$, $R^2$ et $R^3$ ont les significations précédentes et Z représente un groupe éliminable avec un dithio- ou trithiophosphate de formule générale

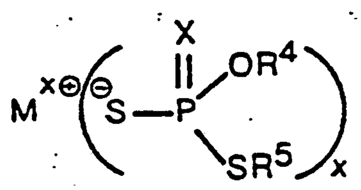

III

dans laquelle $R^4$, $R^5$ et X ont les significations précédentes,
$M^{x\oplus}$ est un ion de métal alcalin, de métal alcalino-terreux ou d'ammonium éventuellement substitué et
x représente la valence du cation $M^{x\oplus}$, ou
b) on traite un dérivé de la pyrimidine de formule générale

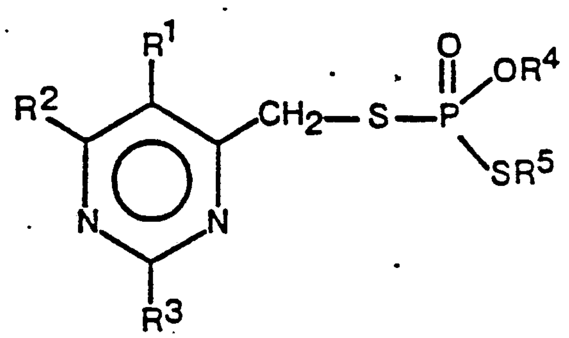

I'

dans laquelle $R^1$, $R^2$, $R^3$, $R^4$ et $R^5$ ont les significations précédentes
à l'exception du dérivé I' où $R^2$ est

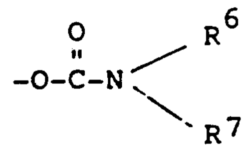

,

avec un agent de sulfuration.

24. Procédé selon la revendication 23 dans lequel $R^2$ représente l'hydrogène le fluor, le chlore, un alkyle en $C_1$ à $C_4$, un alcoxy en $C_1$ à $C_6$, un 2-propényloxy, un 2-propi-nyloxy, un alkyl $C_{1\text{-}6}$thio, un di(alkyl $C_{1\text{-}4}$)amino ou un groupe (a) ou (b) et R représente l'hydrogène, le fluor, le chlore, un alkyle en $C_1$ à $C_6$, un cycloalkyle $C_3$-$C_6$, un (alcoxy $C_{1\text{-}4}$) méthyle, un (alkyl $C_{1\text{-}4}$ thio)méthyle, un alcoxy en $C_1$ à $C_6$, un 2-propényloxy, un 2-propinyloxy, un alkyl $C_{1\text{-}6}$thio, un di (alkyl $C_{1\text{-}4}$)amino, un phényle non substitué, monosubstitué ou disubstitué, les substituants étant le fluor, le chlore, un trifluorométhyle et/ou un méthoxy et $R^1$, $R^4$, $R^5$ et X ayant les significations indiquées dans la revendication 23.

25. Procédé de préparation d'un pesticide, caractérisé en ce qu'on mélange au moins un des composés cites dans les revendications 1 à 22 avec des agents de formulation.

26. Procédé pour la lutte contre les parasites, caractérisé en ce que l'on traite le produit à protéger ou les

parasites eux-mêmes à l'aide d'une quantité efficace d'un des composés ou des pesticides mentionnés dans les revéndications 1 à 22.

27. Utilisation d'un des composés ou des agents mentionnés dans les revendications 1 à 22 pour la lutte contre les parasites.

**Claims** for the Contracting State AT

1. A pest control composition, characterized in that it contains an effective amount of at least one compound of the general formula

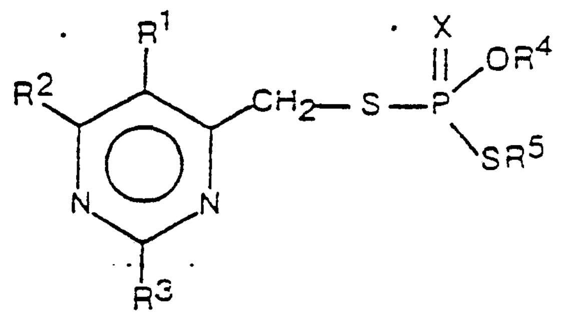

I

wherein $R^1$ signifies hydrogen, fluorine or chlorine,

$R^2$ signifies hydrogen, fluorine, chlorine, $C_{1-4}$-alkyl, $C_{1-6}$-alkoxy, 2-pro-penyloxy, 2-propynyloxy, 2-($C_{1-4}$-alkoxy)-ethoxy, unsubstituted phenoxy, phenoxy monosubstituted or disubstituted with halogen, $C_{1-6}$-alkylthio, $C_{1-4}$-alkylamino, di($C_{1-4}$-alkyl)-amino or a group (a) or (b)

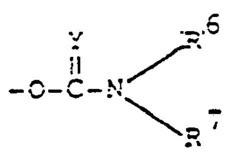

(a)

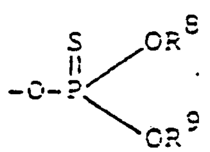

(b)

$R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyl-oxy, 2-propynyloxy, $C_{1-6}$-alkylthio, $C_{1-4}$-alkylamino, di($C_{1-4}$-alkyl)-amino or unsubstituted, monosubstituted or disubstituted phenyl, the substituents being fluorine, chlorine, trifluoromethyl and/or methoxy,

$R^4$ signifies $C_{1-3}$-alkyl,

$R^5$ signifies $C_{1-6}$-alkyl,

$R^6$ and $R^7$ or $R^8$ and $R^9$ each independently signify $C_{1-3}$-alkyl

and X and Y each independently signify oxygen or sulphur as well as formulation adjuvants.

2. A pest control composition according to claim 1, wherein $R^2$ signifies hydrogen, fluorine, chlorine, $C_{1-4}$-alkyl, $C_{1-6}$-alkoxy, 2-propenyloxy, 2-propynyl-oxy, $C_{1-6}$-alkylthio, di($C_{1-4}$-alkyl)amino or a group (a) or (b) and $R^3$ signifies hydrogen, fluorine, cnlorine, $C_{1-6}$-alkyl, $C_{3-6}$-cycloalkyl, ($C_{1-4}$-alkoxy)-methyl, ($C_{1-4}$-alkylthio)methyl, $C_{1-6}$-alkoxy, 2-pro-penyloxy, 2-propynyloxy, $C_{1-6}$-alkylthio, di($C_{1-4}$-alkyl)amino or unsubstituted, monosubstituted or disubstituted phenyl, the substituents being fluorine, chlorine, trifluoromethyl and/or methoxy, and $R^1$, $R^4$, $R^5$ and X have the significances given in claim 1.

3. A pest control composition according to claim 2, wherein $R^1$ signifies hydrogen.

4. A pest control composition according to claim 2 or 3, wherein $R^2$ signifies methoxy, ethoxy, isopropoxy or 2-propynyloxy.

5. A pest control composition according to any one of claims 2 to 4, wherein $R^3$ signifies hydrogen, methyl, isopropyl, tert.butyl, cyclopropyl, methoxy, ethoxy, methylthio, ethylthio, dimethylamino or diethylamino.

6. A pest control composition according to claim 1, wherein $R^3$ signifies isopropylamino.

7. A pest control composition according to any one of claims 2 to 5, wherein $R^4$ signifies ethyl.

8. A pest control composition according to any one of claims 2 to 5 and 7, wherein $R^5$ signifies n-propyl.

9. A pest control composition according to any one of claims 2 to 5, 7 and 8, wherein X signifies oxygen.

10. A pest control composition according to claim 2, wherein $R^2$ and/or $R^3$ signifies $C_{1-6}$-alkoxy, 2-propenyloxy or 2-propynyloxy.

11. A pest control composition according to claim 2, wherein $R^1$, $R^2$ and/or $R^3$ signifies chlorine.

12. A pest control composition according to claim 1, wherein $R^2$ signifies 2-($C_{1-4}$-alkoxy)-ethoxy or unsubstituted phenoxy or phenoxy monosubstituted or disubstituted with halogen.

13. A pest control composition according to claim 2, wherein $R^2$ and/or $R^3$ signifies $C_{1-6}$-alkylthio.

14. A pest control composition according to claim 1, wherein $R^2$ and/or $R^3$ signifies $C_{1-4}$-alkylamino or di($C_{1-4}$-alkyl)amino.

15. A pest control composition according to claim 2, wherein $R^2$ signifies $C_{1-4}$-alkyl and $R^3$ signifies $C_{1-6}$-alkyl, $C_{3-6}$-cycloalkyl, ($C_{1-4}$-alkoxy)methyl, $C_{1-6}$-alkoxy, di($C_{1-4}$-alkyl)amino or phenyl optionally substituted with methoxy.

16. A pest control composition according to claim 2, wherein $R^1$, $R^2$ and/or $R^3$ signifies hydrogen.

17. A pest control composition according to claim 2, wherein $R^1$, $R^2$ and/or $R^3$ signifies fluorine.

18. A pest control composition according to claim 2, wherein $R^2$ signifies a group (a) or (b).

19. A pest control composition according to claim 2, characterized in that it contains an effective amount of O-ethyl S-(2,6-diethoxy-4-pyrimidinylmethyl) S-(n-propyl) dithiophosphate as well as formulation adjuvants.

20. A pest control composition according to claim 2, characterized in that it contains an effective amount of O-ethyl S-[2-isopropyl-6-(2-propynyloxy)-4-pyrimidinyl-methyl] S-(n-propyl) dithiophosphate as well as formulation adjuvants.

21. A pest control composition according to claim 2, characterized in that it contains an effective amount of at least one compound selected from:

O-Ethyl S-(6-ethoxy-2-methyl-4-pyrimidinylmethyl) S-(n-propyl) dithiophosphate,
O-ethyl S-(sec.butyl) S-(6-ethoxy-2-methyl-4-pyrimi-dinylmethyl) dithiophosphate,
O-ethyl S-(6-ethoxy-2-cyclopropyl-4-pyrimidinylmethyl) S-(n-propyl) dithiophosphate,
O-ethyl S-[2-(tert.butyl)-6-chloro-4-pyrimidinyl-methyl] S-(n-propyl(dithiophosphate, O-ethyl S-(sec.butyl) S-(2,6-diethoxy-4-pyrimidinyl-methyl) dithiophosphate,
O-ethyl S-[6-ethoxy-2-(tert.butyl)-4-pyrimidinyl-methyl] S-(n-propyl) dithiophosphate,
O-ethyl S-[2-(tert.butyl)-6-methoxy-4-pyrimidinyl-methyl] S-(n-propyl) dithiophosphate,
O-ethyl S-(2-chloro-6-dimethylamino-4-pyrimidinyl-methyl) S-(n-propyl) dithiophosphate,
O-ethyl S-(2,6-dichloro-4-pyrimidinylmethyl) S-(n-propyl) dithiophosphate,
O-ethyl S-(2-chloro-6-methoxy-4-pyrimidinylmethyl) S-(n-propyl) dithiophosphate,
O-ethyl S-(6-chloro-2-isopropyl-4-pyrimidinylmethyl) S-(n-propyl) dithiophosphate,
O-ethyl S-(6-ethoxy-2-isopropyl-4-pyrimidinylmethyl) S-(n-propyl) dithiophosphate,
O-ethyl S-(2,6-dimethoxy-4-pyrimidinylmethyl) S-(n-propyl) dithiophosphate,
O-ethyl S-(2-ethyl-6-chloro-4-pyrimidinylmethyl) S-(n-propyl) dithiophosphate,
O-ethyl S-(6-ethoxy-2-ethyl-4-pyrimidinylmethyl) S-(n-propyl) dithiophosphate,
O-ethyl S-(6-dimethylcarbamoyloxy-2-methyl-4-pyrimi-dinylmethyl) S-(n-propyl) dithiophosphate,
O-ethyl S-[6-(O,O-dimethyl-thiophosphoro)-2-methyl-4-pyrimidinylmethyl] S-(n-propyl) dithiophosphate,
O-ethyl S-(6-ethoxy-2-methylthio-4-pyrimidinylmethyl) S-(n-propyl) dithiophosphate,
O-ethyl S-(6-ethylthio-2-cyclopropyl-4-pyrimidinyl-methyl) S-(n-propyl) dithiophosphate
O-ethyl S-(2,6-diisopropoxy-4-pyrimidinylmethyl) S-(n-propyl) dithiophosphate,
O-ethyl S-(6-isopropoxy-2-isopropyl-4-pyrimidinyl-methyl) S-(n-propyl) dithiophosphate,
O-ethyl S-[2-isopropyl-6-(2-propenyloxy)-4-pyrimi-dinylmethyl] S-(n-propyl) dithiophosphate,
O-ethyl S-(6-ethoxy-2-chloro-4-pyrimidinylmethyl) S-(n-propyl) dithiophosphate,
O-ethyl S-(2,6-diethylthio-4-pyrimidinylmethyl) S-(n-propyl) dithiophosphate,
O-ethyl S-[6-ethoxy-2-(p-chlorophenyl)-4-pyrimidinyl-methyl] S-(n-propyl) dithiophosphate
O-ethyl S-(2,6-diethoxy-4-pyrimidinylmethyl) S-(n-propyl) trithiophosphate,
O-ethyl S-(2,6-dichloro-4-pyrimidinylmethyl) S-(n-propyl) trithiophosphate and
O-ethyl S-(6-ethoxy-2-methyl-4-pyrimidinylmethyl) S-(sec.butyl) trithiophosphate,
as well as formulation adjuvants.

22. A pest control composition according to claim 1, characterized in that it contains an effective amount of at least one compound selected from:

O-Ethyl S-[2-(p-chlorophenyl)-6-(O,O-diethyl-thiophos-phoro)-4-pyrimidinylmethyl] S-(n-propyl) dithiophosphate,
O-ethyl S-ethyl S-(2,6-diethoxy-4-pyrimidinylmethyl) dithiophosphate,
O-ethyl S-(6-dimethylthiocarbamoyloxy-2-methyl-4-pyrimidinylmethyl) S-(n-propyl) dithiophosphate,
O-ethyl S-(2-methyl-6-phenoxy-4-pyrimidinylmethyl) S-(n-propyl) dithiophosphate,
O-ethyl S-(6-dimethylamino-2-methyl-4-pyrimidinyl-methyl) S-(n-propyl) dithiophosphate,
O-ethyl S-(2-ethoxy-6-dimethylcarbamoyloxy-4-pyrimi-dinylmethyl) S-(n-propyl) dithiophosphate,
O-ethyl S-(2-diethylamino-6-chloro-4-pyrimidinyl-methyl) S-(n-propyl) dithiophosphate,
O-ethyl S-(6-chloro-2-isopropylamino-4-pyrimidinyl-methyl) S-(n-propyl) dithiophosphate,
O-ethyl S-(2-chloro-6-diethylamino-4-pyrimidinyl-methyl) S-(n-propyl) dithiophosphate, O-ethyl S-(2-chloro-6-isopropylamino-4-pyrimidinyl-methyl) S-(n-propyl) dithiophosphate,
O-ethyl S-(sec.butyl) S-[2-isopropyl-6-(2-propynyl-oxy)-4-pyrimidinylmethyl] dithiophosphate,

O-ethyl S-(6-ethoxy-2-methoxy-4-pyrimidinylmethyl) S-(n-propyl) dithiophosphate,
O-ethyl S-[2-methoxy-6-(2-propynyloxy)-4-pyrimidinyl-methyl] S-(n-propyl) dithiophosphate,
O-ethyl S-(6-diethylamino-2-methoxy-4-pyrimidinyl-methyl) S-(n-propyl) dithiophosphate,
O-ethyl S-(6-ethoxy-2-diethylamino-4-pyrimidinyl-methyl) S-(n-propyl) dithiophosphate,
O-ethyl S-(6-isopropoxy-2-methoxy-4-pyrimidinylmethyl) S-(n-propyl) dithiophosphate,
O-ethyl S-(6-methoxy-4-pyrimidinylmethyl) S-(n-propyl) dithiophosphate,
O-ethyl S-(6-ethoxy-4-pyrimidinylmethyl) S-(n-propyl) dithiophosphate,
O-ethyl S-[6-(2-propynyloxy)-4-pyrimidinylmethyl] S-(n-propyl) dithiophosphate,
O-ethyl S-(6-phenoxy-4-pyrimidinylmethyl) S-(n-propyl) dithiophosphate,
O-ethyl S-(2-isopropyl-6-methyl-4-pyrimidinylmethyl) S-(n-propyl) dithiophosphate,
O-ethyl S-[2-isopropyl-6-(2-methoxyethoxy)-4-pyrimi-dinylmethyl] S-(n-propyl) dithiophosphate,
O-ethyl S-(6-fluoro-2-isopropyl-4-pyrimidinylmethyl) S-(n-propyl) dithiophosphate,
O-ethyl S-(2-chloro-6-fluoro-4-pyrimidinylmethyl) S-(n-propyl) dithiophosphate,
O-ethyl S-(2-ethoxy-6-isopropoxy-4-pyrimidinylmethyl) S-(n-propyl) dithiophosphate,
S-(2,6-diethoxy-4-pyrimidinylmethyl) O-methyl S-methyl dithiophosphate,
S-(2,6-diethoxy-4-pyrimidinylmethyl) O-methyl S-(n-propyl) dithiophosphate and
O-ethyl S-(5-chloro-2,6-diethoxy-4-pyrimidinylmethyl) S-(n-propyl) dithiophosphate,
as well as formulation adjuvants.

23. A process for the manufacture of compounds of the general formula

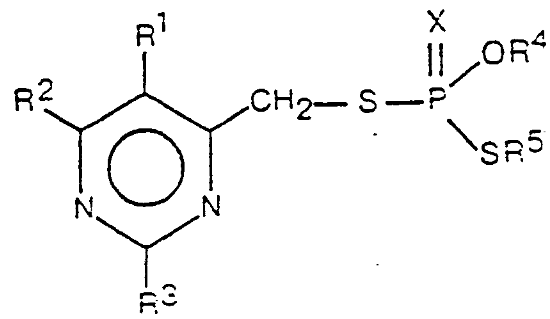

I

wherein $R^1$ signifies hydrogen, fluorine or chlorine,

$R^2$ signifies hydrogen, fluorine, chlorine, $C_{1-4}$-alkyl, $C_{1-6}$-alkoxy, 2-pro-penyloxy, 2-propynyloxy, 2-($C_{1-4}$-alkoxy)-ethoxy, unsubstituted phenoxy, phenoxy monosubstituted or disubstituted with halogen, $C_{1-6}$-alkylthio, $C_{1-4}$-alkylamino, di($C_{1-4}$-alkyl)-amino or a group (a) or (b)

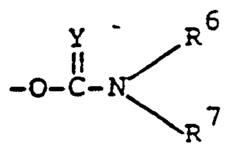

(a)

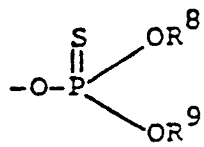

(b)

$R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyl-oxy, 2-propynyloxy, $C_{1-6}$-alkylthio, $C_{1-4}$-alkylamino, di($C_{1-4}$-alkyl)-amino or unsubstituted, monosubstituted or disubstituted phenyl, the substituents being fluorine, chlorine, trifluoromethyl and/or methoxy,

$R^4$ signifies $C_{1-3}$-alkyl,

$R^5$ signifies $C_{1-6}$-alkyl,

$R^6$ and $R^7$ or $R^8$ and $R^9$ each independently signify $C_{1-3}$-alkyl

and X and Y each independently signify oxygen or sulphur,

characterized by

a) reacting a pyrimidine derivative of the general formula

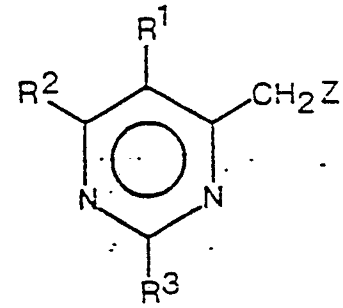

II

wherein $R^1$, $R^2$ and $R^3$ have the significances given above and Z signifies a leaving group, with a dithiophosphate or trithiophosphate of the general formula

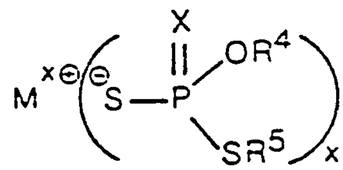

III

wherein $R^4$, $R^5$ and X have the significances given above, $M^{x\oplus}$ stands for an alkali metal, alkaline earth metal or optionally substituted ammonium ion and x signifies the valency of the cation $M^{x\oplus}$,
or
b) treating a pyrimidine derivative of the general formula

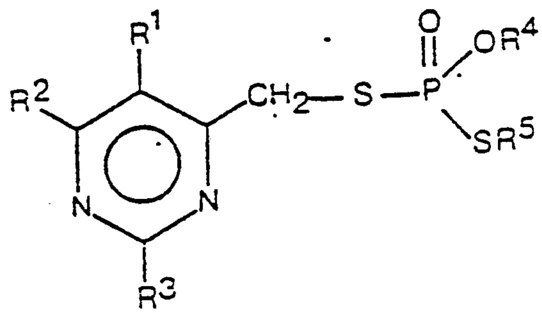

I'

wherein $R^1$, $R^2$, $R^3$, $R^4$ and $R^5$ have the significances given above, with the exception of the derivatives I' in which $R^2$ is

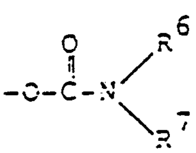

, with a sulphurizing agent.

24. A process according to claim 23, wherein $R^2$ signifies hydrogen, fluorine, chlorine, $C_{1-4}$-alkyl, $C_{1-6}$-alkoxy, 2-propenyloxy, 2-propenyloxy, $C_{1-6}$-alkylthio, di($C_{1-4}$-alkyl)amino or a group (a) or (b) and $R^3$ signifies hydrogen, fluorine, chlorine, $C_{1-6}$-alkyl, $C_{3-6}$-cycloalkyl, ($C_{1-4}$-alkoxy)methyl, ($C_{1-4}$-alkylthio)methyl, $C_{1-6}$-alkoxy, 2-propenyloxy, 2-pro-pynyloxy, $C_{1-6}$-alkylthio, di($C_{1-4}$-alkyl)amino or unsubstituted, monosubstituted or disubstituted phenyl, the substituents being fluorine, chlorine, trifluoromethyl and/or methoxy, and $R^1$, $R^4$, $R^5$ and X have the significances given in claim 23.

25. A process for the manufacture of a pest control composition, characterized by mixing at least one of compounds set forth in claims 1 to 22 with formulation adjuvants.

26. A method for the control of pests, characterized by treating the locus to be protected or the pests themselves with an effective amount of one of the compounds or pest control compositions set forth in claims 1 to 22.

27. The use of one of the compounds or compositions set forth in claims 1 to 22 for the control of pests.